# EUROPEAN PATENT SPECIFICATION

(11) **EP 0 674 634 B1**
(45) Date of publication and mention of the grant of the patent: **16.04.2003**
(21) Application number: 93900063.4
(22) Date of filing: 21.12.1992
(51) Int. Cl.: C07D 411/04, C07D 473/00, C07D 473/40, C07D 327/04

(54) **PROCESS FOR PREPARING SUBSTITUTED 1,3-OXATHIOLANES WITH ANTIVIRAL PROPERTIES**
VERFAHREN FÜR DIE HERSTELLUNG VON ANTIVIRALEN SUBSTITUIERTEN 1, 3-OXATHIOLANEN
PROCEDES DE PREPARATION DE 1,3-OXATHIOLANES SUBSTITUEES AUX PROPRIETES ANTIVIRALES

(43) Date of publication of application: 04.10.1995
(73) Proprietor: BIOCHEM PHARMA INC, Laval, Quebec H7V 4A7 (CA)
(72) Inventor: MANSOUR, Tarek, Apt.102, Montreal, Quebec H3A 2B1 (CA); TSE, Allan H.L., Ville Saint-Laurent, Quebec H4N 1V7 (CA); EVANS, Colleen, A., Montreal, Quebec H3A 2B1 (CA); JIN, Haolun, Pierrefonds, Quebec H8Y 3H3 (CA); ZACHARIE, Boulos, Lavaldes Rapides, Laval, Quebec H7N 4A1 (CA); NGUYEN-BA, Nghe, LaPrairie, Quebec J5R 5M5 (CA); BELLEAU, Bernard +di, Westmount, Quebec H3Y 2R3 (CA)
(74) Representative: Ritter, Stephen David
(86) International application number: CA9200557
(87) International publication number: WO94014802

(56) References cited:
- EP-A- 0 382 526
- EP-A- 0 515 157
- WO-A-91/11186
- WO-A-91/17159
- WO-A-92/08717
- WO-A-92/10496
- WO-A-92/14743
- WO-A-92/15308
- WO-A-92/18517

## Description

The present invention relates to processes for preparing substituted 1,3-oxathiolanes with antiviral activity and intermediates of use in their preparation.

### BACKGROUND OF THE INVENTION

Nucleosides, and in particular, 1,3-oxathiolanes and their analogues and derivatives are an important class of therapeutic agents. For example, a number of nucleosides have shown antiviral activity against retroviruses such as human immunodeficiency viruses (HIV), hepatitis B virus (HBV) and human T-lymphotropic virus (HTLV).

The most potent anti-HIV compounds thus far reported are 2',3'-dideoxynucleosides, more particularly, 2',3'-dideoxycytidine (DDC) and 3'-azido-2',3'-dideoxythymidine (AZT). These compounds are also active against other kinds of retroviruses such as the Moloney murine leukemia virus. However, clinically, both compounds are toxic.

A structurally distinct class of compounds known as 2-substituted-5-substituted-1,3-oxathiolanes has now been discovered and found to have superior antiviral and antiretroviral activity without cell toxicity. See, e.g., EP 0382526A and WO 91/17159.

Because of the increasing incidence and the life-threatening characteristics of AIDS, there is a great need to develop a general synthetic scheme for substituted 1,3-oxathiolanes which is efficient, amenable to large scale, inexpensive and based on readily available starting material. It is therefore an advantage of the present invention to provide synthesis of substituted 1,3-oxathiolanes that is readily feasible.

### DESCRIPTION OF THE INVENTION

The processes of this invention may be used to prepare the compounds of formula (I) and pharmaceutically acceptable salts or esters thereof: 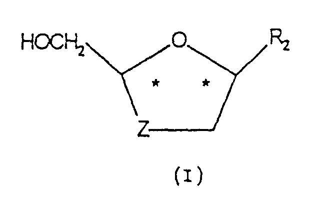 wherein R₂ is a purine or pyrimidine base or an analogue or derivative thereof; and Z is S, S=O or SO₂.

It will be appreciated by those skilled in the art that the compounds of formula (I) contain at least two chiral centers (shown as * in formula (I)) and thus exist in the form of two pairs of optical isomers (i.e., enantiomers) and mixtures thereof including racemic mixtures. Thus the compounds of formula (I) may be either *cis* isomers, as represented by formula (II), or *trans* isomers, as represented by formula (III), or mixtures thereof. Each of the *cis* and *trans* isomers can exist as one of two enantiomers or as mixtures thereof including racemic mixtures. The preparation of all such isomers and mixtures thereof including racemic mixtures are included within the scope of the invention.

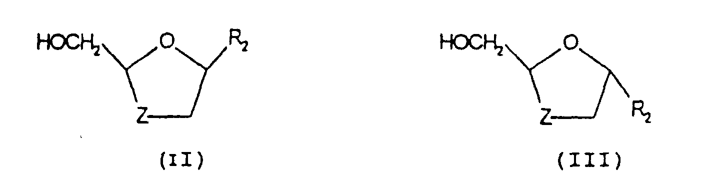

It will also be appreciated that when Z is S=O the compounds exist in two additional isomeric forms as shown in formulas (IIa) and (IIb) which differ in the configuration of the oxide oxygen atom relative to the 2,5-substituents. The processes of this invention additionally embrace the preparation of such isomers and mixtures thereof.

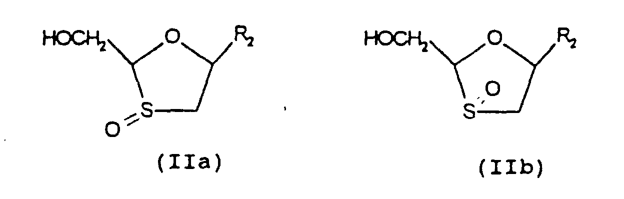

The purine or pyrimidine base or analogue or derivative thereof R₂ will be linked at any position of the base, preferably at the N9- or N1-position, respectively.

By "purine or pyrimidine base" or an analogue or derivative thereof is meant a purine or pyrimidine base found in native nucleosides or an analogue thereof which mimics such bases in that their structures (the kinds of atoms and their arrangement) are similar to the native bases but may either possess additional or lack certain of the functional properties of the native bases. Such analogues include those derived by replacement of a CH₂ moiety by a nitrogen atom (for example, 5-azapyrimidines such as 5-azacytosine) or vice versa (for example 7-deazapurines, for example 7-deazadenosine or 7-deazaguanosine) or both (e.g., 7-deaza-8-azapurines). By derivatives of such bases or analogues are meant those compounds wherein ring substituents are either incorporated, removed or modified by conventional substituents known in the art, e.g., halogen, hydroxyl, amino, C₁₋₆ alkyl. Such purine or pyrimidine bases, analogues and derivatives will be well known to those skilled in the art.

Conveniently the group R₂ is selected from: 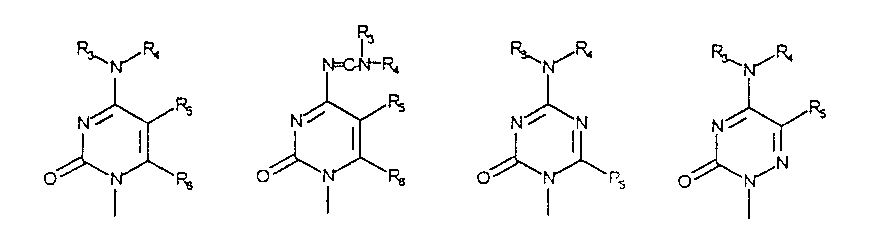 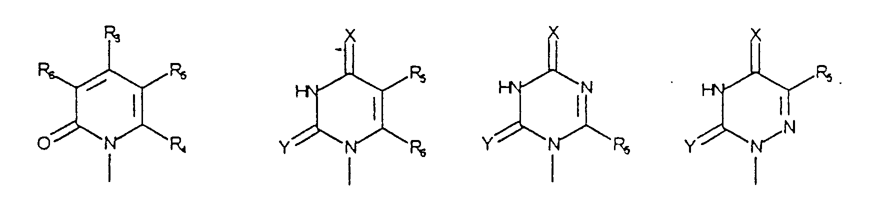 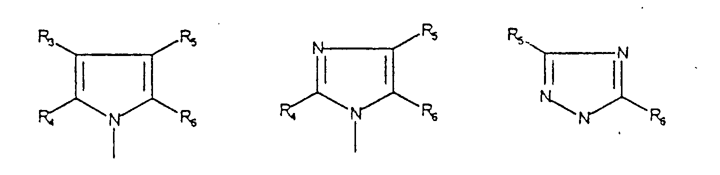 wherein:
X is oxygen or sulfur; Y is oxygen or sulfur;
R₃ and R₄ are independently selected from the group consisting of hydrogen, hydroxyl, amino, substituted or unsubstituted C₁₋₆ alkyl, or alkenyl having up to 6 carbon atoms or alkynyl having up to 6 carbon atoms, and substituted or unsubstituted C₁₋₁₀ acyl or aracyl;
R₅ and R₆ are independently selected from the group consisting of hydrogen, halogen, hydroxyl, amino, cyano, carboxy, carbamoyl, alkoxycarbonyl, hydroxymethyl, trifluoromethyl, thioaryl, substituted or unsubstituted C₁₋ ₆ alkyl or alkenyl having up to 6 carbon atoms or alkynyl having up to 6 carbon atoms, and substituted or unsubstituted C₁₋₁₀ acyloxy;
and 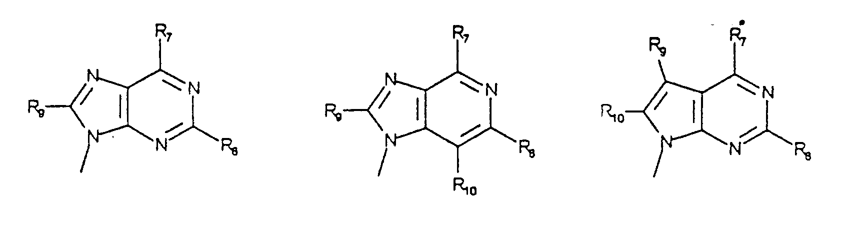 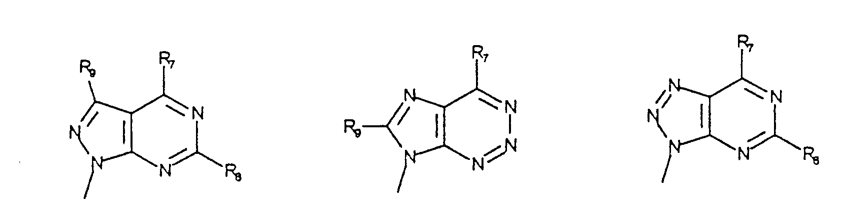 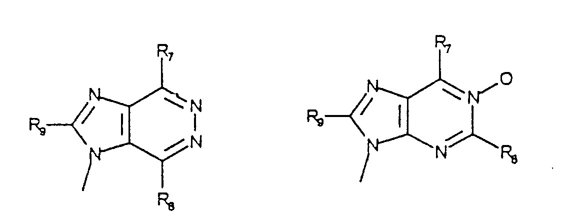 wherein:
R₇ and R₈ are independently selected from the group consisting of hydrogen, hydroxy, alkoxy, thiol, thioalkyl, amino, substituted amino, halogen, cyano, carboxy, alkoxycarbonyl, carbamoyl, substituted or unsubstituted C₁₋₆ alkyl, or alkenyl, or alkynyl, and substituted or unsubstituted C₁₋₁₀ acyloxy; and
R₉ and R₁₀ are independently selected from the group consisting of hydrogen, hydroxyl, alkoxy, amino, substituted amino, halogen, azido, substituted or unsubstituted C₁₋₆ alkyl or alkenyl or alkynyl, and substituted or unsubstituted C₁₋₁₀ acyloxy.

Preferably R₂ is 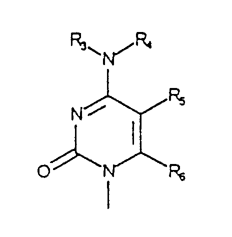 wherein R₃ and R₆ are hydrogen, and R₄ and R₅ are as defined above.

Z is preferably -S-.

By "a pharmaceutically acceptable salt or ester" is meant any pharmaceutically acceptable salt, ester, or salt of such ester, of a compound of formula (I) or any other compound which, upon administration to the recipient, is capable of providing (directly or indirectly) a compound of formula (I) or an antivirally active metabolite or residue thereof.

It will be appreciated by those skilled in the art that the compounds of formula (I) may be modified to provide pharmaceutically acceptable derivatives thereof, at functional groups in both the base moiety, R₂, and at the hydroxymethyl group of the oxathiolane ring. Modification at all such functional groups is included within the scope of the processes of this invention. However, of particular interest are pharmaceutically acceptable derivatives (e.g., esters) obtained by modification of the 2-hydroxymethyl group of the oxathiolane ring.

Preferred esters of the compounds of formula (I) produced by the process of this invention include the compounds in which OH is replaced by a carboxyl function R(CO)O- in which the non-carbonyl moiety R is selected from hydrogen; straight or branched chain alkyl (e.g. methyl, ethyl, n-propyl, t-butyl, n-butyl); alkoxyalkyl (e.g. methoxymethyl); aralkyl (e.g. benzyl); aryloxyalkyl (e.g. phenoxymethyl); aryl (e.g. phenyl optionally substituted by halogen, C₁₋₄ alkyl or C₁₋₄ alkoxy); substituted dihydropyridinyl (e.g. N-methyldihydropyridinyl); sulphonate esters such as alkyl-or aralkylsulphonyl (e.g. methanesulphonyl); sulfate esters; amino acid esters (e.g. L-valyl or L-isoleucyl) and mono-, di- or tri-phosphate esters. Also included within the scope of such esters are esters derived from polyfunctional acids such as carboxylic acids containing more than one carboxyl group, for example, dicarboxylic acids HOOC(CH₂)_{q}COOH where q is an integer of 0 to 10 (for example, succinic acid) or phosphoric acids.

Methods for preparing such esters are well known. See, for example, Hahn et al., "Nucleotide Dimers as anti-Human Immunodeficiency Virus Agents", Nucleotide Analogues, pp. 156-159 (1989) and Busso et al., "Nucleotide Dimers Supress HIV Expression In Vitro", AIDS Research and Human Retroviruses, 4(6), pp.449-455 (1988). Where esters are derived from such acids, each acidic group is preferably esterified by a compound of formula (I) or other nucleoside or analogs and derivatives thereof to provide esters of the formula: 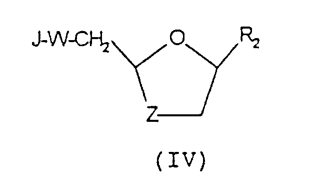 where W is -OC-(CH₂)ₙ-CO- where n is an integer of 0 to 10, a phosphate group, or a thiophosphate group,
J is any nucleoside or nucleoside analog or derivative thereof and Z and R₂ are as defined above. Among the preferred nucleosides and nucleoside analogs are 3'-azido-2',3'-dideoxythymidine; 2',3'-dideoxycytidine; 2',3'-dideoxyadenosine; 2',3'-dideoxyinosine; 2',3'-dideoxythymidine; 2',3'-dideoxy-2',3'-didehydrothymidine; 2',3'-dideoxy-2',3'-didehydrocytidine and ribavirin and those nucleosides whose bases are depicted on pages 4-5 of this specification. The most preferred dimer is a homodimer consisting of two nucleosides of formula (I).

With regard to the above described esters, unless otherwise specified, any alkyl moiety present advantageously contains 1 to 16 carbon atoms, preferably 1 to 4 carbon atoms and could contain one or more double bonds. Any aryl moiety present in such esters advantageously comprises a phenyl group.

In particular, the esters may be a C₁₋₁₆ alkyl ester, an unsubstituted benzoyl ester or a benzoyl ester substituted by at least one halogen (bromine, chlorine, fluorine or iodine), C₁₋₆ alkyl or alkenyl, saturated or unsaturated C₁₋₆ alkoxy, nitro or trifluoromethyl groups.

Pharmaceutically acceptable salts of the compounds of formula (I) include those derived from pharmaceutically acceptable inorganic and organic acids and bases. Examples of suitable acids include hydrochloric, hydrobromic, sulfuric, nitric, perchloric, fumaric, maleic, phosphoric, glycollic, lactic, salicylic, succinic, *p*-toluenesulfonic, tartaric, acetic, citric, methanesulfonic, formic, benzoic, malonic, naphthalene-2-sulfonic, and benzenesulfonic acids. Other acids such as oxalic, while not in themselves pharmaceutically acceptable, may be useful in the preparation of salts useful as intermediates in obtaining the compounds of the invention and their pharmaceutically acceptable acid addition salts.

Salts derived from appropriate bases include alkali metal (e.g. sodium), alkaline earth metal (e.g. magnesium), ammonium and N(R')₄⁺ (where R' is C₁₋₄ alkyl) salts.

In the processes for preparing the compounds of this invention, the following definitions are used:
R₁ is a hydroxyl protecting function such as an acyl having from 1 to 16 carbon atoms unsubstituted or substituted with a heteroatom (e.g. benzoyl), or a silyl function such as trialkylsilyl (e.g. t-butyldimethylsilyl);
R₂ is a purine or pyrimidine base or an analogue or derivative thereof;
R_{w} is hydrogen or R₁;
Rₓ is a substituted or unsubstituted C₁₋₆ alkyl;
R_{y} is a substituted or unsubstituted C₁₋₁₂ alkyl or substituted or unsubstituted C₆₋₂₀ aryl; and
L is a leaving group.

As used in the processes of this invention, a "leaving group" is an atom or group which is displaceable upon reaction with an appropriate base, with or without a Lewis acid. Suitable leaving groups include alkoxy carbonyl groups such as ethoxy carbonyl; halogens such as iodine, bromine or chlorine, fluorine; substituted or unsubstituted saturated or unsaturated thiolates, such as thiomethyl or thiophenyl; substituted or unsubstituted saturated or unsaturated selenino compounds, such as phenyl selenide or alkyl selenide; substituted or unsubstituted saturated or unsaturated aliphatic or aromatic ketones such as methyl ketone; or -OR_{z} where R_{z} is hydrogen or a substituted or unsubstituted saturated or unsaturated alkyl group, e.g., a C₁₋₆ alkyl or alkenyl group such as methyl; a substituted or unsubstituted aliphatic or aromatic acyl group, e.g., a C₁₋₆ aliphatic acyl group such as acetyl and an aromatic acyl group such as benzoyl; a substituted or unsubstituted saturated or unsaturated alkoxy carbonyl group, such as methyl carbonate and phenyl carbonate; substituted or unsubstituted sulphonyl imidazolide; substituted or unsubstituted carbonyl imidazolide; substituted or unsubstituted aliphatic or aromatic amino carbonyl group, such as phenyl carbamate; substituted or unsubstituted alkyl imidate group such as trichloroacetamidate; substituted or unsubstituted saturated or unsaturated phosphinoyl, such as diethylphosphinoyl; substituted or unsubstituted aliphatic or aromatic sulphonyl group, such as tosylate.

One process according to the invention is illustrated in SCHEME 1. The process of SCHEME 1 using specific reagents and compounds is depicted, for example, in SCHEMES 1A and 1B.

The various steps involved in the synthesis as illustrated in SCHEME 1 may be briefly described as follows:
Step 1: A mercaptoacetaldehyde monomer produced from the dimer in a suitable inert solvent (preferably, pyridine, toluene or DMSO) is reacted directly with any aldehyde of the formula R_{w}OCH₂CHO (VII) to yield an oxathiolane lactol of formula (XIII).
   Alternatively, the glycoaldehyde derivative of formula (VII) may be generated from the dimer by any means known in the art.
Step 2: The hydroxyl group of the compound of formula (XIII) is converted to a leaving group with a suitable reagent in a compatible organic solvent to yield an important oxathiolane intermediate of formula (XIV).
Step 3: The oxathiolane intermediate of formula (XIV) is reacted with a previously silylated purine or pyrimidine base in the presence of a Lewis acid (preferably TMSOTf, TMSI, TiCl₄ or SnCl₄) to give a purin-9'-yl or pyrimidin-1'-yl substituted oxathiolane of formula (IX) where Z is sulfur. The compounds of formula (IX) are often predominantly obtained in the *cis* isomer.
   Optionally, the sulfur may be oxidized at this stage or at any other following stage to obtain compounds where Z is S=O or SO₂.
Step 4: The base R₂ shown in formula (IX) is acylated with acetic anhydride in a suitable solvent to yield a compound of formula (X) where R₂' is acylated R₂ which provides for easier separation of isomers.
   Therefore, at this stage, the compound of formula (X) is optionally separated to its *cis* or *trans* isomer.
Step 5: The acetyl functionality of R₂' of compound of formula (X) are hydrolyzed under basic conditions to yield an oxathiolane of formula (XI).

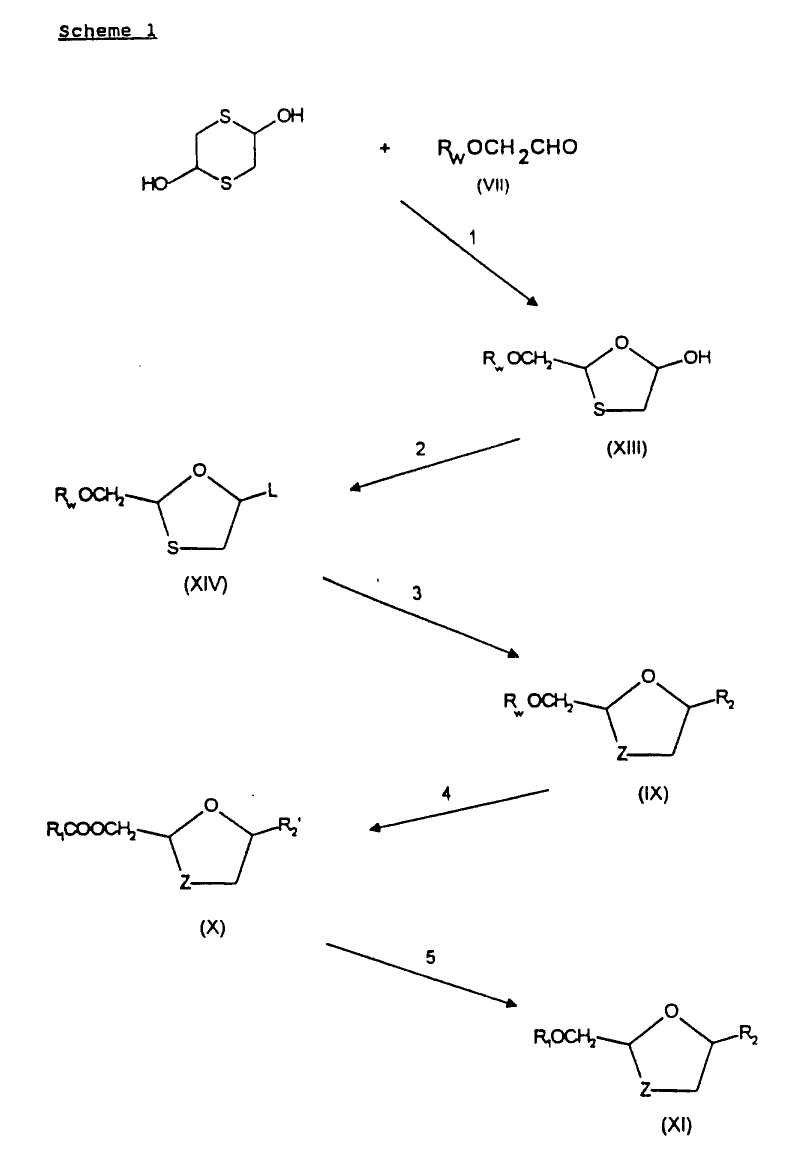

### Scheme 1a:

Step 1: A mercaptoacetaldehyde monomer produced from the dimer in pyridine is reacted directly with benzoyloxyacetaldehyde (VII-A) to yield an oxathiolane lactol of formula (XIII-A).
Step 2: The hydroxyl group of the compound of formula (XIII-A) is converted to a leaving group with acetyl chloride in a compatible organic solvent to yield intermediate of formula (XIV-A).
Step 3: The oxathiolane intermediate of formula (XIV-A) is reacted with a previously silylated cytosine to give a cytosin-1'-yl oxathiolane of formula (IX-A) where Z is sulfur.
Step 4: The amine function of the base in compound of formula (IX-A) is acylated with acetic anhydride in pyridine to yield a compound of formula (X-A) which provides for easier separation of isomers.
Step 5: The N-acetyl function of the compound of formula (X-A) are hydrolyzed under basic conditions to yield an oxathiolane of formula (XI-A).

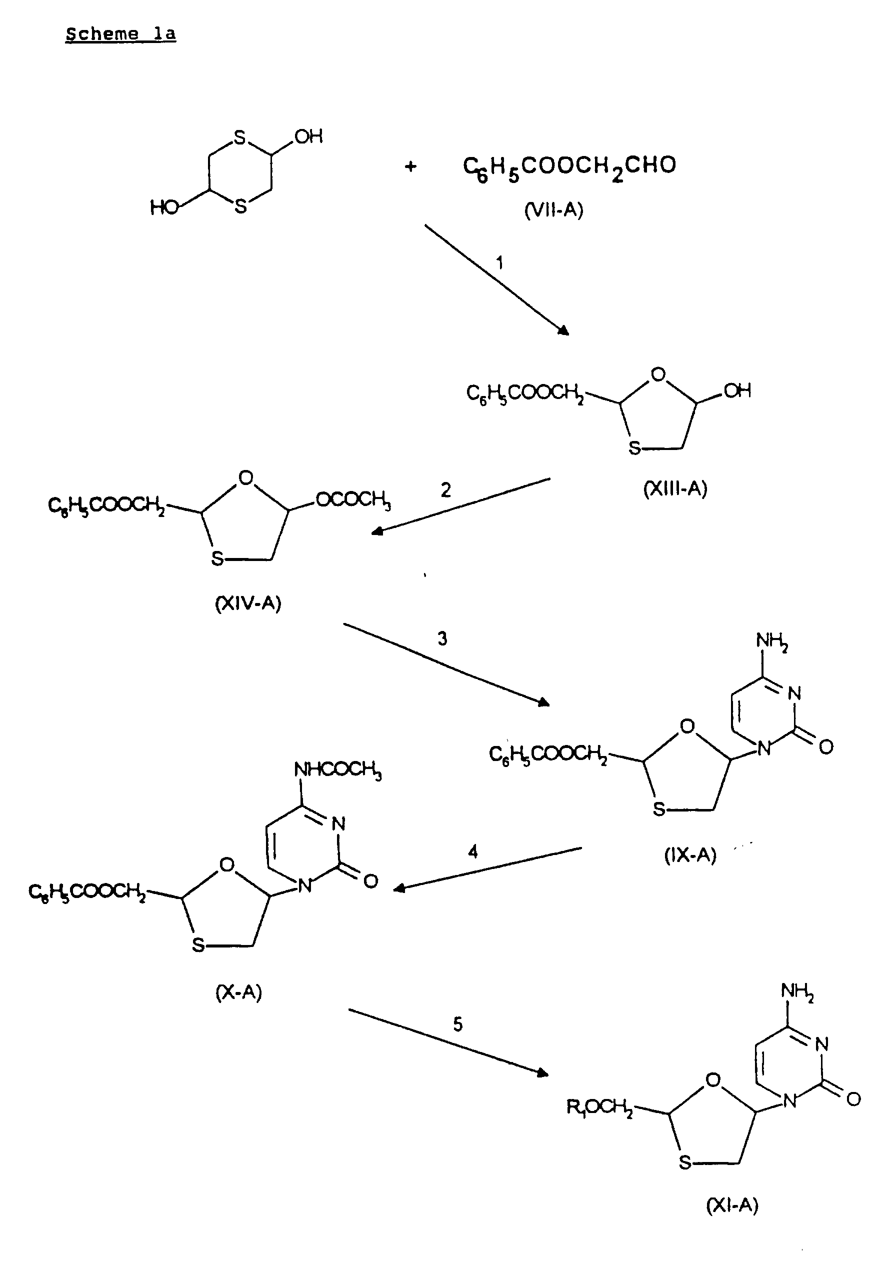

### Scheme 1b:

The glycoaldehyde dimer (VII-B) is used as a source of the glycoaldehyde.

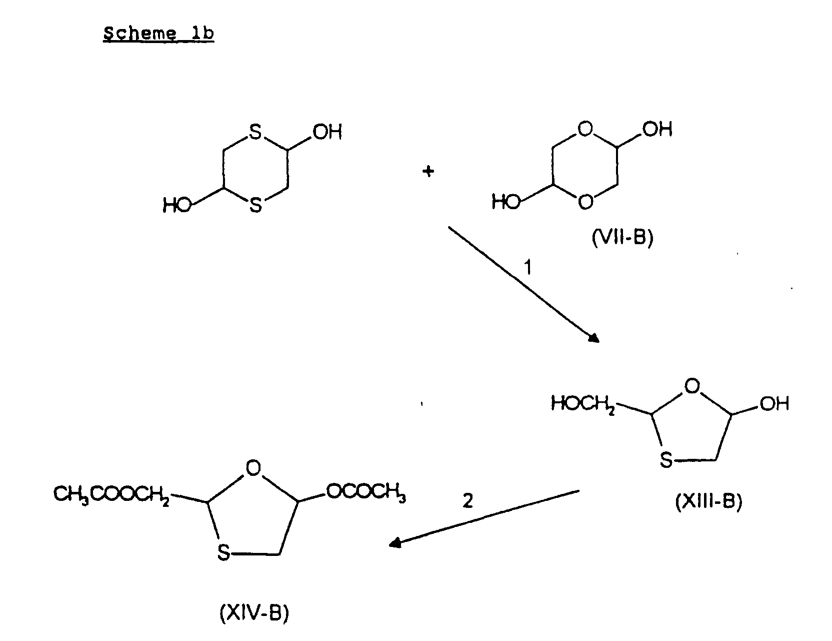

A second and preferred process for producing oxathiolane compounds is illustrated in SCHEME 2. This process is illustrated using specific reagents and compounds in SCHEME 2A.

The various steps involved in the synthesis as illustrated in SCHEME 2 may be briefly described as follows:
Step 1: Mercaptoacetaldehyde monomer produced from the dimer in a suitable solvent is reacted directly with any organic glyoxylate of the formula R_{y}OOCCHO to yield an oxathiolane lactol of formula (XV).
Step 2: The hydroxyl group of the compound of formula (XV) is converted to a leaving group with a suitable reagent in a compatible organic solvent to yield an important oxathiolane intermediate of formula (XVI).
Step 3: The oxathiolane intermediate of formula (XVI) is reacted with a previously silylated purine or pyrimidine base, in the presence of a Lewis acid, to give purin-9'-yl or pyrimidinyl-1'-yl substituted oxathiolane of formula (XVII) where Z is S, predominantly as the *cis*-isomer.
   Optionally, the sulfur may be oxidized at this stage or at any other following stage to give compounds where Z is S=O or SO₂.
Step 4: The ester group of the oxathiolane of formula (XVII) is selectively reduced with a suitable reducing agent in a compatible organic solvent to yield an oxathiolane nucleoside of formula (XVIII).
   At this stage, the compound of formula (XVIII) is optionally separated to its *cis* and *trans* isomers.
Step 5: The hydroxyl group of the compound of formula (XVIII) is protected with a suitable silyl protecting group in an appropriate solvent to yield an oxathiolane of formula (XIX).
Step 6: The R₂ base of formula (XIX-A) can be interconverted to another base R₂' by reaction with a suitable reagent to yield an oxathiolane of formula (XX).
Step 7: The protecting group R₁ of the compound of formula (XX) is removed under neutral conditions using a suitable reagent in a suitable solvent to yield the oxathiolane of formula (I).

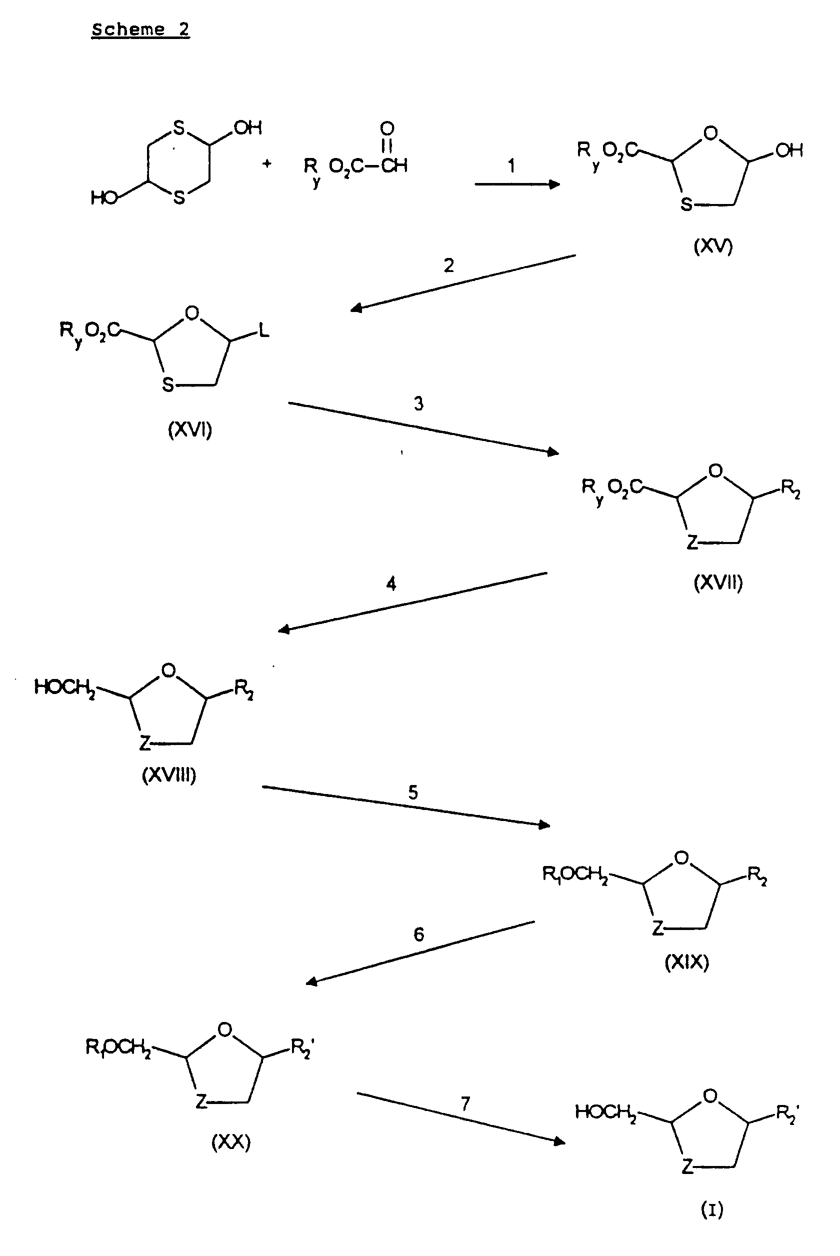

### Scheme 2a:

Step 1: Mercaptoacetaldehyde dimer in pyridine is reacted directly with ethyl glyoxylate to yield an oxathiolane lactol of formula (XV-A).
Step 2: The hydroxyl group of the compound of formula (XV-A) is converted to an acetal leaving group with acetyl chloride in a compatible organic solvent to yield intermediate of formula (XVI-A).
Step 3: The oxathiolane intermediate of formula (XVI-A) is reacted with previously silylated uracil, in the presence of trimethylsilyl iodide, to give uracil-1'-yl oxathiolane of formula (XVII-A), predominantly as the *cis*-isomer.
Step 4: The ester group of the oxathiolane of formula (XVII-A) is selectively reduced with sodium borohydride in methanol to yield an oxathiolane nucleoside of formula (XVIII-A).
Step 5: The hydroxyl group of the compound of formula (XVIII-A) is protected with t-butyldimethyl silyl in dimethylformamide (DMF) to yield an oxathiolane of formula (XIX-A).
Step 6: The uracil base of formula (XIX-A) can be interconverted to cytosine, by reaction with p-chlorophenoxy phosphorous oxychloride followed by amination with ammonia in methanol to yield an oxathiolane of formula (XX-A).
Step 7: The silyl group of the compound of formula (XX-A) is removed under neutral conditions using tetra n-butyl ammonium fluoride in tetrahydrofuran to yield the oxathiolane of formula (I).

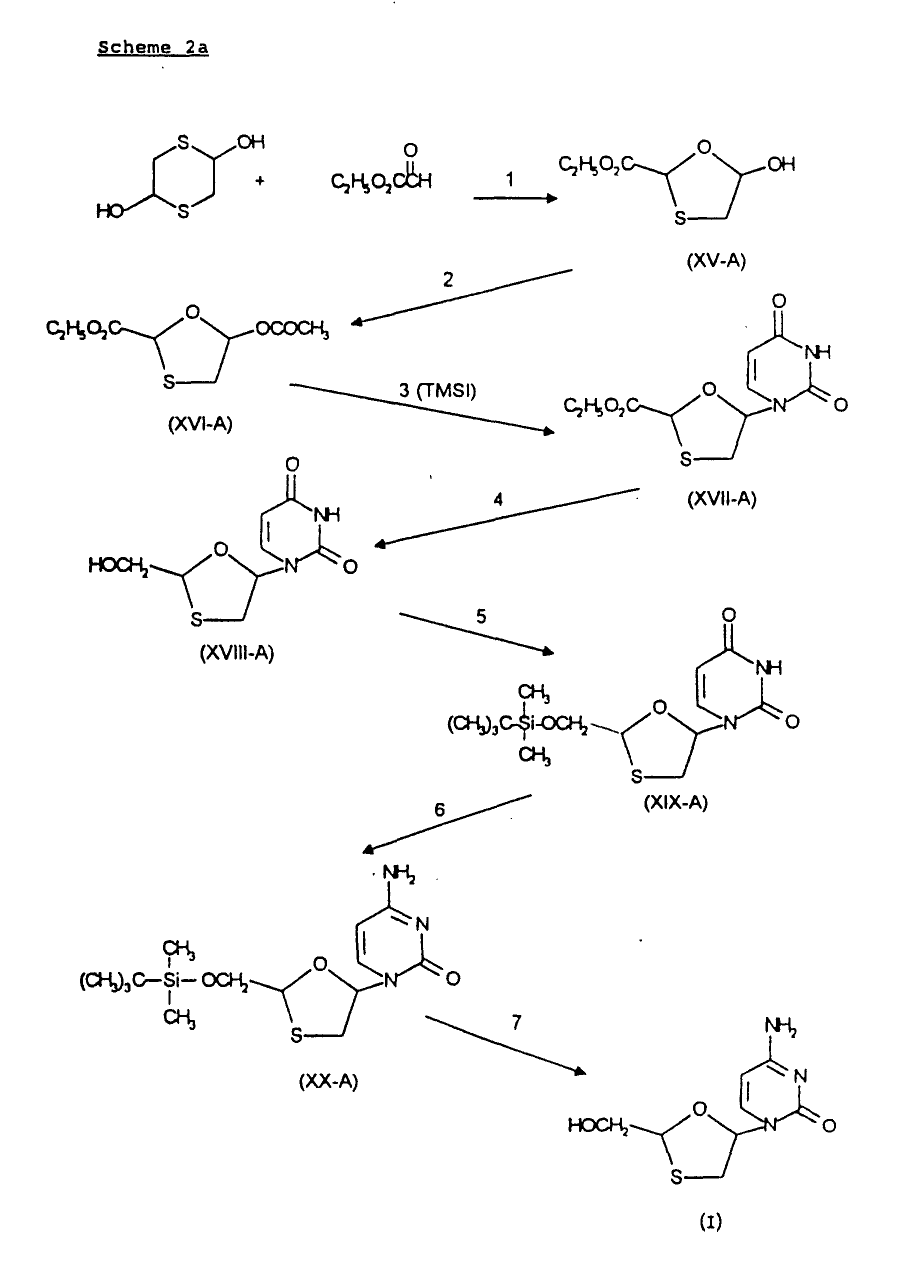

Although the process of Scheme 2 generally provides nucleoside analogues predominantly in their *cis* form, such a process is most preferred for pyrimidine bases because of high *cis*-selectivity.

For purines, although the process of Scheme 2 does yield more *cis* isomer than *trans*, the ratio obtained is moderate. An alternative process has been designed to obtain purin-yl nucleosides in high *cis*:*trans* ratios.

Briefly, steps 1 and 2 of Scheme 2 remain the same. However, the coupling procedure (step 3) between the compound of formula (XVI) and the base (preferably purine) is modified as follows:
Step 3a: The oxathiolane intermediate of formula (XVI) is reacted with a halogen-containing silyl Lewis acid such as trimethylsilyl iodide, to give an intermediate of formula (XXVI): 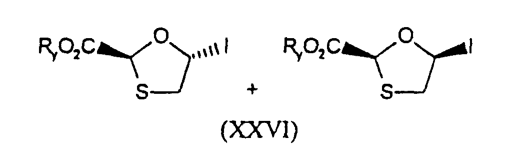 which is the iodo ester of the intermediate of formula (XVI).
Step 3b: The intermediate of formula (XXVI) is then mixed with a base (preferably a purine) under basic conditions to yield the intermediate of formula (XVII) predominantly as the *cis* isomer.

As an alternative to process 2, a third process according to this invention for producing oxathiolane compounds is illustrated in SCHEME 3. This process is illustrated using specific reagents and compounds, for example, in SCHEME 3A.

The various steps involved in the synthesis as illustrated in SCHEME 3 may be briefly described as follows:
Step 1: Similar to Scheme 2.
Step 2: The hydroxyl group of the intermediate of formula (XV), is converted to a leaving group with a suitable reagent in a compatible organic solvent to yield an important intermediate of formula (XXI).
Step 3': The ester group of the intermediate of formula (XXI) is selectively reduced with a suitable reducing agent in a compatible organic solvent and the resultant hydroxyl group is directly protected with a suitable group R₁ to yield an oxathiolane of formula (XXII).
Step 4': The oxathiolane of formula (XXII) is reacted with previously silylated purine or pyrimidine base in the presence of a Lewis Acid (preferably, TMSOTf, TMSI, TiCl₄ or SnCl₄) to give a pyrimidin-1'-yl or purin-9'-yl oxathiolane of formula (XXIII) where Z is S (and optionally oxidized to S=O or SO₂).
Step 5': The base R₂ shown in formula (XXIII) is acylated with acetic anhydride in a solvent to yield a compound of formula (XXIV) where R₂' is an acylated R₂ which provides for easier separation of isomers.
   Therefore, at this stage, the compound of formula (X) is optionally separated to its *cis* or *trans* isomer.
Step 6': The acetyl functionality of the compound of formula (XXIV) is hydrolyzed under basic conditions to yield an oxathiolane of formula (XXV).
Step 7': Removal of the R₁ protecting group is effected by suitable reagents in a compatible solvent to yield an oxathiolane of formula (I).

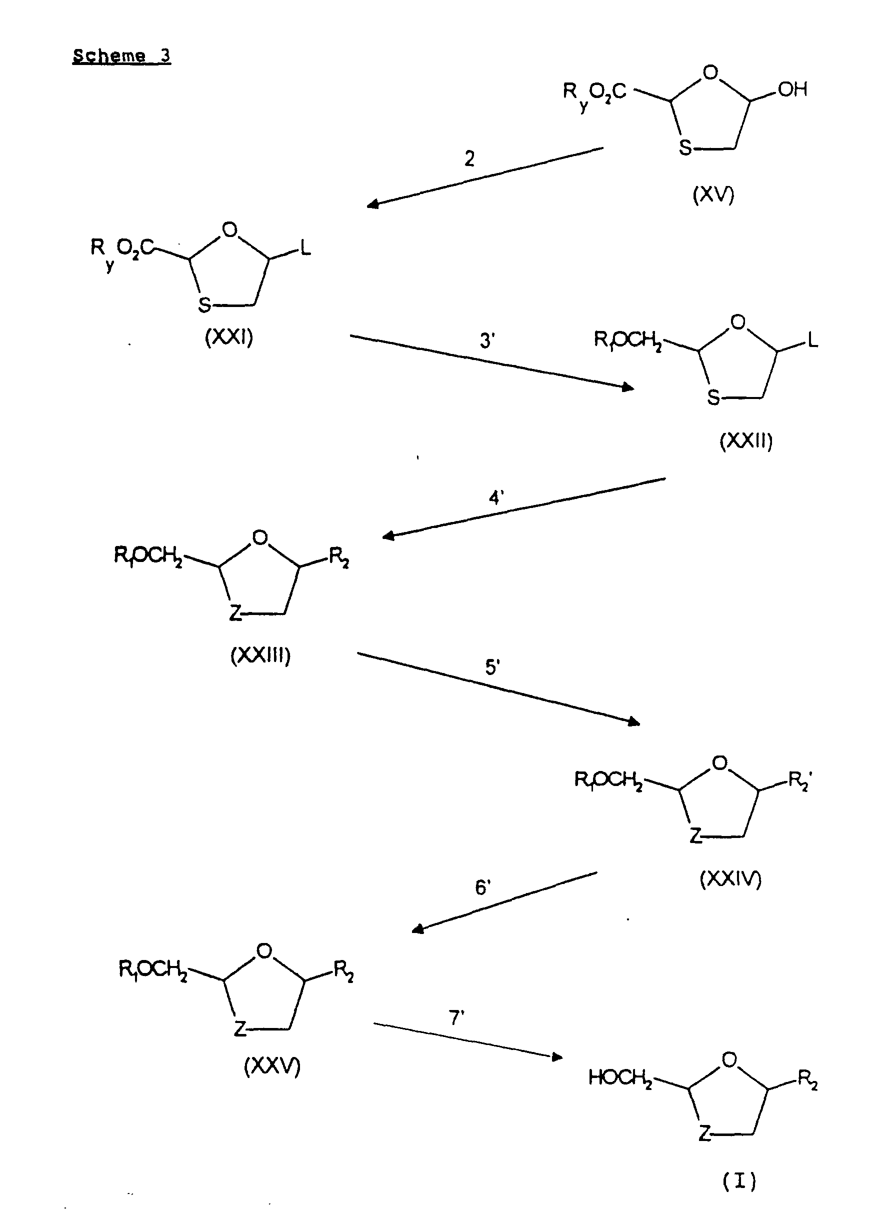

### Scheme 3a:

Step 2: The hydroxyl group of the intermediate of formula (XV-A), is converted to an acetal leaving group with methyl chloroformate in a compatible organic solvent to yield intermediate of formula (XXI-A).
Step 3': The ester group of the intermediate of formula (XXI-A) is selectively reduced with sodium borohydride in methanol and the resultant hydroxyl group is directly protected with t-butyldiphenylsilyl to yield an oxathiolane of formula (XXII-A).
Step 4': The oxathiolane of formula (XXII-A) is reacted with previously silylated cytosine, in the presence of trimethylsilyltriflate or iodotrimethylsilane, to give cytosin-1'-yl oxathiolane of formula (XXIII-A).
Step 5': The amine function of the cytosine of compound (XXIII-A) is acylated with acetic anhydride in pyridine to yield a compound of formula (XXIV-A) so that the *cis-* and *trans*-isomers may be separated.
Step 6': The acetyl functionality of the compound of formula (XXIV-A) is hydrolyzed under basic conditions to yield an oxathiolane of formula (XXV-A).
Step 7': Removal of the silyl group is effected by using tetra-n-butylammonium fluoride in tetrahydrofuran yield an oxathiolane of formula (I).

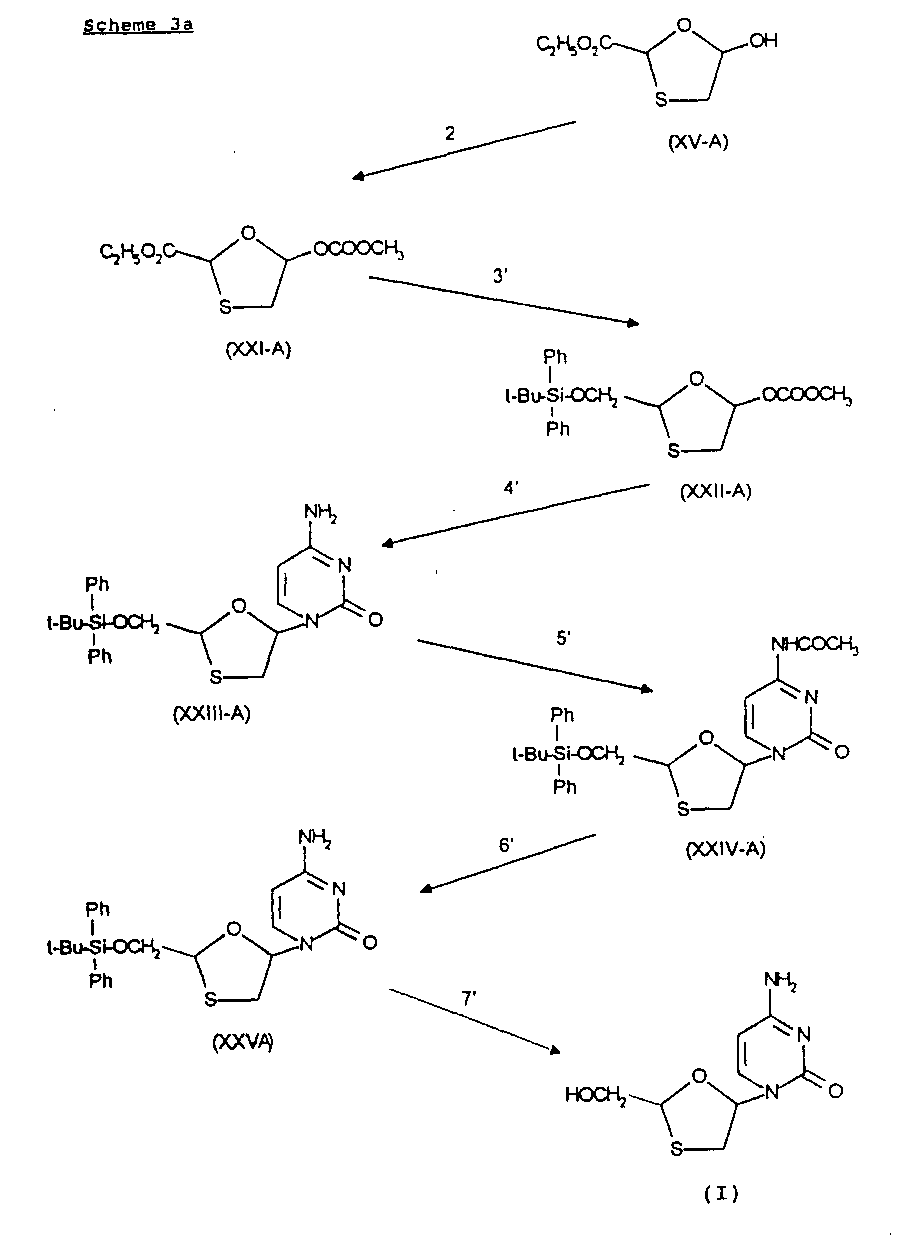

In the processes of this invention, the following intermediates are of particular importance:
trans-2-hydroxymethyl-5-acetoxy-1,3-oxathiolane;
cis-2-benzoyloxymethyl-5-hydroxy-1,3-oxathiolane, trans-2-benzoyloxymethyl-5-hydroxy-1,3-oxathiolane and mixtures thereof;
cis-2-benzoyloxymethyl-5-(4',5'-dichlorobenzoyloxy)-1,3-oxathiolane, trans-2-benzoyloxymethyl-5-(4',5'-dichlorobenzoyloxy)-1,3-oxathiolane and mixtures thereof;
cis-2-benzoyloxymethyl-5-trimethylacetoxy-1,3-oxathiolane, trans-2-benzoyloxymethyl-5-trimethylacetoxy-1,3-oxathiolane and mixtures thereof;
cis-2-benzoyloxymethyl-5-(2',2',2'-trichloroethoxycarbonyloxy)1,3-oxathiolane, trans-2-benzoyloxymethyl-5-(2',2',2'-trichloroethoxy carbonyloxy)1,3-oxathiolane and mixtures thereof;
cis-2-benzoyloxymethyl-5-ethoxycarbonyloxy-1,3-oxathiolane, trans-2-benzoyloxymethyl-5-ethoxycarbonyloxy-1,3-oxathiolane and mixtures thereof;
cis-2-benzoyloxymethyl-5-methoxycarbonyloxy-1,3-oxathiolane, trans-2-benzoyloxymethyl-5-methoxycarbonyloxy-1,3-oxathiolane and mixtures thereof; cis-2-benzoyloxymethyl-5-acetoxy-1,3-oxathiolane, trans-2-benzoyloxymethyl-5-acetoxy-1,3-oxathiolane and mixtures thereof;
cis-2-carboethoxy-5-methoxycarbonyloxy-1,3-oxathiolane, trans-2-carboethoxy-5-methoxycarbonyloxy-1,3-oxathiolane and mixtures thereof;
cis-2-carboethoxy-5-acetoxy-1,3-oxathiolane, trans-2-carboethoxy-5-acetoxy-1,3-oxathiolane and mixtures thereof;
cis-2-carboethoxy-5-(N4'-acetylcytosin-1'-yl)-1,3-oxathiolane;
cis-2-carboethoxy-5-(cytosin-1'-yl)-1,3-oxathiolane;
cis-2-carboethoxy-5-(uracil-1'-yl)-1,3-oxathiolane;
cis-2-benzoyloxymethyl-5-(cytosin-1'-yl)-1,3-oxathiolane;
cis-2-ethyl-5-iodo-1,3-oxathiolan-2-carboxylate, trans-2-ethyl-5-iodo-1,3-oxathiolan-2-carboxylate and mixtures thereof;
cis-2-ethyl-5-(6'-chloropurin-9'-yl)-1,3-oxathlolan-2-carboxylate, trans-2-ethyl-5-(6'-chloropurin-9'-yl)-1,3-oxathiolan-2-carboxylate and mixtures thereof; and
cis-2-ethyl-5-(6'-chloropurin-7'-yl)-1,3-oxathiolan-2-carboxylate, trans-2-ethyl-5-(6'-chloropurin-7'-yl)-1,3-oxathiolan-2-carboxylate and mixtures thereof; and cis 2-carboethoxy-5-hydroxy-1,3-oxathiolane, trans 2-carboethoxy-5-hydroxy-1,3-oxathiolane and mixtures thereof; and cis 2-benzoyloxymethyl-5-benzoyloxy-1,3-oxathiolane, trans 2-benxoyloxymethyl-5-benzoyloxy-1,3-oxathiolane and mixtures thereof; and cis 2-acetoxymethyl-5-acetoxy-1,3-oxathiolane, trans-acetoxymethyl-5-acetoxy-1,3-oxathiolane and mixtures thereof.

Some of the steps described hereinabove have been reported in the context of purine nucleoside synthesis, for example, in "Nucleoside Analogues - Chemistry, Biology and Medical Applications", R.T. Walker et al, Eds, Plenum Press, New York (1979) at pages 193-223, the text of which is incorporated herein by reference.

It will be appreciated that the reactions of the above described processes may require the use of, or conveniently may be applied to, starting materials having protected functional groups, and deprotection might thus be required as an intermediate or final step to yield the desired compound. Protection and deprotection of functional groups may be effected using conventional means. Thus, for example, amino groups may be protected by a group selected from aralkyl (e.g., benzyl), acyl or aryl (e.g., 2,4-dinitrophenyl); subsequent removal of the protecting group being effected when desired by hydrolysis or hydrogenolysis as appropriate using standard conditions. Hydroxyl groups may be protected using any conventional hydroxyl protecting group, for example, as described in "Protective Groups in Organic Chemistry", Ed. J.F.W. McOmie (Plenum Press, 1973) or "Protective Groups in Organic Synthesis" by Theodora W. Greene (John Wiley and Sons, 1991). Examples of suitable hydroxyl protecting groups include groups selected from aralkyl (e.g., benzyl, diphenylmethyl or triphenylmethyl), heterocyclic groups such as tetrahydropyranyl, acyl, (e.g., acetyl or benzoyl) and silyl groups such as trialkylsilyl (e.g., t-butyldimethylsilyl). The hydroxyl protecting groups may be removed by conventional techniques. Thus, for example, alkyl, silyl, acyl and heterocyclic groups may be removed by solvolysis, e.g., by hydrolysis under acidic or basic conditions. Aralkyl groups such as triphenylmethyl may similarly be removed by solvolysis, e.g., by hydrolysis under acidic conditions. Aralkyl groups such as benzyl may be cleaved, for example, by hydrogenolysis. Silyl groups may also conveniently be removed using a source of fluoride ions such as tetra-n-butylammonium fluoride.

In the above processes the compounds of formula (I) are generally obtained as a mixture of the cis and trans isomers. However, in the process depicted in Scheme 2, the ratio of *cis*:*trans* may approach 15:1 for pyrimidines, whereas it may approach 10:1 for the purines in the case of the modified process of Scheme 2.

These isomers may be separated, for example, by acetylation, e.g., with acetic anhydride followed by separation by physical means, e.g., chromatography on silica gel and deacetylation, e.g., with methanolic ammonia or by fractional crystallization.

Pharmaceutically acceptable salts of the compounds of the invention may be prepared as described in United States Patent No. 4,383,114. Thus, for example, when it is desired to prepare an acid addition salt of a compound of formula (I), the product of any of the above procedures may be converted into a salt by treatment of the resulting free base with a suitable acid using conventional methods.

Pharmaceutically acceptable acid addition salts may be prepared by reacting the free base with an appropriate acid optionally in the presence of a suitable solvent such as an ester (e.g., ethyl acetate) or an alcohol (e.g., methanol, ethanol or isopropanol). Inorganic basic salts may be prepared by reacting the free base with a suitable base such as an alkoxide (e.g., sodium methoxide) optionally in the presence of a solvent such as an alcohol (e.g., methanol). Pharmaceutically acceptable salts may also be prepared from other salts, including other pharmaceutically acceptable salts, of the compounds of formula (I) using conventional methods.

A compound of formula (I) may be converted into a pharmaceutically acceptable phosphate or other ester by reaction with a phosphorylating agent, such as POCl₃, or a suitable esterifying agent, such as an acid halide or anhydride, as appropriate. An ester or salt of a compound of formula (I) may be converted to the parent compound, for example, by hydrolysis.

Where the compound of formula (I) is desired as a single isomer it may be obtained either by resolution of the final product or by stereospecific synthesis from isomerically pure starting material or any convenient intermediate.

Resolution of the final product, or an intermediate or starting material therefore may be effected by any suitable method known in the art: see for example, Stereochemistry of Carbon Compounds, by E.L. Eliel (McGraw Hill, 1962) and Tables of Resolving Agents, by S.H. Wilen.

The invention will be further described by the following examples. All temperatures are in degrees celsius.

Examples 1 to 7, and 19 to 23 relate to the process as depicted in Scheme 1. Examples 8 to 10, and 13 to 18 relate to the process as depicted in Scheme 2, and Examples 11, 12, and 19 to 21 relate to the process as depicted in Scheme 3. Examples 24 and 25 relate to the modified process as depicted in Scheme 2 (preferably for purines) and summarized on page 20 of this application.

### EXAMPLES.

### EXAMPLE 1

### CIS AND TRANS 2-BENZOYLOXYMETHYL-5-HYDROXY -1,3-OXATHIOLANE

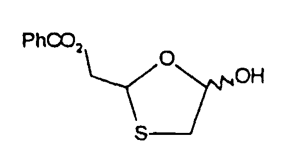

A solution of 216.33 g (1.32 mol) of benzoyloxyacetaldehyde and 100.31 g (0.66 mol) of 1,4-dithiane-2,5-diol in 373 ml (4.61 mol) of pyridine was heated at 60-65°C under nitrogen atmosphere for 1 hour until all solids dissolved. After cooling, pyridine was removed by distillation and the residue was purified on a silica gel column using EtOAc: hexanes (1:2) as eluent to give 268.5 g of the title compounds (2:1 *trans:cis*); ¹H NMR (CDCl₃) δ 3.03 (m, CH₂S), 4.40 (m, CH₂O), 4.70 (brs, 0.66H), 4.83 (brs, 0.33H), 5.45 (m, 0.33H), 5.62 (t, 0.66H, J=5Hz), 5.73 (brs, 0.33H), 5.88 (brs, 0.,66H), 7.94 (d, 0.66H, J=7.3 Hz), 7.98 (d, 1.33H, J=7.3 Hz), 7.49 (t, 1H, J=7 Hz), 7.99 (d, 2H, J=7.3 Hz) ¹³C NMR (CDCl₃) *trans* isomer δ 37.9, 65.9, 80.6, 99.6, 129.5, 129.3, 128.2, 133.0, 166.2;
*cis* isomer δ 38.5, 65.9, 82.1, 100.4, 128.3, 129.3, 133.0, 166.3.

### EXAMPLE 2

### CIS AND TRANS 2-BENZOYLOXYMETHYL-5-ACETOXY -1,3-OXATHIOLANE

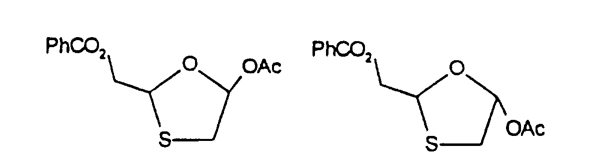

To a solution of 29.76 g (0.132 mol) of *cis* and *trans* 2-benzoyloxymethyl-5-hydroxy-1,3-oxathiolane (as prepared in Example 1) in dichloromethane (65 mL) and pyridine (32 mL) was added dropwise 28.1 mL (0.395 mol) of acetyl chloride at 0 - 5° C over 1.5 to 2 hours. The reaction mixture was stirred at 0 - 5°C for 30 minutes then it was poured carefully onto a cold (0°C) solution of saturated sodium bicarbonate. The organic layer was separated and the water layer was extracted with dichloromethane (3 X 20 mL). The combined organic layers were washed with saturated sodium bicarbonate (3 X 20 mL) and brine (20 mL), and was dried over sodium sulfate. Following filtration, the solvents were removed in vacuo to give 32.1 g of crude product which was purified by Kugelrohr distillation or filtration through a short silica gel column (eluent hexanes: EtOAc 3:1). The purified product consisted of a 3:1 mixture of *trans : cis* isomers.
¹H NMR (CDCl₃)δ 2.09 (s, 0.75H), 2.10 (s, 2.25H), 3.22 (m, 2H), 4.54 (m, 2H), 5.68 (m, 1H), 6.64 (d, 0.25H, J=4.2Hz), 6.72 (d, 0.75H, J=4.1Hz), 7.45 (dd, 2H, J=7.6Hz), 7.55 (t, 1H, J=7.3 Hz), 8.05 (dd, 2H, J=7.4 Hz).
¹³C NMR (CDCl₃) *trans* isomer δ 20.7, 37.3, 65.8, 83.1, 98.9, 128.2, 129.4, 129.5, 133.0, 165.7, 169.6.
*Cis* isomer δ 20.7, 37.9, 67.5, 84.4, 99.1, 128.2, 129.4, 129.5, 133.0, 165.7, 169.5.

The *trans* compound can be isolated by washing the mixture with ethanol and removing the solvent *in vacuo.* m.p. 67 - 68°C
¹H NMR (DMSO- d₆) δ 2.10 (s, 3H), 3.18 (d, 1H), 3.39 (dd, 1H), 4.48 (d, 2H), 5.67 (d, 1H), 6.65 (d, 1H), 7.56 (m, 2H), 7.70 (m, 1H), 7.98 (m, 2H).

### EXAMPLE 3

### TRANS-2-BENZOYLOXYMETHYL-5-ACETOXY-1,3-OXATHIOLANE

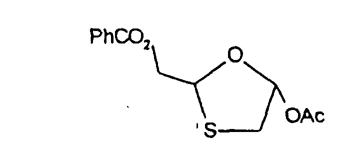

A solution benzoyloxyacetaldehyde (ca. 465 g) in toluene (ca. 21) was treated with 1,4 -dithiane-2,5-diol (227.2 g, 1.49 mol) and the suspension was stirred and heated at 75 - 80°C for 5 hours. The mixture was cooled to 25 - 30°C and the remaining solid (unreacted dithiane) was collected by filtration.
The filtrate was diluted with pyridine (362 mL, 4.48 mol) and the resulting solution was cooled to 0 - 5°C. Acetyl chloride (316.8 mL, 4.46 mol) was added during 20 minutes such that the temperature was maintained in the range 0 - 20° and the mixture was then stirred at 27 - 30°C for 30 minutes. The reaction mixture was cooled to 5 - 10°C and 1M hydrochloric acid (1.9l, 1.9 mol) was added such that the temperature was maintained in the range 5 - 20°C. The phases were separated and the aqueous phase was extracted with toluene (1.9l). The combined organic phases were washed with saturated aqueous sodium bicarbonate solution (2.8l). The organic was concentrated *in vacuo* at ca. 45°C to an oil. This oil was diluted with ethanol (IMS, 3l) and was reconcentrated to an oil. This was treated with ethanol (IMS, 2.5l), the mixture stirred at 0 - 5°C for 3.5 hours and the resultant suspension was stored at 2°C for 17 hours. The product was isolated by filtration to give the title compound as a cream coloured solid, 147.3 g; m.p. 67 - 68°C;
¹H NMR (DMSO-d₆): δ 7.98 (m, 2H, aromatic), 7.70 (m, 1H, aromatic), 7.56 (m, 2H, aromatic), 6.65 (d, 1H, C₅-H), 5.67 (d, 1H, C₂-H), 4.48 (d, 2H, CH₂-C₂), 3.39 (dd, 1H, C₄-H₂), 3.18 (d, 1H, C₄-H₂), 2.10 (s, 3H, OCO-CH₃).

### EXAMPLE 4

### CIS AND TRANS 2-BENZOYLOXYMETHYL-5-(3',4'-DICHLOROBENZOYLOXY)-1,3-OXATHIOLANE

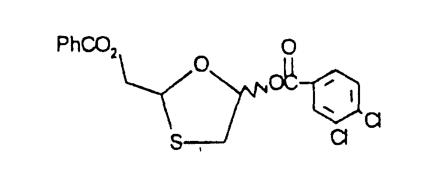

A mixture of *cis* and *trans* 2-benzoyloxymethyl-5-hydroxy-1,3-oxathiolane (as prepared in example 1) (8.99 g, 39.8 mmol) was reacted with 8.3 g (39.6 mmol) of 3,4-dichlorobenzoylchloride in dichloromethane (30 mL) and pyridine (9.6 mL) as described in Example 2 to yield 4.86 g of the desired compounds in 1:1 ratio .
¹H NMR (CDCl₃) δ 3.35 (m, 2H), 4.55 (m, 2H), 5.72 (m, 1H), 6.80 (m, 0.5H), 6.93 (m, 0.5H), 7.26 (d, 1H, J=6.8Hz), 7.38 (m, 1H), 7.82 (m, 2H)
¹³C NMR (CDCl₃) δ, 37.4, 38.1, 65,9, 67.3, 83.5, 84.9, 100.1, 100.4, 128.5, 129.5, 129.6, 129.7, 129.8, 130.7, 131.7 ,133.1, 133.3, 133.4, 138.3, 163.5, 163.6 166.0, 166.2.

### EXAMPLE 5

### CIS AND TRANS 2-BENZOYLOXYMETHYL-5-TRIMETHYLACETOXY -1,3-OXATHIOLANE

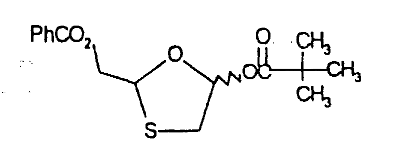

A mixture of *cis* and *trans* 2-benzoyloxymethyl-5-hydroxy-1,3-oxathiolane (8.9 g, 39.6 mmol) (as prepared in example 1) was reacted with 14.6 mL (118.8 mmol) of trimethylacetylchloride in dichloromethane (35 mL) and pyridine (9.6 mL) as described in example 2 to yield 7.94 g of the desired compound in 1:1 ratio .
¹H NMR (CDCl₃) δ 1.20 (s, 9H), 3.16 (dd, 1H), 3.30 (m, 1H), 4.50 (m, 2H), 5.60 (m, 1H), 6.65 (d, 0.5H, J=4.7Hz), 6.68 (d, 0.5H, J=4.1Hz), 7.43 (m, 2H), 7.53, (m, 1H), 8.05 (d, 2H, J=7.8Hz).
¹³C NMR (CDCl₃) δ, 26.6, 37.3, 37.9, 38.4, 38.7, 66.0, 68.1, 83.1, 84.5, 99.2, 99.7, 128.5, 129.7, 129.8, 129.9, 133.3, 166.2, 177.4.

### EXAMPLE 6

### CIS AND TRANS 2-BENZOYLOXYMETHYL-5-(2',2',2'-TRICHLOROETHOXYCARBONYLOXY)-1,3-OXATHIOLANE

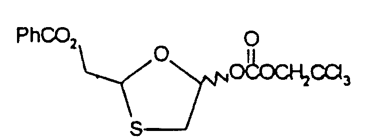

A mixture of *cis* and *trans* 2-benzoyloxymethyl-5-hydroxy-1,3-oxathiolane 4.47 g (19.8 mmol) (as prepared in example 1) was reacted with 8.2 mL (59.4 mmol) of 2,2,2-trichloroethylchloroformate in pyridine (4.8 mL) and dichloromethane (150 mL) as described in example 2 to give 6.0 g of the title compound in 2:1 ratio .
¹H NMR (CDCl₃) δ 3.32 (m, 2H), 4.74 (m, 2H), 4.80 (s, 2H), 5.71 (m, 1H), 6.55 (brs, 0.33H), 6.62 (d, 0.66H), 7.41 (dd, 2H), 7.53 (t, 1H), 8.00 (d, 2H).
¹³C NMR (CDCl₃) *trans* isomer δ 37.0, 65.6, 77.0, 83.6, 93.8, 102.9, 128.4, 129.6, 129.7, 133.3, 133.4, 153.1, 165.8.
*Cis* isomer δ 37.6, 67.6, 85.0, 93.9, 102.9, 128.4, 129.6, 129.7, 133.3, 152.6, 165.8.

### EXAMPLE 7

### CIS AND TRANS-2-BENZOYLOXYMETHYL-5-ETHOXYCARBONYLOXY -1,3- OXATHIOLANE

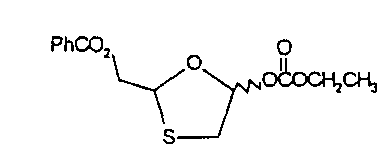

A mixture of *cis* and *trans* 2-benzoyloxymethyl-5-hydroxy-1,3-oxathiolane 1.49 g (6.6 mmol) (as prepared in Example 1) was reacted with ethylchloroformate 1.3 mL (13.2 mmol) and pyridine (3.3 mL) as described in example 2 to give 1.51 g of the title compound.
¹H NMR (CDCl₃) δ 1.19 (t, 3H, J=6.5 Hz), 3.16 (m, 2H), 4.10 (q, 2H, J=6.5), 4.43 (m, 2H), 5.61 (m, 1H), 6.45 (d, 0.33H, J=3.5 Hz), 6.54 (d, 0.66H, J=4.1, Hz), 7.36 (dd, 2H, J=7.4 Hz), 7.46 (t, 1H, 7.6 Hz), 7.95 (d, 2H, J=7.2 Hz).
¹³C NMR (CDCl₃) *trans* δ 13.4, 36.7, 63.7, 65.5, 82.9, 101.6, 129.3, 129.4, 128.1, 132.8, 153.4, 165.6
*cis* δ 13.4, 37.3, 63.7, 67.0, 84.3, 101.7, 129.3, 129.4, 128.1, 132.8, 153.3, 165.6.

### EXAMPLE 8

### CIS AND TRANS-2-CARBOETHOXY-5-HYDROXY-1,3-OXATHIOLANE

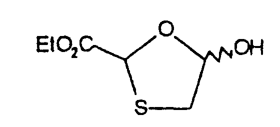

A mixture of the mercaptoacetaldehyde dimer (5.1 g, 33.65 mmol), ethyl glyoxylate (8.58 g, 2.5 equivalents), and a magnetic stirring bar were placed in a round bottom flask. After flushing with argon, the mixture was heated with a heat gun with stirring until a pale yellow oil was obtained (about 3 to 5 minutes). The crude product was then purified by flash column chromatography (45% ethyl acetate in hexanes) to give the desired material (7 g, 58% yield) as a mixture of isomers epimeric at C-5.
Note:Ethyl glyoxylate was prepared according to the procedure reported by T.R. Kelly and coworkers [Synthesis, 544 (1972)].
¹H NMR (CDCl₃) δ 1.30 (m, 3H), 3.11 (m, 2H), 4.21 (m, 2H), 5.56 (s, 0.5H), 5.59 (s, 0.5H), 5.89 (m, 0.5H), 6.02 (m, 0.5H)
¹³C NMR (CDCl₃) δ 13.7, 38.2, 40.0. 61.8, 62.5, 77.7, 79.8, 101.3, 103.0, 170.1.

### EXAMPLE 9

### CIS AND TRANS 2-CARBOETHOXY-5-METHOXYCARBONYLOXY-1,3-OXATHIOLANE

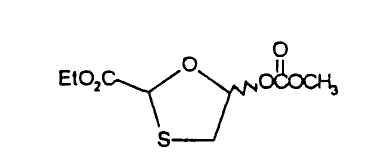

To a cold (-25°C) stirred solution of the crude hydroxy compound (10 g) (as prepared in example 8) and pyridine (9.1 mL, 0.113 mmol) in dry dichloromethane (20 mL) under argon was added methyl chloroformate (8.7 mL, 0.113 mmol) slowly over a period of 5 minutes. Upon completion of addition, the cooling bath was removed and the reaction mixture was stirred for 3 hours. Water (20 mL) was added to the mixture and stirring was continued for another 5 minutes. The resulting mixture was diluted with dichloromethane (150 mL) and washed with 1 M HCl (3 x 40 mL), saturated sodium bicarbonate (40 mL), brine (40 mL), and then was dried over sodium sulphate. Removal of the solvent under reduced pressure gave 9.2 g of the crude product which was purified by flash column chromatography (25% ethyl acetate in hexanes). The trans carbonate was obtained in pure form (4.02 g), as indicated by the NMR spectrum of the material. However, an additional 1.65 g was obtained and found to be contaminated with the trans compound (20%) as determined by NMR integration.
¹H NMR (CDCl₃) *trans* δ 1.29 (t, 3H, J=7.1 Hz), 3.24 (d, 1H, J=11.9 Hz), 3.44 (d of d, 1H, J=4.1, 11.9 Hz), 3.82 (s, 3H), 4.24 (q, 2H, J=7.1 Hz), 5.65 (s, 1H), 6.69 (d, 1H, J=4.1 Hz).

### EXAMPLE 10

### CIS AND TRANS 2-CARBOETHOXY-5-ACETOXY-1,3- OXATHIOLANE

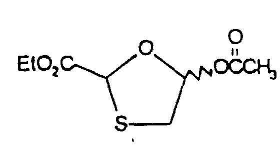

To a cold (0°C) stirred solution of the hydroxy compound (6.0 g, 33.7 mmol) obtained as in Example 8, and pyridine (5.45 mL) in dry dichloromethane (25 mL) under argon was added slowly acetyl chloride (3.60 mL, 1.5 equivalents) over a period of 20 minutes. The resultant mixture was stirred for 1 hour and 45 minutes. Analysis of the reaction mixture by TLC showed that all starting material was consumed. The excess acetyl chloride was quenched by the addition of methanol (2 mL). The mixture was diluted with ether (150 mL) and was washed with water (3 X 40 mL), 1 M HCL (40 mL) saturated sodium bicarbonate (40 mL), and then dried over anhydrous sodium sulphate. Removal of the solvent under reduced pressure gave 4.67 g of the crude product. The combined aqueous washings was extracted with ethyl acetate (3 x 50 mL). Concentration of the extract provided another 1 g of the crude product. The combined crude product was subjected to flash column chromatography (25% ethyl acetate in hexanes) to afford 2.2 g of the *trans* acetate (the less polar component). The corresponding *cis* acetate was obtained as a mixture (1.71 g) contaminated with small amount of the *trans* isomer.
¹H NMR (CDCl₃) *trans* δ 1.30 (t, 3H, J=7.1 Hz), 2.10 (s, 3H), 3.17 (d, 1H, J=11.8 Hz), 3.44 (dd, 1H, J=9, 11.8 Hz), 4.25 (q, 2H, J=7.1 Hz), 5.65 (s, 1H), 6.80 (d, 1H, J=4.0 Hz).

### EXAMPLE 11

### TRANS-2-HYDROXYMETHYL-5-ACETOXY-1,3-OXATHIOLANE

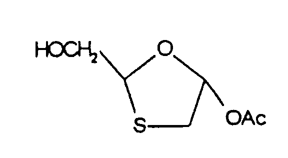

Sodium borohydride (27 mg, 0.708 mmol) was added to a magnetically stirred solution of *trans*-2-carboethoxy-5-acetoxy-1,3-oxathiolane (52 mg, 0.236 mmol) in methanol (1 mL) at 0°C under an argon atmosphere. The resultant solution was stirred for 25 minutes at 0°C. The reaction was quenched with 2 drops of saturated ammonium chloride solution followed by dilution with diethyl ether (4 mL). This mixture was stirred at room temperature for 15 minutes and then was dried over anhydrous magnesium sulphate. The drying agent was removed by suction filtration and the filtrate was concentrated under reduced pressure. The crude product obtained was subjected to column chromatography (50% EtOAc-hexane) to afford 21 mg (50%) of the title compound.
¹H NMR (CDCl₃) δ: 2.11 (s, 3H), 2.22-2.35 (m,1H), 3.16 (d, 1H, J=11.6 Hz), 3.33 (d of d, 1H, J=4.2, 11.6 Hz), 3.70-3.92 (m,2H), 5.46-5.54 (m,1H), 6.69 (d, 1H, J=4.2 Hz).

### EXAMPLE 12

### CIS AND TRANS-2-BENZOYLOXYMETHYL-5-METHOXYCARBONYLOXY -1,3-OXATHIOLANE

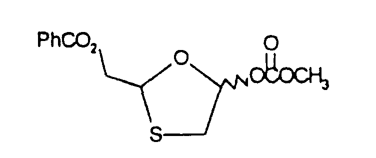

A solution of 17.93 g (0.118 mmol) of mercaptoacetaldehyde dimer and 38.70 g (0.236 mmol) of benzoyloxyacetaldehyde in 57.3 mL (3 eq) of pyridine was heated until all the solid dissolved. After cooling, 300 mL of anhydrous methylene chloride were added and the mixture was cooled at 0°C for ca. 30 minutes. To this solution at 0°C, was slowly added a solution of methylchloroformate (57.3 mL, 0.71 mmol) in 80 mL of methylene chloride. The mixture was stirred for 12 hrs and diluted with ca. 200 mL of methylene chloride and washed several times with brine to remove pyridinium salt and then the organic layer was washed with water. The organic layer was dried over magnesium sulphate at 0°C and then filtered. Residual pyridine was removed *in vacuo* and the organic residue was purified by flash chromatography using hexanes:ethyl acetate (2:1) as eluent to yield a mixture of 2:1 *trans:cis* carbonates (56.3 g, 80%).
¹H NMR (CDCl₃) δ 3.25 (d, 1H J=3.1 Hz), 3.30 (dd, 1H, J=3:1 Hz), 3.73 (s, 0.1 H), 3.75 (s, 2 H), 4.47 (m, 2H), 5.66 (m, 2H), 6.50 (brd, 0.33H), 6.56 (d, 0.66H, J=3.81 Hz), 7.38 (d, 2H, J=7.3 Hz), 7.51 (t,1H, J=7.2 Hz), 8.00 (dd, 2H, J=7.3 Hz).
¹³C NMR (CDCl₃) *trans* isomer δ 36.9, 54.6, 65.7, 83.2, 101.9, 126.3, 128.4, 128.5, 133.1, 154.3, 166.0,
*cis* isomer δ 37.6, 54.6, 67.3, 84.7, 102.1, 126.3, 128.4, 128.5, 133.1, 154.3, 165.9.

### EXAMPLE 13

### CIS-2-CARBOETHOXY-5-(URACIL-1'-YL)-1,3- OXATHIOLANE

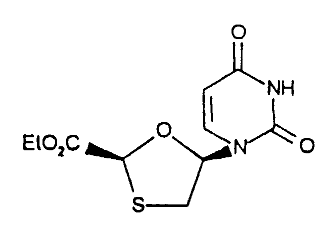

To a stirred solution of the acetate (468 mg, 2.13 mmol) as obtained in example 10 and *bis*-silylated uracil (653 mg, 1.2 equivalents) in dichloromethane under argon was added trimethylsilyl iodide (303 µL, 1 equivalent). The resultant yellow solution was stirred for 6.5 hours at room temperature. As indicated by TLC (silica gel), all starting material was consumed. The reaction was quenched with a 1:1 mixture of saturated solutions of sodium bicarbonate and sodium thiosulphate (5 mL).
After 15 minutes of stirring, the mixture was transferred to a separatory funnel with the aid of more dichloromethane (30 mL). The aqueous phase was removed and the organic layer was washed with saturated sodium bicarbonate-sodium thiosulphate solution 1:1, 10 ml, water (10 mL), brine (10 mL), and then was dried over anhydrous sodium sulphate. Removal of the solvent under reduced pressure gave the crude product which was triturated with a 1:1 mixture of ethyl acetate-hexane (about 10 mL). The precipitate was collected by suction filtration and then was dried under vacuum to afford 346 mg (60%) of the nucleoside as a crystalline white solid. Analysis of the triturate by TLC showed that it contained the desired product but no attempt was made to isolate these compound. The 300 MHz proton NMR spectrum of the product indicated that it consisted of one isomer only.
¹H NMR (CDCl₃) δ 1.34 (t, 3H, J=7.2 Hz), 3.16 (dd, 1H, J=7.7 Hz), 3.42 (dd, 1H, J=4.8, 12.0 Hz), 4.29 (q, 2H, J=7.1 Hz), 5.82 (dd, 1H, J=2.1, 8.2 Hz), 6.46 (dd, 1H, J=4.7, 7.5 Hz), 8.32 (d, 1H, J=8.2 Hz), 8.53 (brs, 1H) ¹³C NMR (CDCl₃) δ 14.2, 35.4, 62.8, 78.1, 89.5, 103.5, 140.8, 151.1, 163.9, 170.9

### EXAMPLE 14

### CIS-2-CARBOETHOXY-5-(URACIL-1'-YL)-1,3- OXATHIOLANE

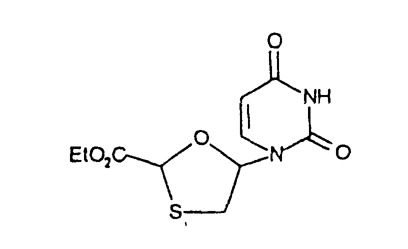

To a stirred solution of a mixture of the *cis* and *trans* carbonates (Example 9)(4:1 by NMR) (60 mg, 0.254 mmol) and silylated uracil (78 mg, 1.2 equivalents) in dry dichloromethane (1.5 mL) under argon was added TMS-I (36 µL, 1.0 equivalent). The resultant light yellow suspension was stirred at room temperature for 80 minutes at which time all starting material was consumed (TLC). The reaction was quenched with a 1:1 mixture (1 mL) of saturated sodium bicarbonate and sodium thiosulphate followed by dilution with dichloromethane (4 mL). The mixture was stirred until a colorless biphasic suspension was obtained. This suspension was transferred to a separatory funnel with the aid of more dichloromethane (25 mL) and was washed with saturated sodium thiosulphate, brine, and then was dried over anhydrous sodium sulphate. Removal of the solvent *in vacuo* provided the crude product. Trituration of the crude product with a 1:1 mixture (3 mL) of dichloromethane and ethyl acetate gave a white solid which was collected by suction filtration and was dried under vacuum (31 mg). The NMR spectrum of this material indicated that it consisted of the *cis* nucleoside only. The triturate was concentrated under reduced pressure and then was subjected to flash column chromatography (1:1 ethyl acetate-dichloromethane) to produce another 8 mg of white solid. The NMR spectrum of this substance showed that it was a 2.5:1 mixture of the *cis* and *trans* nucleosides favouring the *cis* isomer. The total yield of this reaction was 58% and the stereoselectivity was about 13:1 in favour of the *cis* isomer which displayed the same physical data as reported in Example 13.

### EXAMPLE 15

### CIS-2-HYDROXYMETHYL-5-(URACIL-1'-YL)-1,3- OXATHIOLANE

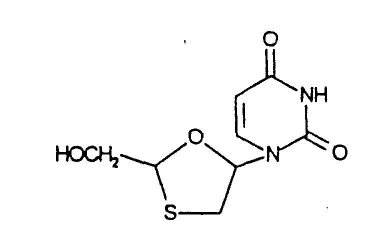

To a stirred solution of the condensation product obtained in examples 13 or 14, (33 mg, 0.107 mmol) in a solvent mixture (2:1) of dichloromethane-methanol (1.5 mL) at room temperature under argon was introduced sodium borohydride (8 mg, 2 equivalents). The resulting mixture was stirred for 1 hour. Analysis of the reaction mixture by TLC indicated that substantial amount of starting material was present. More hydride (approx. 10 mg) was added and stirring was continued for another 1.5 hours. The excess hydride was quenched by addition of one drop of saturated ammonium chloride solution. After dilution with tetrahydrofuran (3 mL), the gelatinous mixture was stirred for 30 minutes. The inorganic salt was removed by suction filtration through a pad of celite. Concentration of the filtrate under reduced pressure provided the crude product which was subjected to column chromatography (100% ethyl acetate, silica gel) to afford the desired alcohol (25 mg, 90%) as a white solid.
The 300 MHz proton NMR spectrum of the compound thus obtained was found to be identical to that prepared according to different procedures. Thus, the stereochemistry of the nucloside generated by this new route was established.
¹H NMR (DMSO) δ 3.23 (d of d, 1H, J=4.4, 12.0 Hz), 3.45 (d of d, 1H, J=5.6, 11.9 Hz), 3.75 (d, 2H, J=4.4 Hz), 5.20 (t, 1H, J=4.4 Hz), 5.36 (brs, 1H), 5.65 (d of d, 1H, J=2.1, 8.2 Hz), 6.21 (t, 1H, J=5.1 Hz), 7.92 (d, 1H, J=8.2 Hz).
¹³C NMR (DMSO)= δ 36.02, 62.54, 85.91, 86.48, 101.82, 141.05, 150.63, 163.71.

### EXAMPLE 16

### CIS-2-CARBOETHOXY-5-(N-ACETYLCYTOSIN-1'-YL)-1,3-OXATHIOLANE

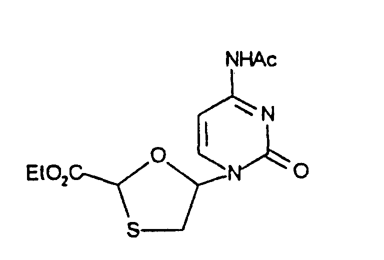

To a stirred suspension of N-acetylcytosine (237 mg, 1.40 mmol) in dichloromethane (2.5 mL) containing 2,6-lutidine (326 µL, 1.40 mmol) was added slowly trimethylsilyl trifluoromethanesulphonate (540 µL, 3.07 mmol). The resultant mixture was stirred for 15 minutes to give a homogeneous solution. A mixture of *cis* and *trans*-2-carboethoxy-5-methoxycarbonyloxy-1,3-oxathiolane (example 9) (300 mg, 1.27 mmol), dissolved in dichloromethane (2 mL), was introduced to the above solution followed by the addition of iodotrimethylsilane (181 µL, 1.27 mmol). The reaction mixture was kept at room temperature for 1 hour and 40 minutes. Water (2 mL), saturated sodium thiosulphate (4 mL) and dichloromethane (6 mL) were added to quench the reaction. The resulting mixture was stirred vigorously for 10 minutes and then was transferred to a separatory funnel with the aid of more dichloromethane (30 mL). The aqueous phase was removed and the organic phase was washed successively with saturated sodium thiosulphate (10 mL), water (10 mL), 1 M hydrochloric acid (10 mL), saturated sodium bicarbonate (10 mL), brine (10 mL), and then was dried (sodium sulphate). The solvent was evaporated under reduced pressure to give the crude product as a light yellow solid (395 mg). The ¹H NMR spectrum of this material indicated that a 7.5:1 (in favour of the *cis* isomer) mixture of the expected coupling products was obtained. This material was triturated with a mixture of dichloromethane (1.5 mL) and a solution of ethyl acetate-hexane (1:1) (6 mL). The white solid formed was collected by suction filtration and was dried under vacuum to afford 262 mg (63% yield) of the desired product as a white powder. The ¹H NMR spectrum of the substance indicated an isomeric purity of greater than 95%. The triturate was concentrated and then was subjected to flash column chromatography (5% MeOH-EtOAc) to provide another 58 mg (14% yield) of the nucleosides as a 1:1 mixture of the *cis* and *trans* isomers (¹H NMR). The title compound displayed the following spectral characteristics:
¹H NMR (CDCl₃) δ 1.34 (t, J=7.1 Hz), 2.28 (s, 3H), 3.23 (d of d, 1H, J=12.3, 6 Hz), 3.68 (d of d, 1H, J=12.4, 4.8 Hz), 4.31 ( 2H, J=7.1 Hz), 5.56 (s, 1H), 6.43 (t, 1H, J=5.2 Hz), 7 7 (d, 1H, J=7.5 Hz), 8.76 (br. d, 1H, J=7.4 Hz), 8.30-9.00 (unresolved m, 1H).

### EXAMPLE 17

### CIS-2-CARBOETHOXY-5-(CYTOSIN-1'-YL)-1,3- OXATHIOLANE

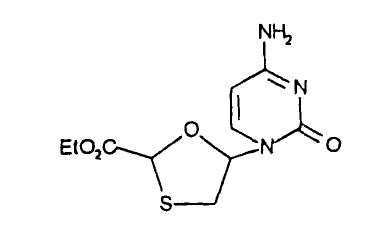

A mixture of *cis*-2-carboethoxy-5-(N4'-acetylcytosin-1'-yl)-1,3-oxathiolane (example 16) (20 mg, 0.061 mmol) in ethanol (1 mL) containing trifluoroacetic acid (9.4 µL, 0.25 mmol) was refluxed under argon for 3 hours and 10 minutes. On cooling to room temperature, a crystalline white solid was formed. This solid was collected by suction filtration and was dried under vacuum to afford 15 mg (86%) of the desired product. The title compound displayed the following spectral characteristics: ¹H NMR (DMSO) δ 1.23 (t, 3H, J=7.1 Hz), 3.32 (d of d, 1H, J=12.4, 5.2 Hz), 3.63 (d of d, 1H, J=12.3, 5.2 Hz), 4.21 (q, 2H, J=7.1 Hz), 5.80 (s, 1H), 6.08 (d, 1H, J=7.7 Hz), 6.32 (t, 1H, J=5.1 Hz), 8.19 (d, 1H, J=7.7 Hz), 8.35 (brs, 1H), 9.12 (brs, 1H).

### EXAMPLE 18

### CIS-2-HYDROXYMETHYL-5-(CYTOSIN-1'-YL)-1,3- OXATHIOLANE (BCH-189)

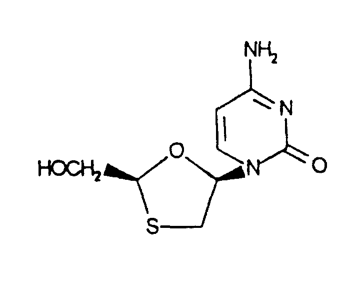

To a stirred suspension of *cis*-2-carboethoxy-5-(cytosin-1'-yl)-1,3-oxathiolane (Example 17) (36 mg, 0.125 mmol) is ethanol at 0°C under argon was added sodium borohydride (9.5 mg, 0.250 mmol). The resultant mixture was stirred for 2 hours 30 minutes at (0°C to RT). The reaction was quenched by the addition of one drop of concentrated ammonium hydroxide, followed by dilution with methanol (1 mL). After the mixture had been stirred for 15 minutes, the solvent was removed under reduced pressure. The crude product thus obtained was sybjected to column chromatography (25% MeOH-EtOAc) to afford 26 mg (85%) of the desired product. The title compound displayed spectral characteristics identical to that reported for BCH-189.

### EXAMPLE 19

### CIS AND TRANS 2-BENZOYLOXYMETHYL-5-(CYTOSIN-1'-YL)-1,3-OXATHIOLANE

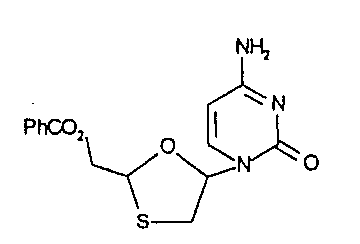

To a solution maintained at 0°C of 2.14 g (7.2 mmol) of carbonate (as in example 7) in 10 mL of freshly distilled 1,2-dichloroethane was added 0.37 g (0.36 mmol) of fused ZnCl₂ and 2.7 mL (2.7 mmol) of TiCl₄. After stirring for 5 minutes a solution of silylated cytosine (from 1 g of cytosine silylated with 1,1,1,3,3,3,-hexamethyldisilazane) in 25 mL of freshly distilled 1,2-dichloroethane was added via a canula (10 -15 min.) The reaction was allowed to warm to RT (3 hours) and stirring continued for 11 hours followed by a short reflux (20 min). The solution was then cooled and quenched with saturated sodium bicarbonate (30 mL).

After stirring for 15 min. the two phase solution was separated and the organic layer together with the emulsion was filtered through a celite. The aqueous layer was extracted (3 X 20 mL) with CH₂Cl₂ and the combined organic layers were washed with brine, separated and dried over MgSO₄. The oil obtained from the organic layer, by evaporation of the solvents in vacuo, was purified by chromatography on silica gel using gradient elution (1:1 hexanes:EtOAc - 9:1 EtOAc: MeOH) to yield 1.32 g of *trans* and *cis* isomers (*trans*/*cis* = 3.5/5 as determined by ¹H NMR).
Spectral properties were identical to those reported earlier.
By varying the amount and the nature of the Lewis acid the yield and the ratio of the *trans* to *cis* isomers were as follows:

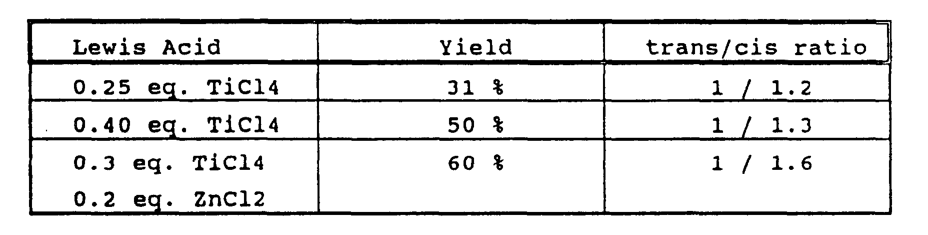

### EXAMPLE 20

### CIS AND TRANS 2-BENZOYLOXYMETHYL-5-(N4'-ACETYL CYTOSIN-1'-YL)-1,3-OXATHIOLANE

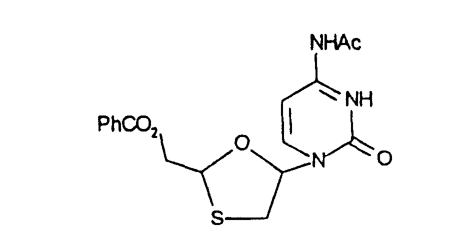

To a solution maintained at 0°C of 2.14 g (7.2 mmol) of *trans* 2-benzoyloxymethyl-5-acetoxy-1,3-oxathiolane (as in Example 3) in 10 mL of freshly distilled acetonitrite was added a solution of silylated cytosine N-acetylcytosine (from 1.37 g of N-acetylcytosine silylated with 1,1,1,3,3,3,-hexamethyldisilazane) in 25 mL of freshly distilled 1,2-dichloroethane via a canula (10 -15 min.) and 0.2 mL of iodotrimethylsilane. The reaction was allowed to stir at 0°C (3 hours) and stirring continued for 11 hours at RT. The solution was then cooled and quenched with saturated sodium bicarbonate (30 mL). After stirring for 15 min. the two phase solution was separated and the organic layer together with the emulsion was filtered through a celite. The aqueous layer was extracted (3 X 20 mL) with CH₂Cl₂ and the combined organic layers were washed with brine, separated and dried over MgSO₄. The oil obtained from the organic layer, by evaporation of the solvents *in vacuo*, was purified by chromatography on silica gel using gradient elution (1:1 hexanes:EtOAc - 9:1 EtOAc: MeOH) to yield 2.43 g of *trans* and *cis* isomers (*trans*/*cis* = 3/7 as determined by ¹H NMR). The physical properties are identical to those reported earlier.
Replacement of iodotrimethylsilane by trimethylsilyltriflate in dichloromethane at RT yielded 2.43 g of *trans* and *cis* isomers in 1:1 ratio as determined by ¹H NMR.

### EXAMPLE 21

### CIS-2-HYDROXYMETHYL-5-(CYTOSIN-1-YL)-1,3-OXATHIOLANE

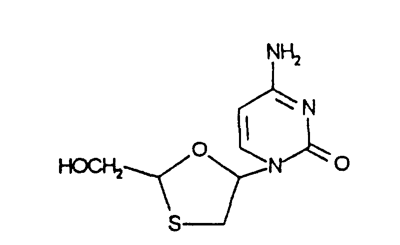

A suspension of *cis*-2-benzoyloxymethyl-5-(cytosin-1'-yl)-1,3-oxathiolane (200g, 0.54 mol) and Amberlite IRA 400 (OH) ion-exchange resin (600g) in IMS was stirred and heated to 60-65°C. The suspension was maintained at this temperature range for 1 hour, and filtered hot. The resin was washed with IMS at 60°C (200 mL).
The combined filtrates were filtered twice through celite J2 and the celite washed sequentially with IMS at 60°C (200 mL) and water at 50-60°C (100 mL).
The combined filtrates were distilled under atmospheric pressure to a volume of 500 mL. Absolute ethanol was added, and the distillation continued until a further 700 mL had been removed. The resultant suspension was allowed to cool, and then stirred overnight at 0-5°C. The suspension was filtered, the product washed with IMS at 0°C (2 x 25 mL), and dried overnight in vacuo at 45-50°C to give the title compound, 81.9 g.

### EXAMPLE 22.

### CIS- AND TRANS-2-ACETOXYMETHYL-5-ACETOXY-1,3-OXATHIOLANE

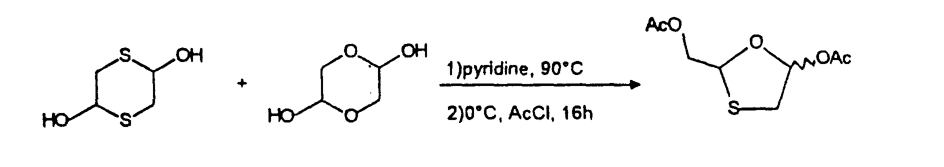

A mixture of glycoaldehyde (1.2g, 0.01 mol) and mercaptoacetaldehyde dimer (1.52g, 0.01 mol) in dry pyridine (20 ml) was heated at 90°C for 2 h. The clear solution was then cooled in an ice-bath to 0°C, followed by adding acetyl chloride (2.8 ml). The mixture was stirred at room temperature overnight (16 h), and poured into saturated aqueous NaHCO₃ solution (100 ml). The product was extracted into methylene chloride (3 x 100 ml), washed with water (2 x 100 ml), dried over MgSO₄ and filtered. The solvent was removed on an evaporator and the oily residue was purified on silica gel hexane:EtOAc 9:1 as eluant to give the product (2.8g) in 59% yield as a mixture of 1:1 *cis*:*trans* isomers.
¹H-NMR (300MHz, CDCl₃): δ in ppm
6.68 (d, 1H, H-5, trans-isomer, J=4.1 Hz)
6.61 (d, 1H, H-5, cis-isomer, J=4.4 Hz)
5.52 (m, 2H, H-2, cis and trans-isomers)
4.37 (dd, 1H, -CH₂OAc, cis-isomer, J=8.0 and 11.7 Hz)
4.26 (m, 2H, -CH₂OAc, trans-isomer)
4.13 (dd, 1H, -CH₂OAc, cis-isomer, J=4.1 and 11.8 Hz)
3.33 (dd, 2H, H-4, cis and trans isomers)
3.11 (dd, 2H, H-4, cis and trans-isomers)
2.11 (s, 3H, CH₃-)
2.08 (s, 3H, CH₃-)

### EXAMPLE 23.

### CIS- AND TRANS-2-BENZOYLOXYMETHYL-5-BENZOYL-1,3-OXATHIOLANE

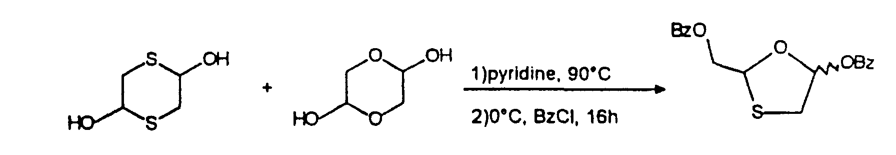

A mixture of glycoaldehyde (1,2g, 0.01 mol) and mercaptoacetaldehyde dimer (1.52g, 0.01 mol) in dry pyridine (20 ml) was heated at 90°C for 2h. The clear solution was then cooled in an ice-bath to 0°C, followed by adding benzoyl chloride (4.6 ml). The mixture was stirred at room temperature overnight (16h), and poured into saturated aqueous NaHCO₃ solution (100 ml). The product was extracted into methylene chloride (3 x 100 ml), washed with water (2 x 100 ml), dried over MgSO₄ and filtered. The solvent was removed on an evaporator and the oily residue was purified on silica gel using hexane:EtOAc 9:1 as eluant to give the product (3.65g) in 53% yield as a mixture of 1:1 cis and trans isomers.
¹H-NMR (300 MHz, CDCl₃): δ in ppm
8.05 (m, aromatic)
7.57 (m, aromatic)
7.45 (m, 4H, aromatic)
6.98 (d, 1H, H-5, trans-isomer, J=3.9 Hz)
6.90 (d, 1H, H-2, cis-isomer, J=3.0 Hz)
5.79 (t, 1H, H-2, trans-isomer, J=5.2 Hz)
5.74 (dd, 1H, H-2, cis-isomer, J=4.9 and 7.3 Hz)
4.58 (m, 4H, -CH₂OBz, cis and trans-isomers)
3.45 (m, 2H, H-4, cis and trans isomers)
3.35 (m, 2H, H-4, cis and trans-isomers).

### EXAMPLE 24.

### CIS- AND TRANS-ETHYL 5-IODO-1,3-OXATHIOLAN-2-CARBOXYLATE

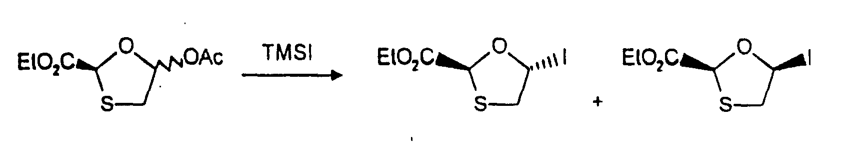

The starting material (21.5 mg, 0.0976 mmol, *cis:trans*=1:1) in dichloromethane-d₂ (0.6 mL) at -78°C under argon atmosphere wa treated with iodotrimethylsilane (0.014 mL, 0.0976 mmol). The slightly yellow solution was left at room temperature for two hours. The starting acetoxyoxathiolane compounds were completely converted to the iodo intermediates and trimethylsilyl acetate. The iodo compounds (in a 6.7:1 ratio of *cis* to *trans* isomer) are unstable to the moisture and had to be used without any purification.
¹H NMR (CD₂Cl₂): δ 0.00 (s, 9H), 1.05 (t, 3H, J=7.1Hz), 1.80 (s, 3H), 3.25-3.50 (m, 2H), 4.00 (q, 2H, J=7.1Hz), 5.43 (s, 0.13H), 5.48 (s, 0.87H) 6.64 (ddd, 0.13H, J=4.3, 2.9, 0.7 Hz), 7.00 (dt, 0.87H, J=4.0, 0.7 Hz);
¹³C NMR (CD₂Cl₂): δ 0.3, 2.5, 14.8, 23.5, 47.7, 48.2, 63.1, 65.5, 69.7, 81.6, 83.7, 168.6.

### EXAMPLE 25.

### CIS- AND TRANS-ETHYL 5-(6'CHLOROPURIN-9'-YL)-1,3-OXATHIOLAN-2-CARBOXYLATE; and CIS- AND TRANS-ETHYL 5-(6'CHLOROPURIN-7'-YL)-1,3-OXATHIOLAN-2-CARBOXYLATE

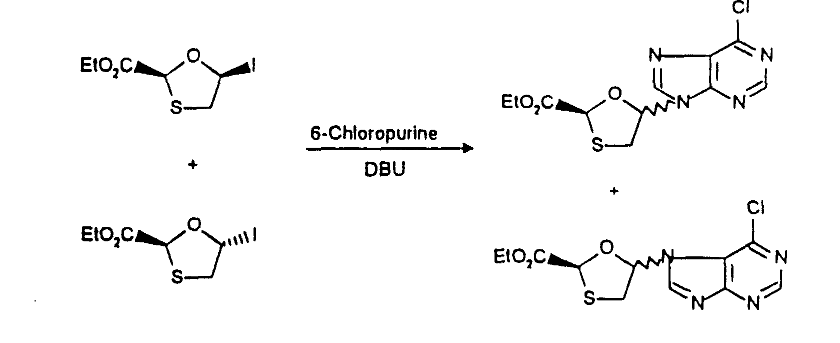

To the 6-chloropurine (15 mg, 0.0976 mmol) in dichloromethane-d₂ (0.15 mL) at room temperature under argon atmosphere was added 1,8-diazabicyclo[5,4,0] undec-7-ene (0.015 mL, 0.0976 mmol). The solution thus formed was added to the iodo intermediates prepared above in dichloromethane-d₂ at -78°C. The mixture was allowed to stay at room temperature for 4 hours and then diluted with dichloromethane (20 mL), washed with saturated aqueus sodium bicarbonate, 1N aqueous hydrogen chloride, water and brine, dried and concentrated. The residue was chromatographed on silica gel with ethyl acetate-dichloromethane to afford the N-9 linked isomers (11.6 mg, 38%, *cis*:*trans*=11:1) and N-7 linked isomers (4.4 mg, 14.3%, *cis:trans*=8.4:1).
¹H NMR for N-9 isomers (CDCl₃): δ 1.26 (t, 3H, J=7.1 Hz), 3.65 (m, 2H), 4.26 (q, 2H, J=7.1 Hz), 5.62 (s, 0.92H), 5.80 (s, 0.08H), 6.75 (t, 0.92H, J=5.4 Hz), 7.02 (dd, J=6.2, 2.0 Hz), 8.39 (s, 0.08H), 8.73 (s, 0.92 Hz), 8.89 (s, 0.92 Hz);
¹H NMR for the N-7 isomers (CDCl₃): δ 1.30 (t, 3H, J=7.1 Hz), 3.38 (d, 0.12H, J=12.5 Hz), 3.54 (dd, 0.88H, J=12.5, 4.5 Hz), 3.75 (dd, 0.88H, J=14.5, 4.5 Hz), 3.96 (dd, 0.12H, J=12.5, 4.5 Hz), 4.29 (q, 2H, J=7.1 Hz), 5.69 (s, 0.88H), 5.90 (s, 0.12H), 7.07 (t, 0.88H, J=4.5 Hz), 7.35 (d, 0.12H, J=4.5 Hz), 8.45 (s, 0.12H), 8.92 (s, 1H), 9.20 (s, 0.88H)

## Claims

1. A process for preparing an oxathiolane compound comprising:
reacting a mercaptocetaldehyde, wherein the mercaptoacetaldehyde is obtained from a mercaptoacetaldehyde dimer dissolved in an inert solvent, with a compound of formula R_{w}OCH₂CHO, under neutral or basic conditions, wherein R_{w} is hydrogen or R₁, wherein R₁ is a hydroxyl protecting group, to obtain a compound of formula (XIII)

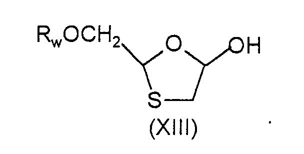

2. A process according to claim 1, further comprising converting the hydroxyl of the compound of formula (XIII) to a suitable leaving function L to obtain a compound of formula (XIV): 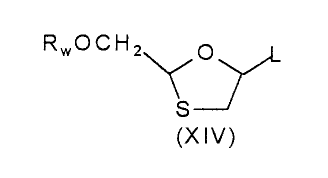 wherein L is alkoxy carbonyl; halogen; substituted or unsubstituted, saturated or unsaturated thiolates; substituted or unsubstituted, saturated or unsaturated selenino; substituted or unsubstituted, saturated or unsaturated aliphatic or aromatic ketones; or -OR; and
R is hydrogen; substituted or unsubstituted, saturated or unsaturated C₁₋₆ alkyl, substituted or unsubstituted, saturated or unsaturated alkenyl having up to 6 carbon atoms; substituted or unsubstituted aliphatic or aromatic acyl; substituted or unsubstituted, saturated or unsaturated alkoxycarbonyl; substituted or unsubstituted sulphonyl imidazolide; substituted or unsubstituted carbonyl imidazolide; substituted or unsubstituted aliphatic or aromatic amino carbonyl group; substituted or unsubstituted alkyl imidate group; substituted or unsubstituted, saturated or unsaturated phosphinoyl; or substituted or unsubstituted, aliphatic or aromatic sulphonyl group.

3. A process according to claim 2, further comprising reacting the compound of formula (XIV) with a silylated pyrimidine base, a silylated purine base or a silylated analogue of a pyrimidine or purine base, optionally in the presence of a Lewis acid, to produce a compound of formula (IX): 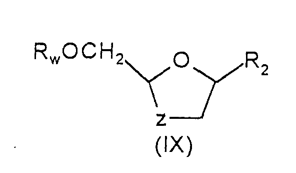 wherein Z is S; and R₂ is selected from the group consisting 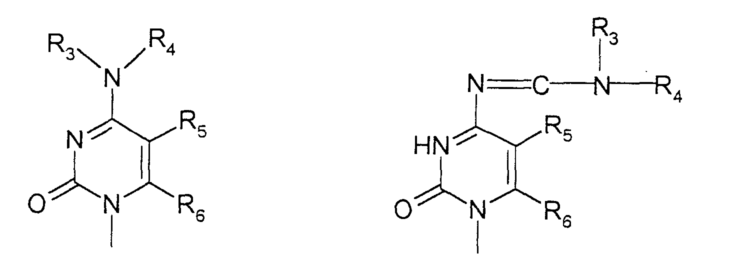 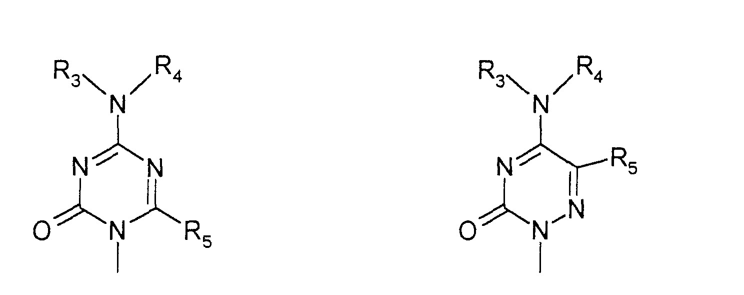 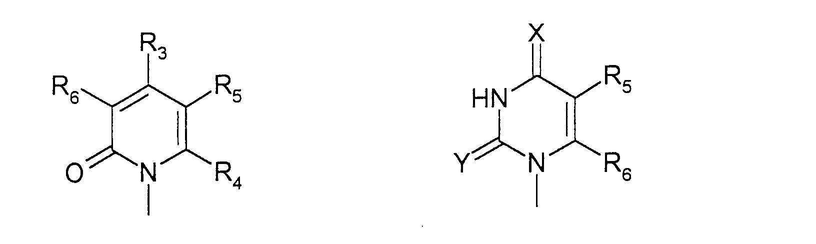 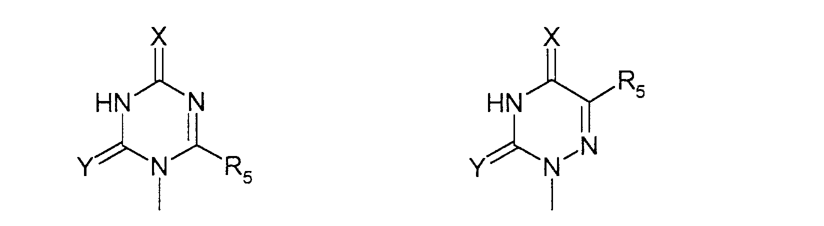 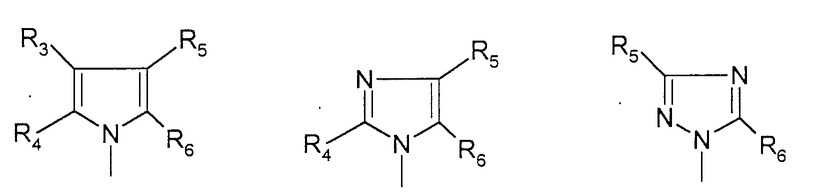 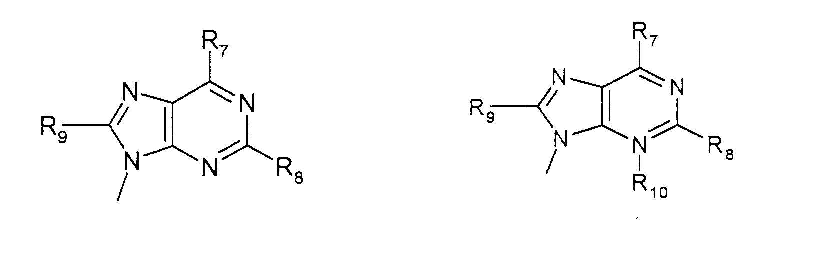 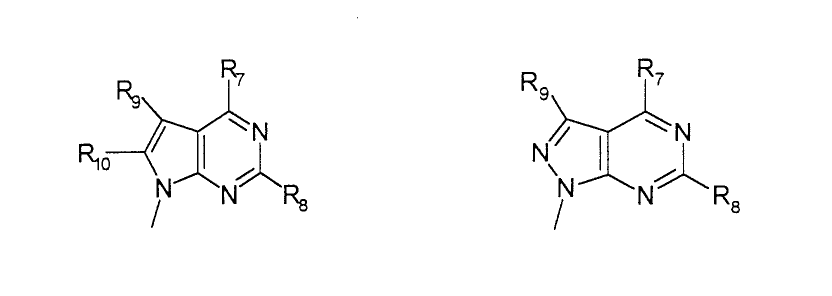 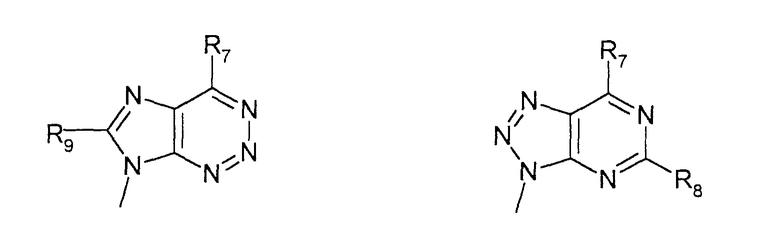 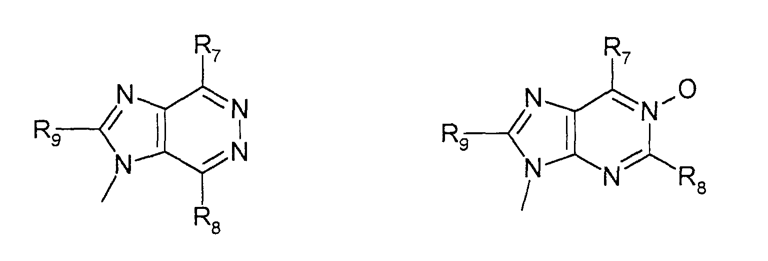 wherein:
X is oxygen or sulfur;
Y is oxygen or sulfur;
R₃ and R₄ are each independently selected from the group consisting of hydrogen, hydroxyl, amino, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyl or aracyl;
R₅ and R₆ are each independently selected from the group consisting of hydrogen, halogen, hydroxyl, amino, cyano, carboxy, carbamoyl, alkoxycarbonyl, hydroxymethyl, trifluoromethyl, thioaryl, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy;
R₇ and R₈ are each independently selected from the group consisting of hydrogen, hydroxy, alkoxy, thiol, thioalkyl, amino, substituted amino, halogen, cyano, carboxy, alkoxycarbonyl, carbamoyl, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy; and
R₉ and R₁₀ are each independently selected from the group consisting of hydrogen, hydroxy, alkoxy, amino, substituted amino, halogen, azido, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy.

4. A process according to claim 3, wherein the reaction with the compound of formula (XIV) is performed in the absence of a Lewis acid.

5. A process according to claim 3, wherein the reaction with the compound of formula (XIV) is performed in the presence of a Lewis acid.

6. A process according to any one of claims 3 to 5, further comprising oxidizing group Z of the compound of formula (IX) to give a compound of formula (IX) wherein group Z is S=O or SO₂.

7. A process according to any one of claims 3 to 6, further comprising acylating the compound of formula (IX) with acetic anhydride to obtain a compound of formula X: 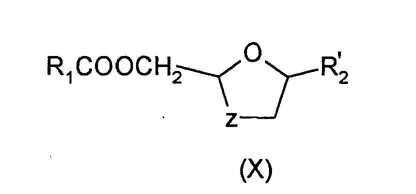 wherein R₁ is a hydroxyl protecting group and R'₂ is an acylated R₂ group.

8. A process according to claim 7, further comprising separating the compound of formula (X)into either its *cis* or *trans* isomers.

9. A process according to claim 7 or claim 8, further comprising hydrolyzing the acyl functions R₂' and R₁COOCH₂ of the compound of formula (X), to obtain a compound of formula I, a geometric isomer thereof, or an optical isomer thereof:

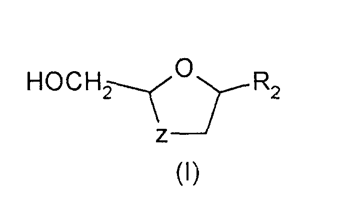

10. A process according to any one of claims 1 to 9, wherein R¹ is an acyl group having 1 to 16 carbon atoms, unsubstituted or substituted with a heteroatom, or is a silyl group.

11. The process according to claim 1, wherein the inert solvent is selected from the group consisting of pyridine, toluene and dimethyl sulfoxide.

12. A process for preparing an oxathiolane compound comprising:
reacting a mercaptoacetaldehyde with a compound having formula RyOOCCHO, wherein R_{y} is substituted or unsubstituted C₁₋₁₂ alkyl or substituted or unsubstituted C₆₋₂₀ aryl to obtain a compound of formula (XV)

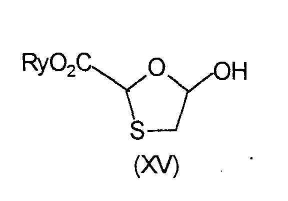

13. A process according to claim 12, further comprising converting the hydroxyl of the compound of formula (XV) to a suitable leaving function L to obtain a compound of formula (XVI): 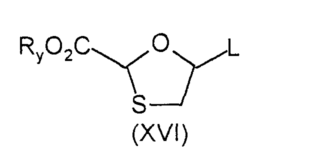 wherein L is alkoxy carbonyl; halogen; substituted or unsubstituted, saturated or unsaturated thiolates; substituted or unsubstituted, saturated or unsaturated selenino; substituted or unsubstituted, saturated or unsaturated aliphatic or aromatic ketones; or -OR; and
R is hydrogen; substituted or unsubstituted, saturated or unsaturated C₁₋₆ alkyl, substituted or unsubstituted, saturated or unsaturated alkenyl having up to 6 carbon atoms; substituted or unsubstituted aliphatic or aromatic acyl; substituted or unsubstituted, saturated or unsaturated alkoxycarbonyl; substituted or unsubstituted sulphonyl imidazolide; substituted or unsubstituted carbonyl imidazolide; substituted or unsubstituted aliphatic or aromatic amino carbonyl group; substituted or unsubstituted alkyl imidate group; substituted or unsubstituted, saturated or unsaturated phosphinoyl; or substituted or unsubstituted, aliphatic or aromatic sulphonyl group.

14. A process according to claim 13, further comprising reacting the compound of formula (XVI) with a silylated pyrimidine base, a silylated purine base, or a silylated analogue of a pyrimidine base or purine base, optionally in the presence of a Lewis acid, to produce a compound of formula (XVII) 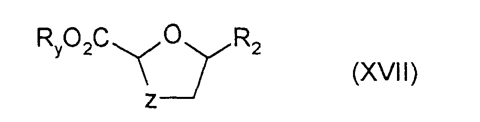 wherein
Z is S; and
R₂ is selected from the group consisting 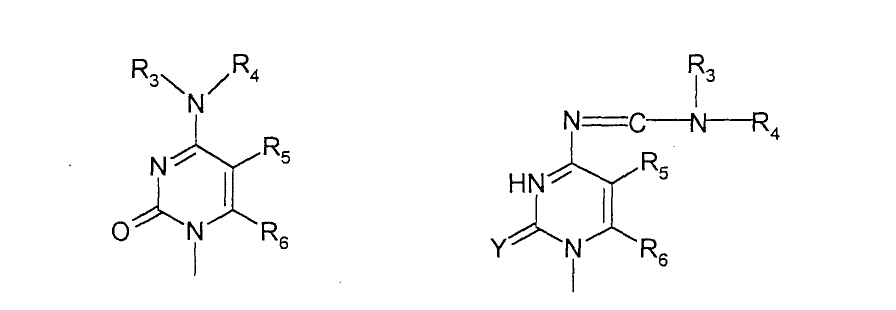 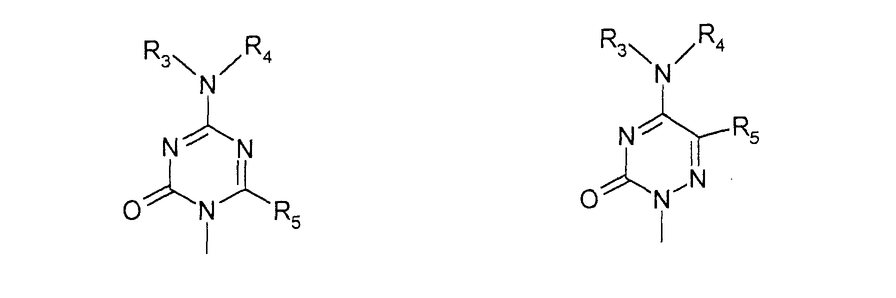 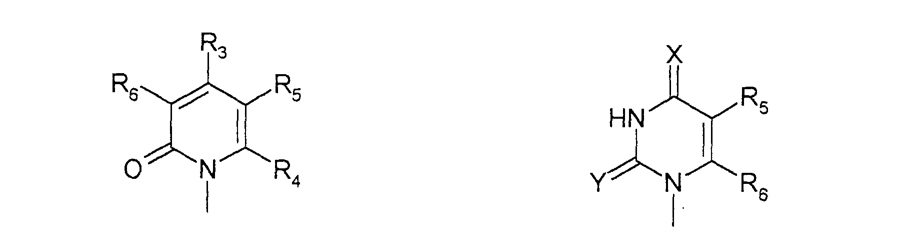 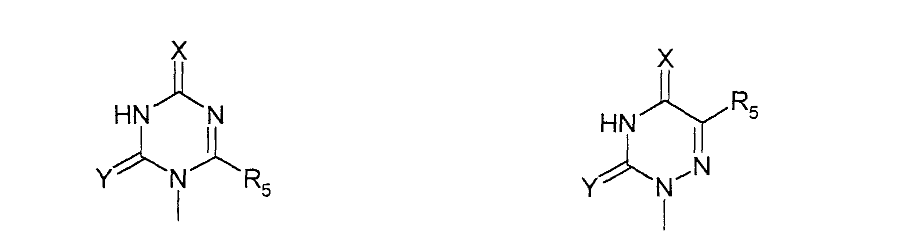 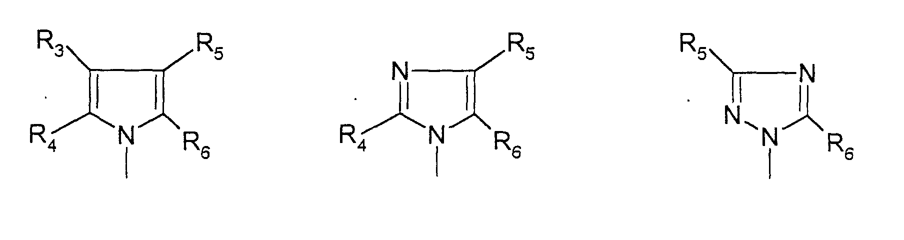 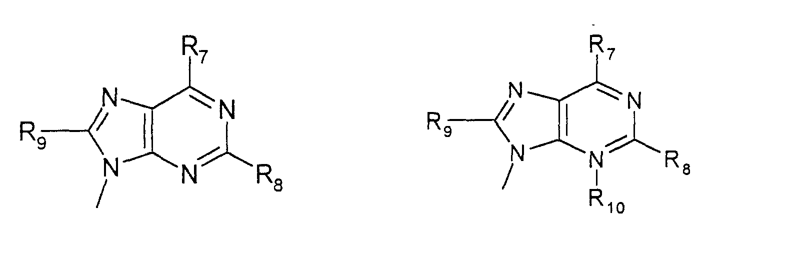 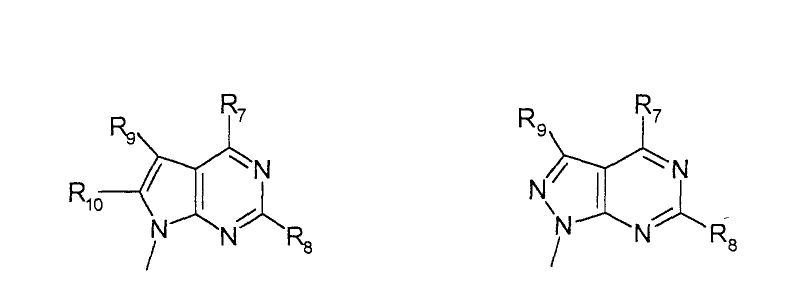 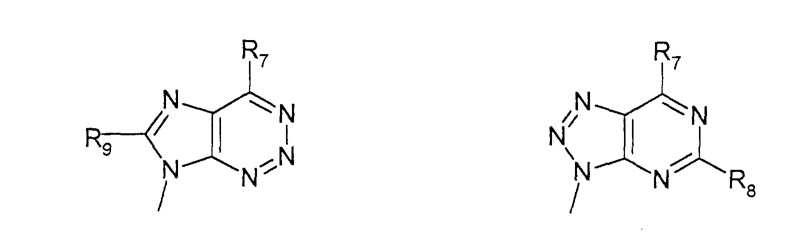 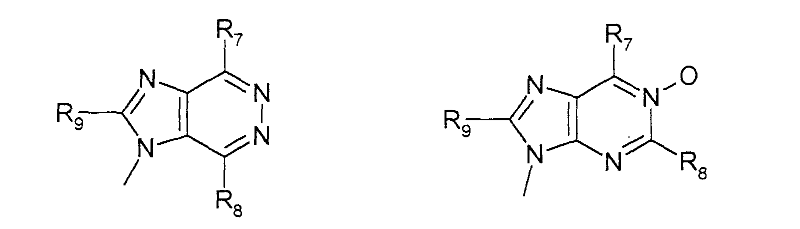 wherein:
X is oxygen or sulfur;
Y is oxygen or sulfur;
R₃ and R₄ are each independently selected from the group consisting of hydrogen, hydroxyl, amino, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyl or aracyl;
R₅ and R₆ are each independently selected from the group consisting of hydrogen, halogen, hydroxyl, amino, cyano, carboxy, carbamoyl, alkoxycarbonyl, hydroxymethyl, trifluoromethyl, thioaryl, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy;
R₇ and R₈ are each independently selected from the group consisting of hydrogen, hydroxy, alkoxy, thiol, thioalkyl, amino, substituted amino, halogen, cyano, carboxy, alkoxycarbonyl, carbamoyl, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy; and
R₉ and R₁₀ are each independently selected from the group consisting of hydrogen, hydroxy, alkoxy, amino, substituted amino, halogen, azido, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy.

15. A process according to claim 14, wherein said compound of formula (XVII) is obtained predominantly in the form of the *cis*-isomer.

16. A process according to claim 14 or 15, further comprising oxidizing Z of the compound of formula (XVII) to give a compound of formula (XVII) wherein Z is S=O or SO₂.

17. A process according to any one of claims 14 to 16, further comprising reducing the RyO₂C group of the compound of formula (XVII) with a suitable reducing agent to obtain a compound of formula (XVIII):

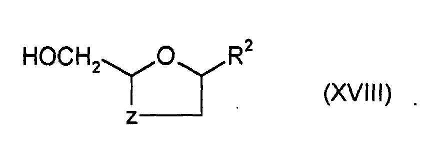

18. A process according to claim 17, further comprising separating the compound into either its *cis* or *trans* isomers.

19. A process according to claim 13, further comprising reacting the compound of formula (XVI) with a halogen-containing silyl Lewis acid to obtain a compound of formula (XXVI): 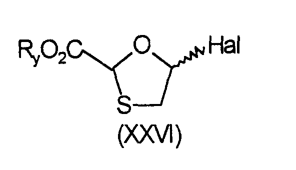 wherein Hal is halogen.

20. The process according to claim 19, further comprising coupling the compound of formula (XXVI) with a base or analogue thereof R₂ under basic conditions, to obtain a compound of formula (XVII): 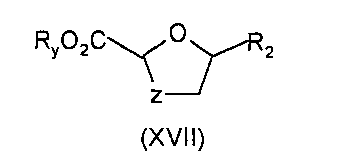 wherein R₂ is selected from the group consisting of: 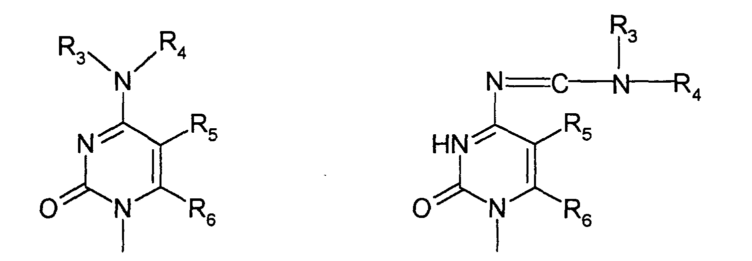 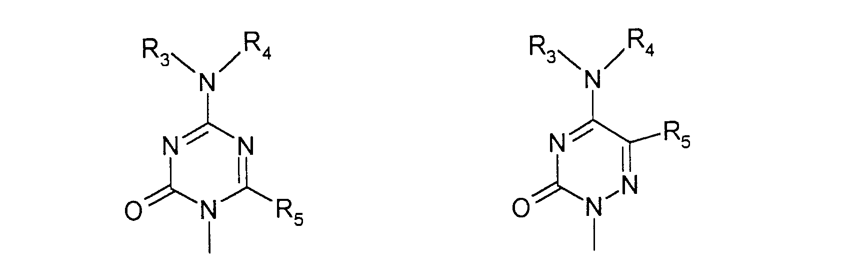 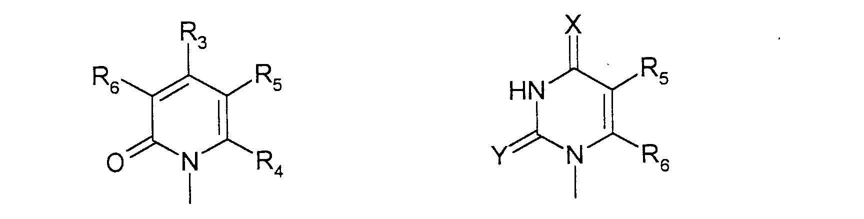 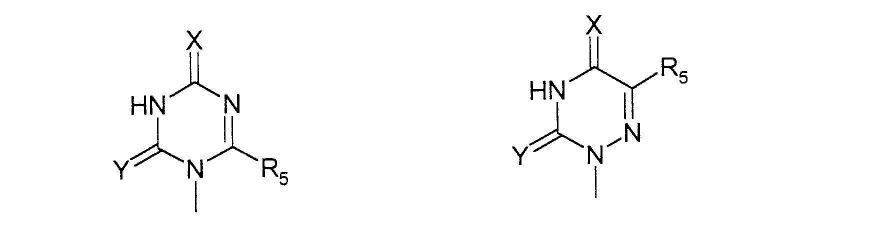 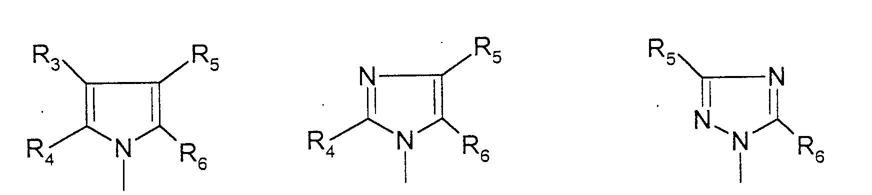 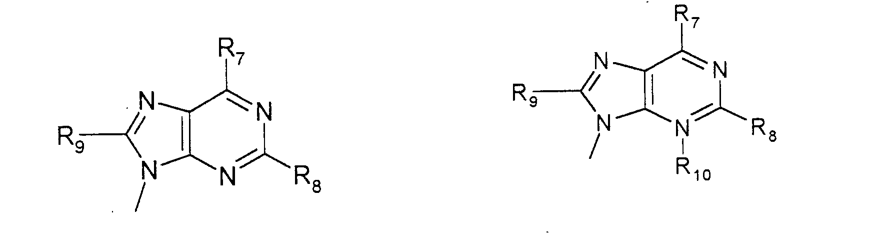 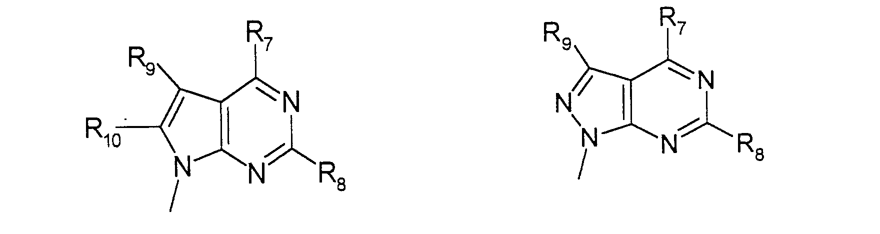 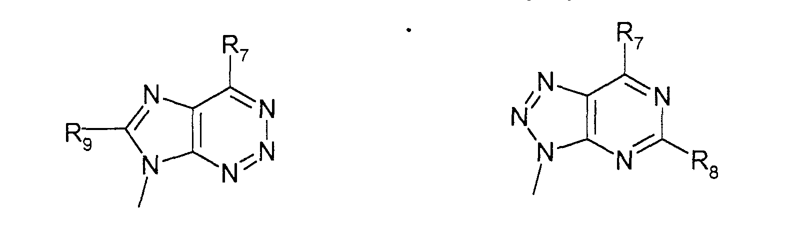 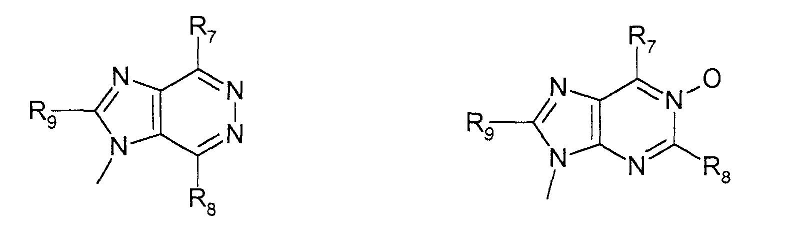 wherein:
X is oxygen or sulfur;
Y is oxygen or sulfur;
R₃ and R₄ are each independently selected from the group consisting of hydrogen, hydroxyl, amino, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyl or aracyl;
R₅ and R₆ are each independently selected from the group consisting of hydrogen, halogen, hydroxyl, amino, cyano, carboxy, carbamoyl, alkoxycarbonyl, hydroxymethyl, trifluoromethyl, thioaryl, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy;
R₇ and R₈ are each independently selected from the group consisting of hydrogen, hydroxy, alkoxy, thiol, thioalkyl, amino, substituted amino, halogen, cyano, carboxy, alkoxycarbonyl, carbamoyl, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy; and
R₉ and R₁₀ are each independently selected from the group consisting of hydrogen, hydroxy, alkoxy, amino, substituted amino, halogen, azido, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy.

21. A process according to claim 19, wherein said Lewis acid is trimethylsilyliodide.

22. A process according to claim 19 or claim 21, wherein Hal is iodine.

23. A process according to anyone of claims 20 to 22, wherein R₂ is a purine base.

24. A process according to claim 23, wherein the purine is 6-chloropurine.

25. A process according to anyone of claims 12 to 24 wherein the mercaptoacetaldehyde is obtained from a mercaptoacetaldehyde dimer dissolved in an inert solvent.

26. The process according to claim 25, wherein the inert solvent is selected from the group consisting of pyridine, toluene and dimethyl sulfoxide.

27. A process according to claim 13, further comprising reducing the ester group of the compound of formula (XVI) and protecting the resulting hydroxymethyl with a hydroxy protecting function R₁ to obtain a compound of formula (XXII): 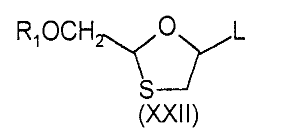 wherein L is alkoxy carbonyl; halogen; substituted or unsubstituted, saturated or unsaturated thiolates; substituted or unsubstituted, saturated or unsaturated selenino; substituted or unsubstituted, saturated or unsaturated aliphatic or aromatic ketones; or -OR; and
R is hydrogen; substituted or unsubstituted, saturated or unsaturated C₁₋₆ alkyl, substituted or unsubstituted, saturated or unsaturated alkenyl having up to 6 carbon atoms; substituted or unsubstituted aliphatic or aromatic acyl; substituted or unsubstituted, saturated or unsaturated alkoxycarbonyl; substituted or unsubstituted sulphonyl imidazolide; substituted or unsubstituted carbonyl imidazolide; substituted or unsubstituted aliphatic or aromatic amino carbonyl group; substituted or unsubstituted alkyl imidate group; substituted or unsubstituted, saturated or unsaturated phosphinoyl; or substituted or unsubstituted, aliphatic or aromatic sulphonyl group.

28. A process according to claim 27, wherein R₁ is an acyl group having 1 to 16 carbon atoms, unsubstituted or substituted with a heteroatom, or is a silyl group.

29. A process according to claim 28, wherein R₁ is t-butyldimethylsilyl or t-butyldiphenylsilyl.

30. A process according to any one of claims 27 to 29, further comprising the step of reacting the compound of formula (XXII) with a silylated pyrimidine base, a silylated purine base or a silylated analogue of a pyrimidine base or purine base, optionally in the presence of a Lewis acid to obtain a compound of formula (XXIII): 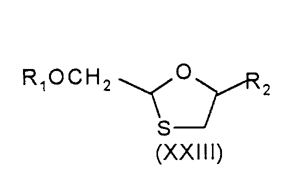 wherein Z is S, and
R² is selected from the group consisting of: 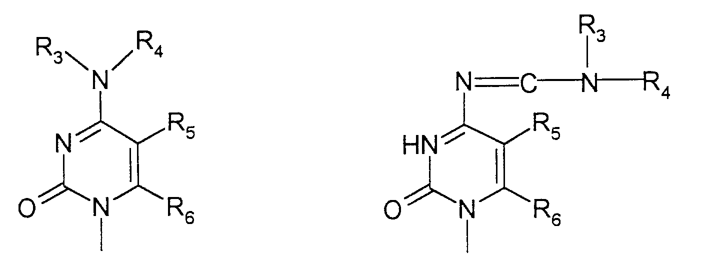 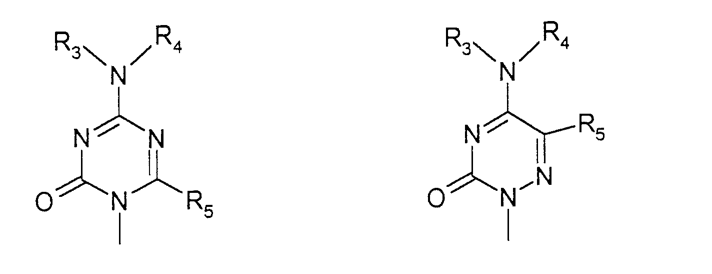 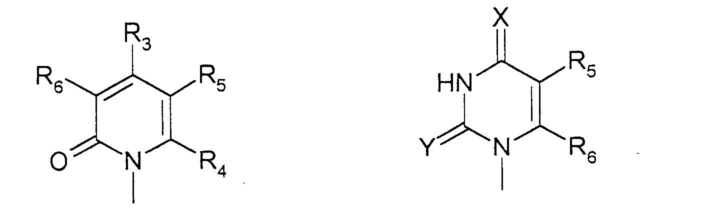 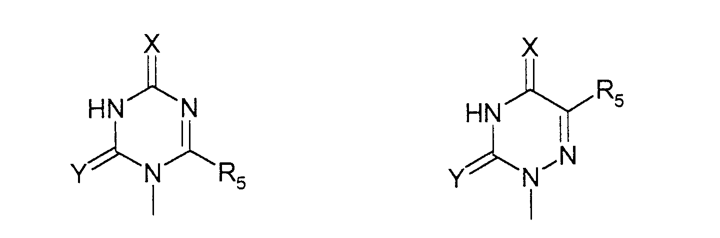 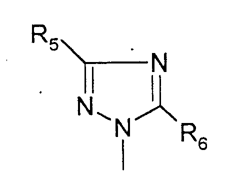 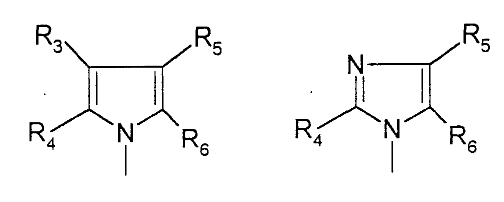 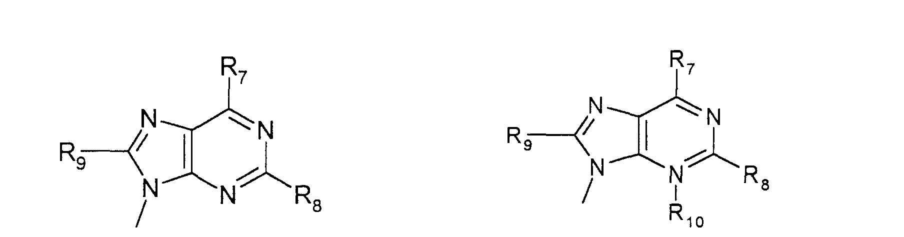 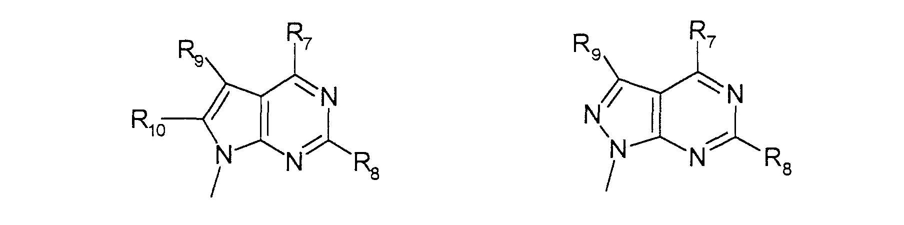 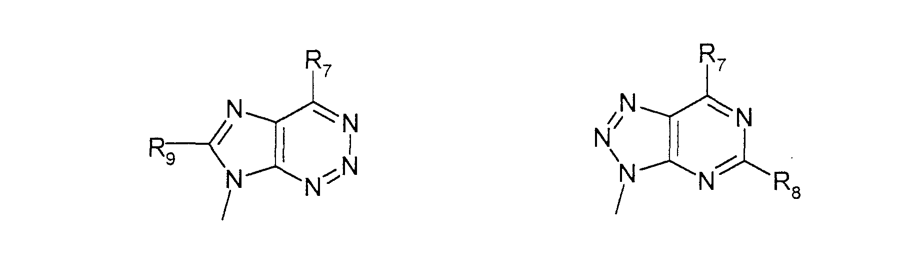 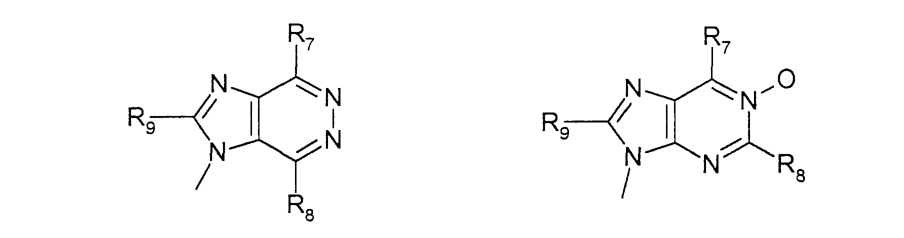 wherein:
X is oxygen or sulfur;
Y is oxygen or sulfur;
R₃ and R₄ are each independently selected from the group consisting of hydrogen, hydroxyl, amino, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyl or aracyl;
R₅ and R₆ are each independently selected from the group consisting of hydrogen, halogen, hydroxyl, amino, cyano, carboxy, carbamoyl, alkoxycarbonyl, hydroxymethyl, trifluoromethyl, thioaryl, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy;
R₇ and R₈ are each independently selected from the group consisting of hydrogen, hydroxy, alkoxy, thiol, thioalkyl, amino, substituted amino, halogen, cyano, carboxy, alkoxycarbonyl, carbamoyl, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy; and
R₉ and R₁₀ are each independently selected from the group consisting of hydrogen, hydroxy, alkoxy, amino, substituted amino, halogen, azido, substituted or unsubstituted C₁₋₆ alkyl, substituted or unsubstituted alkenyl having up to 6 carbon atoms, substituted or unsubstituted alkynyl having up to 6 carbon atoms, and substituted or unsubstituted C₁₋₁₀ acyloxy.

31. A process according to claim 30, wherein the compound of formula (XXIII) is oxidized to obtain a compound of formula (XXIII) wherein Z is S=O or SO₂.

32. A process according to claim 31, comprising acylating R₂ of the compound of formula (XXIII) with acetic anhydride in a solvent to obtain a compound of formula (XXIV): 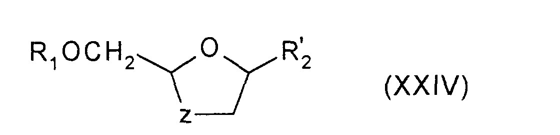 wherein R₂' is an acylated R₂ group.

33. A process according to claim 32, further comprising separating the compound into either its *cis* or *trans* isomers.

34. A process according to claim 32 or claim 33, further comprising hydrolyzing R₂' of the compound of formula XXIV, under basic conditions, to obtain a compound of formula XXV.

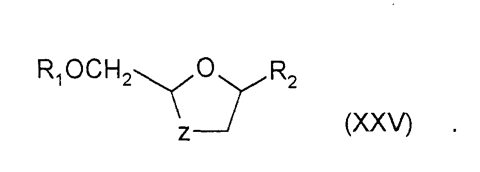

35. A process according to claim 34, further comprising removing the hydroxyl protecting function R₁ from compound (XXIII) to obtain a compound of formula (I):

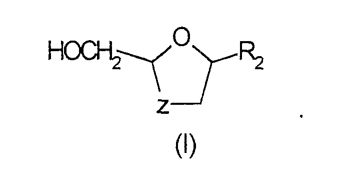

36. A process according to any one of claims 9, 17 and 35, further comprising converting the compound of formula (I) to a pharmaceutically acceptable salt thereof.

37. A process according to any one of claims 9, 17 and 35, further comprising esterifying a compound of formula (I) wherein the OH group is replaced by R-CO-O- and R is H, straight or branched chain alkyl, alkoxyalkyl, arylalkyl, aryloxyalky, or substituted dihydropyridinyl.

38. A process according to any one of claims 9, 17 and 35, further comprising esterifying a compound of formula (I) wherein the OH group is replaced by an alkylsulphonxyl ester, an araalkylsulphonyl ester, a sulfate ester, an amino acid ester, a monophosphate ester, a diphosphate ester, a triphosphate, ester, or an ester derived from HOOC(CH₂)_{q}COOH where q is 0 to 10.

39. A process according to any one of claims 9, 17 and 35, further comprising esterifying a compound of formula (I) where the OH is converted to an ester and said ester is a C₁₋₁₆ alkyl ester, an unsubstituted benzyl ester, or a benzyl ester substituted by at least one halogen C₁₋₆ alkyl, C₂₋₆ alkenyl, saturated or unsaturated C₁₋₆ alkoxy, nitro and trifluoromethyl group.

40. A process according to anyone of claims 27 to 39 wherein the mercaptoacetaldehyde is obtained from a mercaptoacetaldehyde dimer dissolved in an inert solvent.

41. The process according to claim 40, wherein the inert solvent is selected from the group consisting of pyridine, toluene and dimethyl sulfoxide.

42. Intermediates useful for the production of oxathiolane compounds, said intermediates selected from the group consisting of:
(a) *trans*-2-hydroxymethyl-5-acetoxy-1,3-oxathiolane;
(b) *cis*-2-benzoyloxymethyl-5-hydroxy-1,3-oxathiolane, *trans*-2-benzoyloxymethyl-5-hydroxy-1,3-oxathiolane, or a mixture thereof;
(c) *cis*-2-benzoyloxymethyl-5-acetoxy-1,3-oxathiolane, *trans*-2-benzoyloxymethyl-5-acetoxy-1,3-oxathiolane, or a mixture thereof;
(d) *cis*-2-benzoyloxymethyl-5-(4',5'-dichlorobenzoyloxy)-1,3-oxathiolane, *trans*-2-benzoyloxymethyl-5-(4',5'-dichlorobenzoyloxy)-1,3-oxathiolane, or a mixture thereof;
(e) *cis*-2-benzoyloxymethyl-5-trimethylacetoxy-1,3-oxathiolane, *trans*-2-benzoyloxymethyl-5-trimethylacetoxy-1,3-oxathiolane, or a mixture thereof;
(f) *cis*-2-benzoyloxymethyl-5-(2',2',2'-trichloroethoxy-carbonyloxy)-1,3-oxathiolane, *trans*-2-benzoyloxymethyl-5-(2',2',2'-trichloroethoxycarbonyloxy)-1,3-oxathiolane, or a mixture thereof;
(g) *cis*-2-benzoyloxymethyl-5-(ethoxycarbonyloxy)-1,3-oxathiolane, *trans*-2-benzoyloxymethyl-5-(ethoxycarbonyloxy)-1,3-oxathiolane, or a mixture thereof;
(h) *cis*-2-benzoyloxymethyl-5-(methoxycarbonyloxy)-1,3-oxathiolane, *trans*-2-benzoyloxymethyl-5-(methoxycarbonyloxy-1, 3-oxathiolane, or a mixture thereof; and
(i) *cis*-2-benzoyloxymethyl-5-benzoyloxy-1,3-oxathiolane, *trans*-2-benzoyloxymethyl-5-benzoyloxy-1,3-oxathiolane, or a mixture thereof.
(j) *cis*-2-carboethoxy-5-methoxycarbonyloxy-1,3-oxathiolane, *trans*-2-carboethoxy-5-methoxycarbonyloxy-1,3-oxathiolane and mixtures thereof;
(k) *cis*-2-carboethoxy-5-acetoxy-1,3-oxathiolane, *trans*-2-carboethoxy-5-acetoxy-1,3-oxathiolane and mixtures thereof; (l) *cis*-2-carboethoxy-5-(N4'-acetylcytosin-1'-yl)-1,3-oxathiolane;
(m) *cis*-2-carboethoxy-5-(cytosin-1'-yl)-1,3-oxathiolane;
(n) *cis*-2-carboethoxy-5-(uracil-1'-yl)-1,3-oxathiolane;
(o) *cis*-2-benzoyloxymethyl-5-(cytosin-1'-yl)-1,3-oxathiolane;
(p) *cis*-2-ethyl-5-iodo-1,3-oxathiolan-2-carboxylate, *trans*-2-ethyl-5-iodo-1,3-oxathiolan-2-carboxylate and mixtures thereof;
(q) *cis*-2-ethyl-5-(6'-chloropurin-9'-yl)-1,3-oxathiolan-2-carboxylate, *trans*-2-ethyl-5-(6'-chloropurin-9'-yl)-1,3-oxathiolan-2-carboxylate and mixtures thereof;
(r) *cis*-2-ethyl-5-(6'chloropurin-7'-yl)-1,3-oxathiolan-2-carboxylate, *trans*-2-ethyl-5-(6'chloropurin-7'-yl)-1,3-oxathiolan-2-carboxylate and mixtures thereof; and
(s) *cis*-2-acetoxymethyl-5-acetoxy-1,3-oxathiolane, *trans*-2-acetoxymethyl-5-acetoxy-1,3-oxathiolane and mixtures thereof.

43. An intermediate useful for the production of oxathiolane compounds having the formula (XIII): 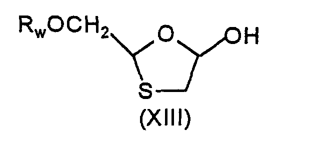 wherein R_{w} is hydrogen or R₁, wherein R₁ is a hydroxyl protecting group selected from aralkyl (eg.benzyl, diphenylmethyl or triphenylmethyl), heterocyclic groups such as tetrahydropyranyl, acyl (e.g. acetyl or benzoyl) and silyl groups such as trialkylsilyl (eg. t-butyldimethylsilyl).

## Patentansprüche

1. Verfahren zur Herstellung einer Oxathiolanverbindung, umfassend
die Umsetzung eines Mercaptoacetaldehyds, wobei das Mercaptoacetaldehyd aus einem in einem inerten Lösungsmittel gelösten Mercaptoacetaldehyddimer erhalten wird, mit einer Verbindung der Formel R_{w}OCH₂CHO unter neutralen oder basischen Bedingungen, wobei R₂ Wasserstoff oder R₁ ist, wobei R₁ eine Hydroxylschutzgruppe ist, um eine Verbindung der Formel (XIII) zu erhalten:

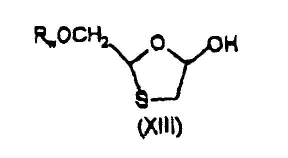

2. Verfahren nach Anspruch 1, das außerdem die Umwandlung des Hydroxyls der Verbindung der Formel (XIII) in eine geeignete Abgangsfunktion L umfasst, um eine Verbindung der Formel (XIV) zu erhalten, 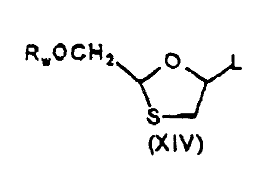 in der L Alkoxycarbonyl, Halogen, substituierte oder unsubstituierte, gesättigte oder ungesättigte Thiolate, substituiertes oder unsubstituiertes, gesättigtes oder ungesättigtes Selenino, substituierte oder unsubstituierte, gesättigte oder ungesättigte aliphatische oder aromatische Ketone oder -OR ist, und
R Wasserstoff, substituiertes oder unsubstituiertes, gesättigtes oder ungesättigtes C₁₋₆-Alkyl, substituiertes oder unsubstituiertes, gesättigtes oder ungesättigtes Alkenyl mit bis zu 6 Kohlenstoffatomen, substituiertes oder unsubstituiertes aliphatisches oder aromatisches Acyl; substituiertes oder unsubstituiertes, gesättigtes oder ungesättigtes Alkoxycarbonyl, substituiertes oder unsubstituiertes Sulfonylimidazolid, substituiertes oder unsubstituiertes Carbonylimidazolid, eine substituierte oder unsubstituierte aliphatische oder aromatische Aminocarbonylgruppe, eine substituierte oder unsubstituierte Alkylimidatgruppe, substituiertes oder unsubstituiertes, gesättigtes oder ungesättigtes Phosphinoyl oder eine substituierte oder unsubstituierte aliphatische oder aromatische Sulfonylgruppe ist.

3. Verfahren nach Anspruch 2, das außerdem die Umsetzung der Verbindung der Formel (XIV) mit einer silylierten Pyrimidinbase, einer silylierten Purinbase oder einem silylierten Analogon einer Pyrimidin- oder Purinbase, ggfs. in Gegenwart einer Lewis-Säure, umfasst, um eine Verbindung der Formel (IX): 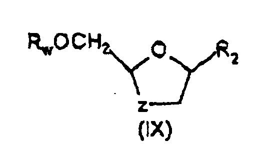 herzustellen, in der Z S ist
und R₂ aus folgender Gruppe ausgewählt wird: 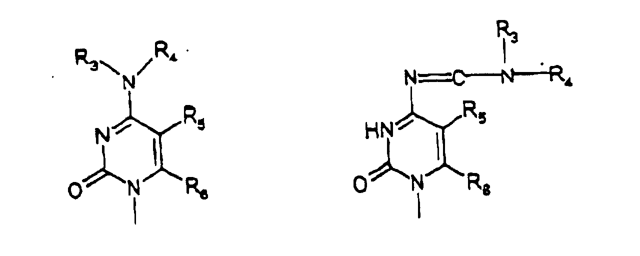 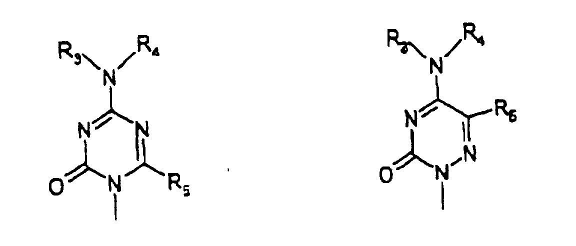 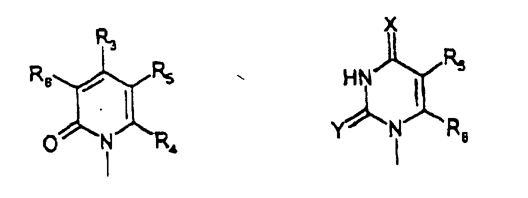 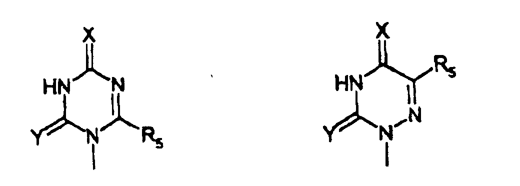 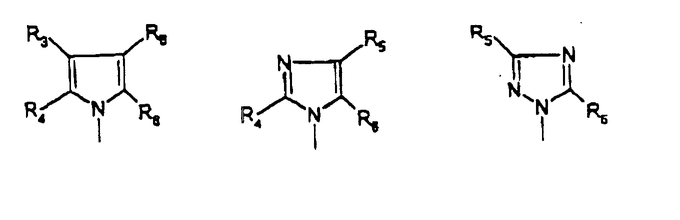 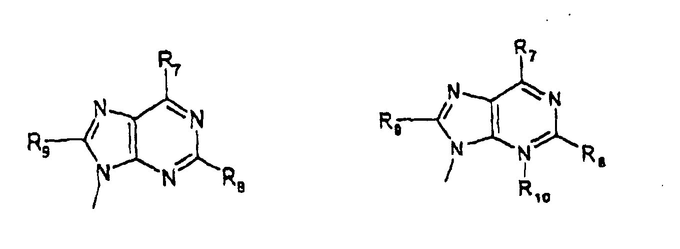 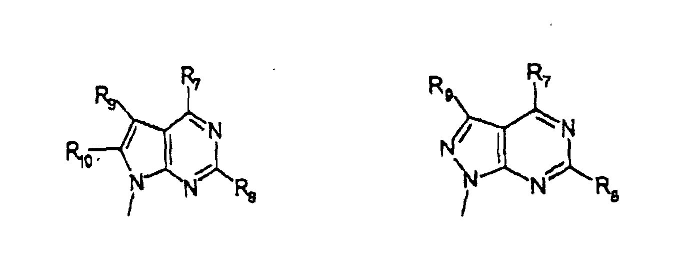 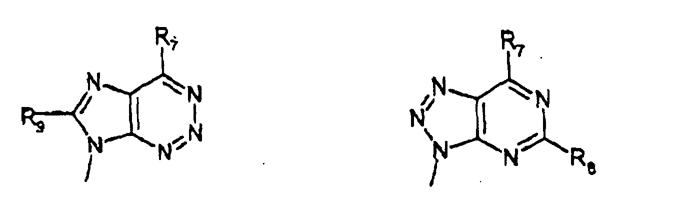 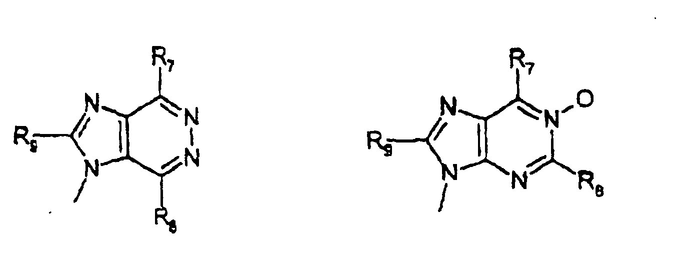 worin
X Sauerstoff oder Schwefel ist;
Y Sauerstoff oder Schwefel ist,
R₃ und R₄ unabhängig voneinander aus der aus Wasserstoff, Hydroxyl, Amino, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyl oder Aracyl bestehenden Gruppe ausgewählt sind,
R₅ und R₆ jeweils unabhängig voneinander aus der aus Wasserstoff, Halogen, Hydroxyl, Amino, Cyano, Carboxy, Carbamoyl, Alkoxycarbonyl, Hydroxymethyl, Trifluormethyl, Thioaryl, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyloxy bestehenden Gruppe ausgewählt sind,
R₇ und R₈ jeweils unabhängig voneinander aus der aus Wasserstoff, Hydroxy, Alkoxy, Thiol, Thioalkyl, Amino, substituiertem Amino, Halogen, Cyano, Carboxy, Alkoxycarbonyl, Carbamoyl, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyloxy bestehenden Gruppe ausgewählt sind, und
R₉ und R₁₀ jeweils unabhängig voneinander aus der aus Wasserstoff, Hydroxy, Alkoxy, Amino, substituiertem Amino, Halogen, Azido, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyloxy bestehenden Gruppe ausgewählt sind.

4. Verfahren nach Anspruch 3, bei dem die Reaktion mit der Verbindung der Formel (XIV) in Abwesenheit einer Lewis-Säure durchgeführt wird.

5. Verfahren nach Anspruch 3, bei dem die Reaktion mit der Verbindung der Formel (XIV) in Gegenwart einer Lewis-Säure durchgeführt wird.

6. Verfahren nach einem der Ansprüche 3 bis 5, das außerdem die Oxidation der Gruppe Z der Verbindung der Formel (IX) umfasst, um eine Verbindung der Formel (IX) zu ergeben, in der die Gruppe Z S=O oder SO₂ ist.

7. Verfahren nach einem der Ansprüche 3 bis 6, das außerdem die Acylierung der Verbindung der Formel (IX) mit Essigsäureanhydrid umfasst, um eine Verbindung der Formel X 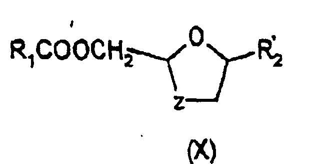 zu ergeben, in der R₁ eine Hydroxylschutzgruppe und R'₂ eine acylierte R₂-Gruppe ist.

8. Verfahren nach Anspruch 7, das außerdem die Trennung der Verbindung der Formel (X) entweder in deren *cis-* oder *trans*-Isomere umfasst.

9. Verfahren nach Anspruch 7 oder 8, das außerdem die Hydrolyse der Acylfünktionen R₂' und R₁COOCH₂ der Verbindung der Formel (X) umfasst, um eine Verbindung der Formel (I), ein geometrisches Isomer davon oder ein optisches Isomer davon zu erhalten:

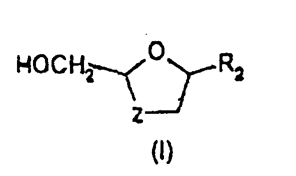

10. Verfahren nach einem der Ansprüche 1 bis 9, bei dem R₁ eine unsubstituierte oder mit einem Heteroatom substituierte Acylgruppe mit 1 bis 16 Kohlenstoffatomen oder eine Silylgruppe ist.

11. Verfahren nach Anspruch 1, bei dem das inerte Lösungsmittel aus der aus Pyridin, Toluol und Dimethylsulfoxid bestehenden Gruppe ausgewählt ist.

12. Verfahren zur Herstellung einer Oxathiolanverbindung, umfassend
die Umsetzung eines Mercaptoacetaldehyds mit einer Verbindung der Formel R_{y}OOCCHO, in der R_{y} substituiertes oder unsubstituiertes C₁₋₁₂-Alkyl oder substituiertes oder unsubstituiertes C₆₋₂₀-Aryl ist, um eine Verbindung der Formel (XV) zu erhalten

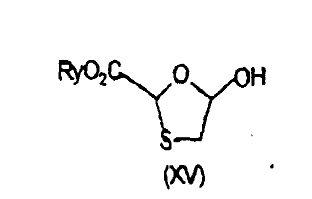

13. Verfahren nach Anspruch 12, das außerdem die Umwandlung des Hydroxyls der Verbindung der Formel (XV) in eine geeignete Abgangsfunktion L umfasst, um eine Verbindung der Formel (XVI) zu erhalten 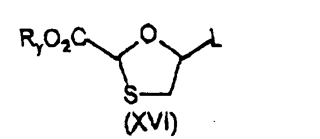 in der L Alkoxycarbonyl, Halogen, substituierte oder unsubstituierte, gesättigte oder ungesättigte Thiolate, substituiertes oder unsubstituiertes, gesättigtes oder ungesättigtes Selenino, substituierte oder unsubstituierte, gesättigte oder ungesättigte aliphatische oder aromatische Ketone oder -OR ist, und
R Wasserstoff, substituiertes oder unsubstituiertes, gesättigtes oder ungesättigtes C₁₋₆-Alkyl, substituiertes oder unsubstituiertes, gesättigtes oder ungesättigtes Alkenyl mit bis zu 6 Kohlenstoffatomen, substituiertes oder unsubstituiertes aliphatisches oder aromatisches Acyl; substituiertes oder unsubstituiertes, gesättigtes oder ungesättigtes Alkoxycarbonyl, substituiertes oder unsubstituiertes Sulfonylimidazolid, substituiertes oder unsubstituiertes Carbonylimidazolid, eine substituierte oder unsubstituierte aliphatische oder aromatische Aminocarbonylgruppe, eine substituierte oder unsubstituierte Alkylimidatgruppe, substituiertes oder unsubstituiertes, gesättigtes oder ungesättigtes Phosphinoyl oder eine substituierte oder unsubstituierte aliphatische oder aromatische Sulfonylgruppe ist.

14. Verfahren nach Anspruch 13, das außerdem die Umsetzung der Verbindung der Formel (XVI) mit einer silylierten Pyrimidinbase, einer silylierten Purinbase oder einem silylierten Analogon einer Pyrimidin- oder Purinbase, ggfs. in Gegenwart einer Lewis-Säure, umfasst, um eine Verbindung der Formel (XVII) 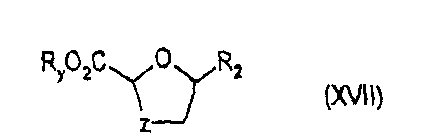 herzustellen, in der Z S ist und
R₂ aus folgender Gruppe ausgewählt wird: 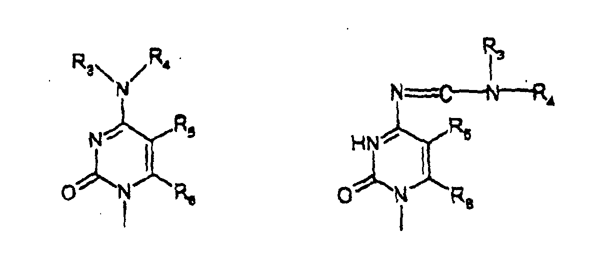 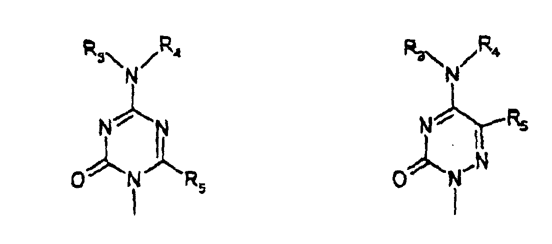 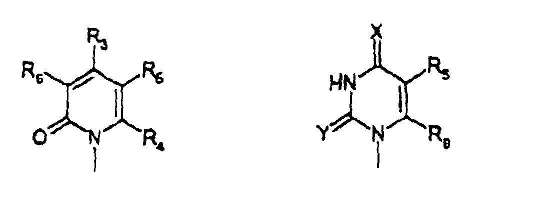 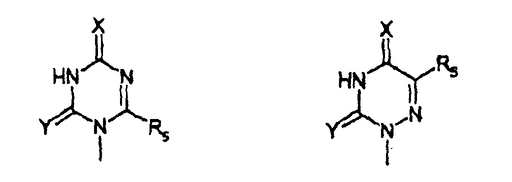 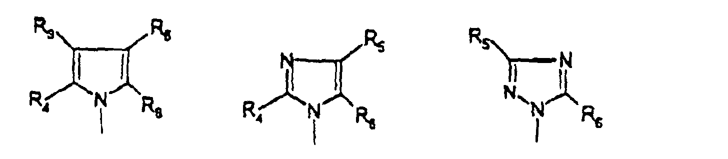 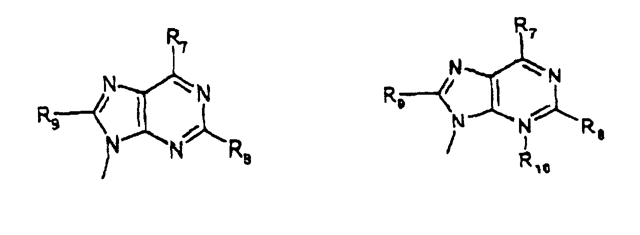 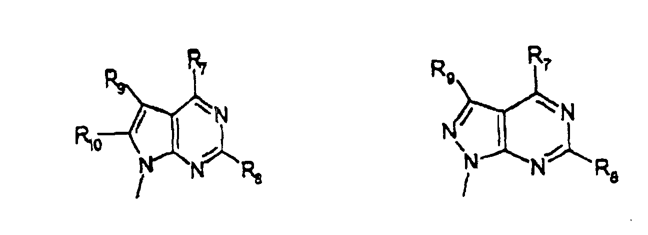 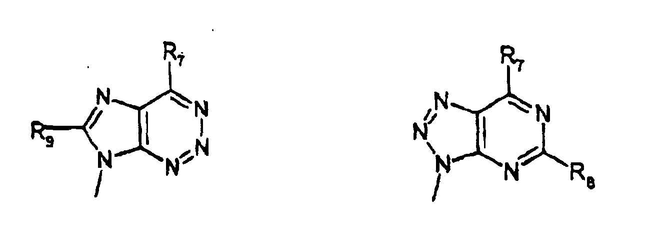 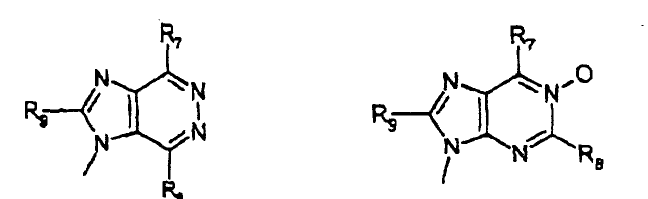 worin
X Sauerstoff oder Schwefel ist;
Y Sauerstoff oder Schwefel ist,
R₃ und R₄ unabhängig voneinander aus der aus Wasserstoff, Hydroxyl, Amino, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyl oder Aracyl bestehenden Gruppe ausgewählt sind,
R₅ und R₆ jeweils unabhängig voneinander aus der aus Wasserstoff, Halogen, Hydroxyl, Amino, Cyano, Carboxy, Carbamoyl, Alkoxycarbonyl, Hydroxymethyl, Trifluormethyl, Thioaryl, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋ ₁₀-Acyloxy bestehenden Gruppe ausgewählt sind,
R₇ und R₈ jeweils unabhängig voneinander aus der aus Wasserstoff, Hydroxy, Alkoxy, Thiol, Thioalkyl, Amino, substituiertem Amino, Halogen, Cyano, Carboxy, Alkoxycarbonyl, Carbamoyl, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyloxy bestehenden Gruppe ausgewählt sind, und
R₉ und R₁₀ jeweils unabhängig voneinander aus der aus Wasserstoff, Hydroxy, Alkoxy, Amino, substituiertem Amino, Halogen, Azido, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyloxy bestehenden Gruppe ausgewählt sind.

15. Verfahren nach Anspruch 14, bei dem die Verbindung der Formel (XVII) überwiegend in Form des *cis*-Isomers erhalten wird.

16. Verfahren nach einem der Ansprüche 14 oder 15, das außerdem die Oxidation der Gruppe Z der Verbindung der Formel (XVII) umfasst, um eine Verbindung der Formel (XVII) zu ergeben, in der Z S=O oder SO₂ ist.

17. Verfahren nach einem der Ansprüche 14 bis 16, das außerdem die Reduktion der R_{y}O₂C-Gruppe in der Verbindung der Formel (XVII) mit einem geeigneten Reduktionsmittel umfasst, um eine Verbindung der Formel (XVIII) zu erhalten:

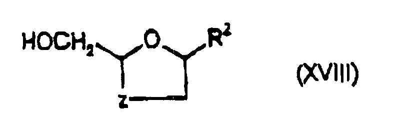

18. Verfahren nach Anspruch 17, das außerdem die Trennung der Verbindung entweder in deren *cis-* oder *trans*-Isomere umfasst.

19. Verfahren nach Anspruch 13, das außerdem die Umsetzung der Verbindung der Formel (XVI) mit einer halogenhaltigen Silyl-Lewis-Säure umfasst, um eine Verbindung der Formel (XXVI) zu erhalten 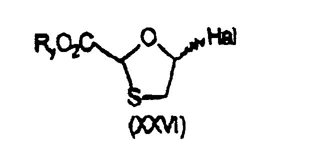 in der Hal Halogen bedeutet.

20. Verfahren nach Anspruch 19, das außerdem die Kupplung der Verbindung der Formel (XXVI) mit einer Base oder einem Analogon von R₂ unter basischen Bedingungen umfasst, um eine Verbindung der Formel (XVII) zu erhalten 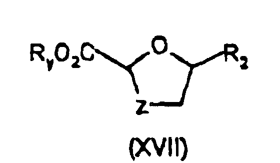 in der R₂ aus folgender Gruppe ausgewählt wird: 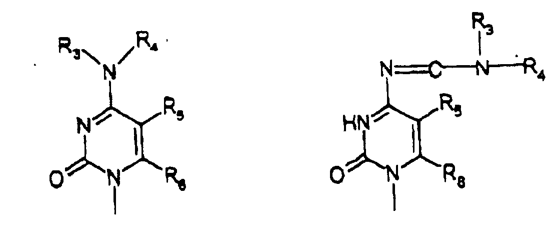 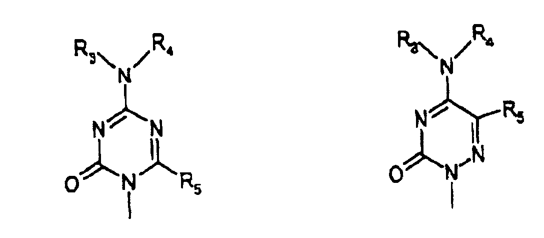 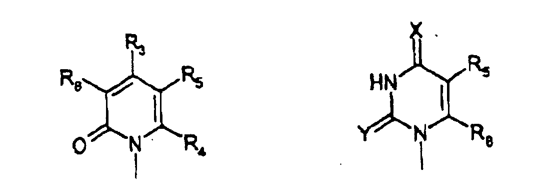 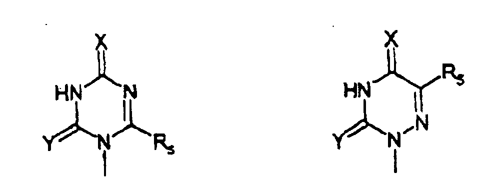 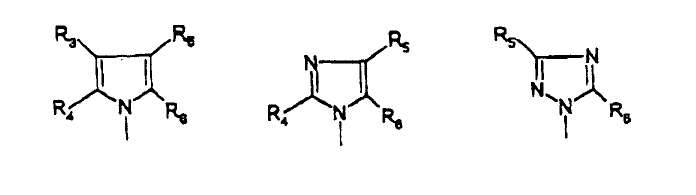 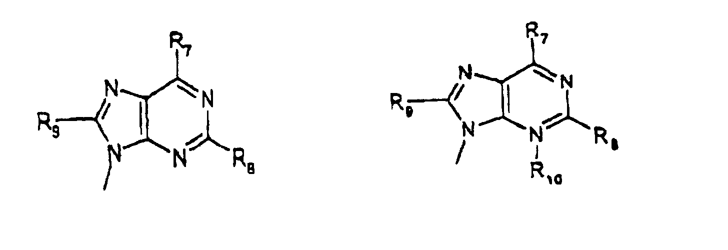 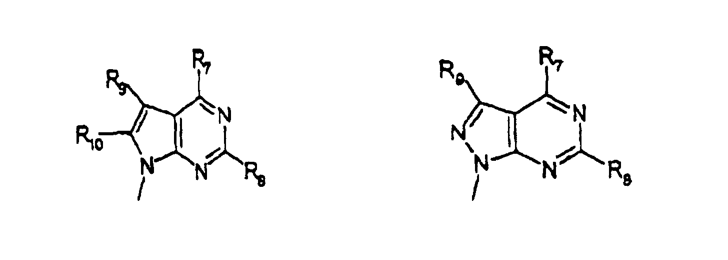 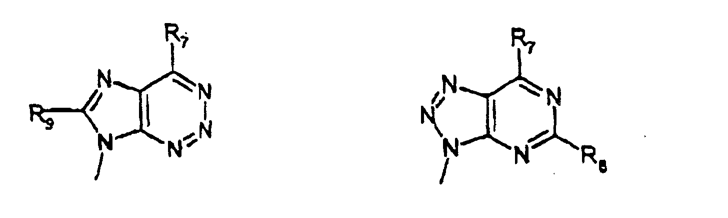 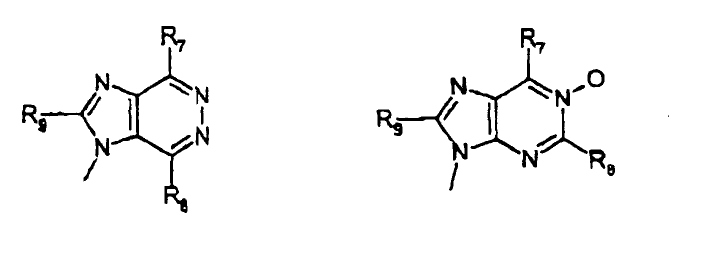 worin
X Sauerstoff oder Schwefel ist;
Y Sauerstoff oder Schwefel ist,
R₃ und R₄ unabhängig voneinander aus der aus Wasserstoff, Hydroxyl, Amino, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyl oder Aracyl bestehenden Gruppe ausgewählt sind,
R₅ und R₆ jeweils unabhängig voneinander aus der aus Wasserstoff, Halogen, Hydroxyl, Amino, Cyano, Carboxy, Carbamoyl, Alkoxycarbonyl, Hydroxymethyl, Trifluormethyl, Thioaryl, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋ ₁₀-Acyloxy bestehenden Gruppe ausgewählt sind,
R₇ und R₈ jeweils unabhängig voneinander aus der aus Wasserstoff, Hydroxy, Alkoxy, Thiol, Thioalkyl, Amino, substituiertem Amino, Halogen, Cyano, Carboxy, Alkoxycarbonyl, Carbamoyl, substituiertem oder unsubstituienem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyloxy bestehenden Gruppe ausgewählt sind, und
R₉ und R₁₀ jeweils unabhängig voneinander aus der aus Wasserstoff, Hydroxy, Alkoxy, Amino, substituiertem Amino, Halogen, Azido, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyloxy bestehenden Gruppe ausgewählt sind.

21. Verfahren nach Anspruch 19, bei dem die Lewis-Säure Trimethylsilyliodid ist.

22. Verfahren nach Anspruch 19 oder 21, bei dem Hal Iod ist.

23. Verfahren nach einem der Ansprüche 20 bis 22, bei dem R₂ eine Purinbase ist.

24. Verfahren nach Anspruch 23, bei dem das Purin 6-Chlorpurin ist.

25. Verfahren nach einem der Ansprüche 12 bis 24, bei dem das Mercaptoacetaldehyd aus einem in einem inerten Lösungsmittel gelösten Mercaptoacetaldehyddimer erhalten wird.

26. Verfahren nach Anspruch 25, bei dem das inerte Lösungsmittel aus der aus Pyridin, Toluol und Dimethylsulfoxid bestehenden Gruppe ausgewählt wird.

27. Verfahren nach Anspruch 13, das außerdem die Reduktion der Estergruppe der Verbindung der Formel (XVI) und das Schützen des resultierenden Hydroxymethyls mit einer Hydroxyschutzfunktion R₁ umfasst, um eine Verbindung der Formel (XXII) 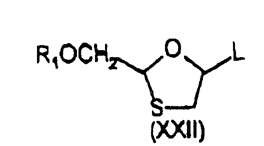 zu erhalten, in der L Alkoxycarbonyl, Halogen, substituierte oder unsubstituierte, gesättigte oder ungesättigte Thiolate, substituiertes oder unsubstituiertes, gesättigtes oder ungesättigtes Selenino, substituierte oder unsubstituierte, gesättigte oder ungesättigte aliphatische oder aromatische Ketone oder -OR ist, und
R Wasserstoff, substituiertes oder unsubstituiertes, gesättigtes oder ungesättigtes C₁₋₆-Alkyl, substituiertes oder unsubstituiertes, gesättigtes oder ungesättigtes Alkenyl mit bis zu 6 Kohlenstoffatomen, substituiertes oder unsubstituiertes aliphatisches oder aromatisches Acyl; substituiertes oder unsubstituiertes, gesättigtes oder ungesättigtes Alkoxycarbonyl, substituiertes oder unsubstituiertes Sulfonylimidazolid, substituiertes oder unsubstituiertes Carbonylimidazolid, eine substituierte oder unsubstituierte aliphatische oder aromatische Aminocarbonylgruppe, eine substituierte oder unsubstituierte Alkylimidatgruppe, substituiertes oder unsubstituiertes, gesättigtes oder ungesättigtes Phosphinoyl oder eine substituierte oder unsubstituierte aliphatische oder aromatische Sulfonylgruppe ist.

28. Verfahren nach Anspruch 27, bei dem R₁ eine unsubstituierte oder mit einem Heteroatom substituierte Acylgruppe mit 1 bis 16 Kohlenstoffatomen oder eine Silylgruppe ist.

29. Verfahren nach Anspruch 28, bei dem R₁ t-Butyldimethylsilyl oder t-Butyldiphenylsilyl ist.

30. Verfahren nach einem der Ansprüche 27 bis 29, das außerdem die Umsetzung der Verbindung der Formel (XXII) mit einer silylierten Pyrimidinbase, einer silylierten Purinbase oder einem silylierten Analogon einer Pyrimidin- oder Purinbase, ggfs. in Gegenwart einer Lewis-Säure, umfasst, um eine Verbindung der Formel (XXIII) 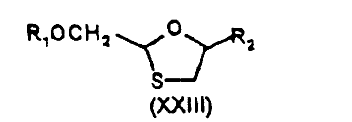 zu erhalten, in der Z S ist
und R₂ aus folgender Gruppe ausgewählt wird: 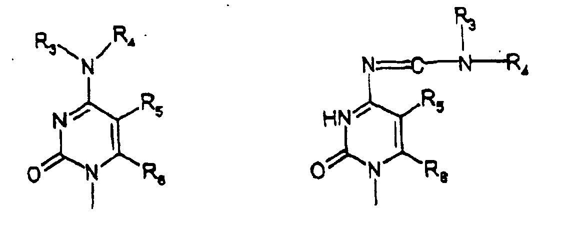 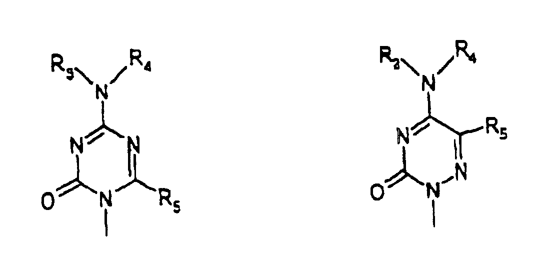 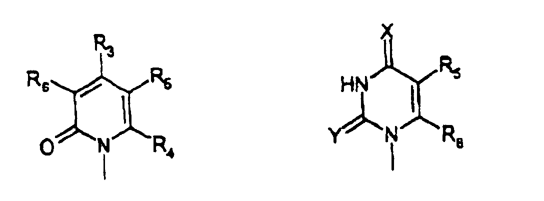 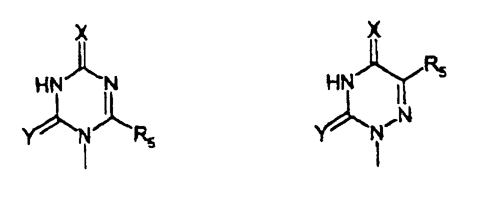 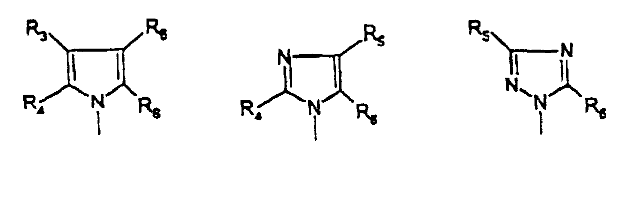 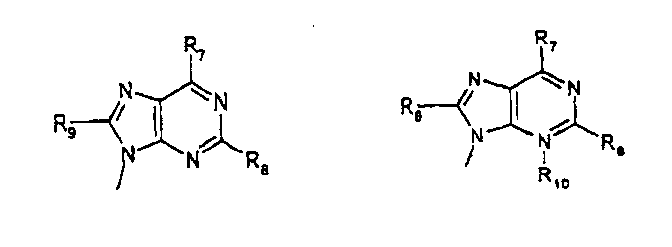 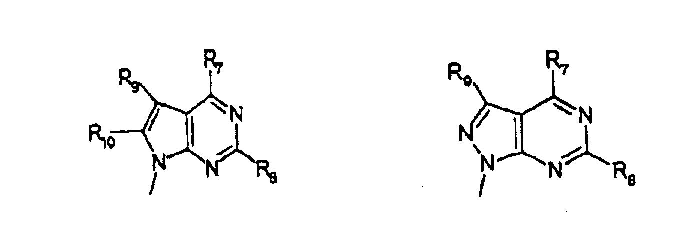 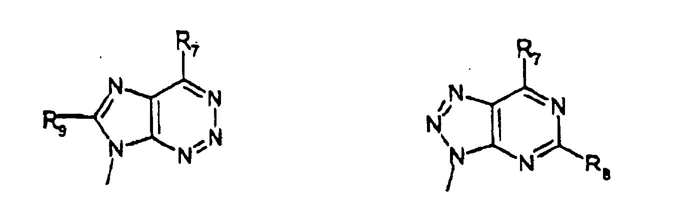 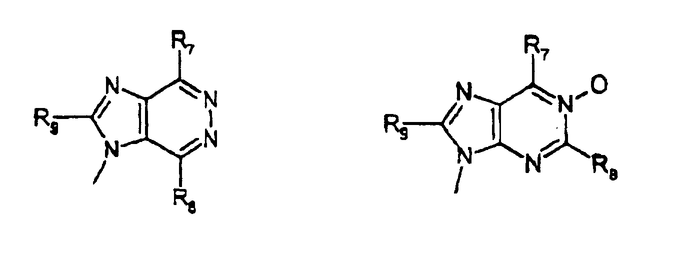 worin
X Sauerstoff oder Schwefel ist;
Y Sauerstoff oder Schwefel ist,
R₃ und R₄ unabhängig voneinander aus der aus Wasserstoff, Hydroxyl, Amino, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyl oder Aracyl bestehenden Gruppe ausgewählt sind,
R₅ und R₆ jeweils unabhängig voneinander aus der aus Wasserstoff, Halogen, Hydroxyl, Amino, Cyano, Carboxy, Carbamoyl, Alkoxycarbonyl, Hydroxymethyl, Trifluormethyl, Thioaryl, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyloxy bestehenden Gruppe ausgewählt sind,
R₇ und R₈ jeweils unabhängig voneinander aus der aus Wasserstoff, Hydroxy, Alkoxy, Thiol, Thioalkyl, Amino, substituiertem Amino, Halogen, Cyano, Carboxy, Alkoxycarbonyl, Carbamoyl, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyloxy bestehenden Gruppe ausgewählt sind, und
R₉ und R₁₀ jeweils unabhängig voneinander aus der aus Wasserstoff, Hydroxy, Alkoxy, Amino, substituiertem Amino, Halogen, Azido, substituiertem oder unsubstituiertem C₁₋₆-Alkyl, substituiertem oder unsubstituiertem Alkenyl mit bis zu 6 Kohlenstoffatomen, substituiertem oder unsubstituiertem Alkynyl mit bis zu 6 Kohlenstoffatomen und substituiertem oder unsubstituiertem C₁₋₁₀-Acyloxy bestehenden Gruppe ausgewählt sind.

31. Verfahren nach Anspruch 30, bei dem die Verbindung der Formel (XXIII) oxidiert wird, um eine Verbindung der Formel (XXIII) zu erhalten, in der Z S=O oder SO₂ ist.

32. Verfahren nach Anspruch 31, umfassend die Acylierung von R₂ aus der Verbindung der Formel (XXIII) mit Essigsäureanhydrid, um eine Verbindung der Formel (XXIV) 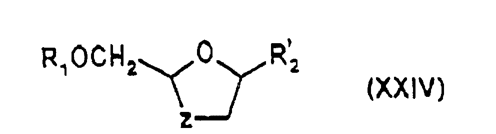 zu erhalten, in R'₂ eine acylierte R₂-Gruppe ist.

33. Verfahren nach Anspruch 32, das außerdern die Trennung der Verbindung entweder in deren *cis-* oder *trans*-Isomere umfasst.

34. Verfahren nach Anspruch 32 oder 33, das außerdem die Hydrolyse von R₂' aus der Verbindung der Formel XXIV unter basischen Bedingungen umfasst, um eine Verbindung der Formel XXV 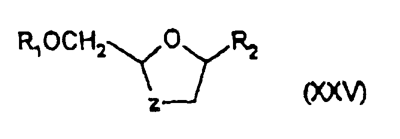 zu erhalten

35. Verfahren nach Anspruch 34, das außerdem die Entfernung der Hydroxylschutzfunktion R₁ aus der Verbindung (XXIII) umfasst, um eine Verbindung der Formel (I) 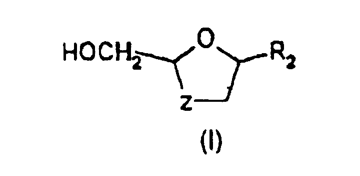 zu erhalten.

36. Verfahren nach einem der Ansprüche 9, 17 und 35, das außerdem die Umwandlung der Verbindung der Formel (I) in ein pharmazeutisch verträgliches Salz davon umfasst.

37. Verfahren nach einem der Ansprüche 9, 17 und 35, das außerdem die Veresterung der Verbindung der Formel (I) umfasst, wobei die OH-Gruppe durch R-CO-O- ersetzt wird und R H, gerad- oder verzweigtkettiges Alkyl, Alkoxyalkyl, Arylalkyl, Aryloxyalkyl oder substituiertes Dihydropyridinyl ist.

38. Verfahren nach einem der Ansprüche 9, 17 und 35, das außerdem die Veresterung einer Verbindung der Formel (I) umfasst, wobei die OH-Gruppe durch einen Alkylsulfonylester, einen Aralkylsulfonylester, einen Sulfatester, einen Aminosäureester, einen Monosphosphatester, einen Diphosphatester, einen Triphosphatester oder einen von HOOC(CH₂)_{q}COOH abgeleiteten Ester, worin q 0 bis 10 ist, ersetzt wird.

39. Verfahren nach einem der Ansprüche 9, 17 und 35, das außerdem die Veresterung einer Verbindung der Formel (I) umfasst, wo OH zu einem Ester umgewandelt wird und dieser Ester ein C₁₋₁₆-Alkylester, ein unsubstituierter Benzylester oder ein Benzylester ist, substituiert durch mindestens ein Halogen-C₁₋₆-Alkyl, C₂₋₆-Alkenyl, gesättigtes oder ungesättigtes C₁₋₆-Alkoxy, eine Nitro- und Trifluormethylgruppe.

40. Verfahren nach einem der Ansprüche 27 bis 39, bei dem das Mercaptoacetaldehyd aus einem in einem inerten Lösungsmittel gelösten Mercaptoacetaldehyddimer erhalten wird.

41. Verfahren nach Anspruch 40, bei dem das inerte Lösungsmittel aus der aus Pyridin, Toluol und Dimethylsulfoxid bestehenden Gruppe ausgewählt ist.

42. Zwischenprodukte, die für die Herstellung von Oxathiolanverbindungen brauchbar sind, wobei die Zwischenprodukte aus folgender Gruppe ausgewählt sind:
(a) *trans*-2-Hydroxymethyl-5-acetoxy-1,3-oxathiolan;
(b) *cis*-2-Benzoyloxymethyl-5-hydroxy-1,3-oxathiolan, *trans*-2-Benzoyloxymethyl-5-hydroxy-1,3-oxathiolan oder einem Gemisch davon;
(c) *cis*-2-Benzoyloxymethyl-5-acetoxy-1,3-oxathiolan, *trans*-2-Benzoyloxymethyl-5-acetoxy-1,3-oxathiolan oder einem Gemisch davon;
(d) *cis*-2-Benzoyloxymethyl-5-(4',5'-dichlorbenzoyloxy)-1,3-oxathiolan, *trans*-2-Benzoyloxymethyl-5-(4',5'-dichlorbenzoyloxy)-1,3-oxathiolan oder einem Gemisch davon;
(e) *cis*-2-Benzoyloxymethyl-5-trimethylacetoxy-1,3-oxathiolan; *trans*-2-Benzoyloxymethyl-5-trimethylacetoxy-1,3-oxathiolan oder einem Gemisch davon;
(f) *cis*-2-Benzoyloxymethyl-5-(2',2',2'-Trichlorethoxycarbonyloxy)-1,3-oxathiolan, *trans*-2-Benzoyloxymethyl-5-(2',2',2'-Trichlorethoxycarbonyloxy)-1,3-oxathiolan oder einem Gemisch davon.
(g) *cis*-2-Benzoyloxymethyl-5-(ethoxycarbonyloxy)-1,3-oxathiolan, *trans*-2-Benzoyloxymethyl-5-(ethoxycarbonyloxy)-1,3-oxathiolan oder einem Gemisch davon;
(h) *cis*-2-Benzoyloxymethyl-5-(methoxycarbonyloxy)-1,3-oxathiolan, *trans*-2-Benzoyloxymethyl-5-(methoxycarbonyloxy)-1,3-oxathiolan oder einem Gemisch davon, und
(i) *cis*-2-Benzoyloxymethyl-5-benzoyloxy-1,3-oxathiolan, *trans*-2-Benzoyloxymethyl-5-benzoyloxy-1,3-oxathiolan oder einem Gemisch davon;
(j) *cis*-2-Carboethoxy-5-methoxycarbonyloxy-1,3-oxathiolan, *trans*-2-Carboethoxy-5-methoxycarbonyloxy-1,3-oxathiolan und Gemische davon;
(k) *cis*-2-Carboethoxy-5-acetoxy-1,3-oxoathiolan, *trans*-2-Carboethoxy-5-acetoxy-1,3-oxoathiolan und Gemische davon;
(l) *cis*-2-Carboethoxy-5-(N4'-acetylcytosin-1'-yl)-1,3-oxathiolan;
(m) *cis*-2-Carboethoxy-5-(cytosin-1'-yl)-1,3-oxathiolan;
(n) *cis*-2-Carboethoxy-5-(uracil-1'-yl)-1,3-oxathiolan;
(o) *cis*-2-Benzoyloxymethyl-5-(cytosin-1'-yl)-1,3-oxathiolan;
(p) *cis*-2-Ethyl-5-iod-1,3-oxathiolan-2-carboxylat, *trans*-2-Ethyl-5-iod-1,3-oxathiolan-2-carboxylat und deren Gemische;
(q) *cis*-2-Ethyl-5-(6'-chlorpurin-9'-yl)-1,3-oxathiolan-2-carboxylat, *trans*-2-Ethyl-5-(6'-chlorpurin-9'-yl)-1,3-oxathiolan-2-carboxylat und deren Gemische;
(r) *cis*-2-Ethyl-5-(6'-chlorpurin-7'-yl)-1,3-oxathiolan-2-carboxylat, *trans*-2-Ethyl-5-(6'-chlorpurin-7'-yl)-1,3-oxathiolan-2-carboxylat und deren Gemische
(s) *cis*-2-Acetoxymethyl-5-acetoxy-1,3-oxathiolan, *trans*-2-Acetoxymethyl-5-acetoxy-1,3-oxathiolan und deren Gemische.

43. Zwischenprodukt, das sich für die Herstellung von Oxathiolanverbindungen der Formel (XIII) eignet 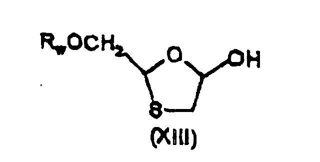 in der R_{w} Wasserstoff oder R₁ ist, wobei R₁ eine Hydroxylschutzgruppe ist, ausgewählt aus Aralkyl (z.B. Benzyl, Diphenylmethyl oder Triphenylmethyl), heterocyclischen Gruppen wie Tetrahydropyranyl, Acyl (z.B. Acetyl oder Benzoyl) und Silylgruppen wie Trialkylsilyl (z.B. t-Butyldimethylsilyl).

## Revendications

1. Procédé de préparation d'un composé oxathiolane comprenant :
la réaction d'un mercaptoacétaldéhyde, dans lequel le mercaptoacétaldéhyde est obtenu à partir d'un dimère de mercaptoacétaldéhyde dissous dans un solvant inerte, avec un composé de formule R_{w}OCH₂CHO, dans des conditions neutres ou basiques, dans laquelle R_{w} est un atome d'hydrogène ou R₁, où R₁ est un groupe protecteur d'hydroxyle, pour donner un composé de formule (XIII)

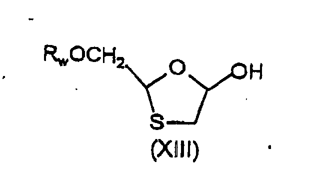

2. Procédé selon la revendication 1, comprenant en outre la transformation du groupe hydroxyle du composé de formule (XIII) en une fonction partante L convenable pour donner un composé de formule (XIV) : 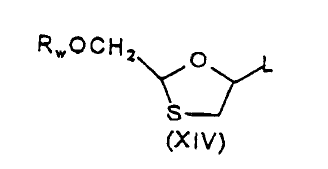 dans laquelle L est un groupe alcoxycarbonyle ; un atome d'halogène ; des thiolates substitués ou non substitués, saturés ou insaturés ; un groupe sélénino substitué ou non substitué, saturé ou insaturé ; des cétones aliphatiques ou aromatiques, substituées ou non substituées, saturées ou insaturées ; ou -OR ; et
R est un atome d'hydrogène, un groupe alkyle en C₁-C₆ substitué ou non substitué, saturé ou insaturé ; un groupe alcényle substitué ou non substitué, saturé ou insaturé, comportant jusqu'à 6 atomes de carbone ; un groupe acyle aliphatique ou aromatique, substitué ou non substitué ; un groupe alcoxycarbonyle substitué ou non substitué, saturé ou insaturé ; un groupe sulfonylimidazolide substitué ou non substitué ; un groupe carbonylimidazolide substitué ou non substitué ; un groupe aminocarbonyle aliphatique ou aromatique, substitué ou non substitué ; un groupe imidate d'alkyle substitué ou non substitué ; un groupe phosphinoyle substitué ou non substitué, saturé ou insaturé ; ou un groupe sulfonyle aliphatique ou aromatique, substitué ou non substitué.

3. Procédé selon la revendication 2, comprenant en outre la réaction du composé de formule (XIV) avec une base de type pyrimidine silylée, une base de type purine silylée, ou un analogue silylé d'une base de type pyrimidine ou purine, éventuellement en présence d'un acide de Lewis, pour produire un composé de formule (IX) : 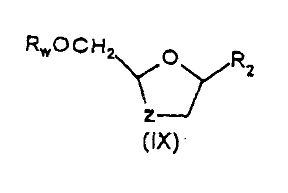 dans laquelle Z est S ; et R₂ est choisi dans le groupe constitué par 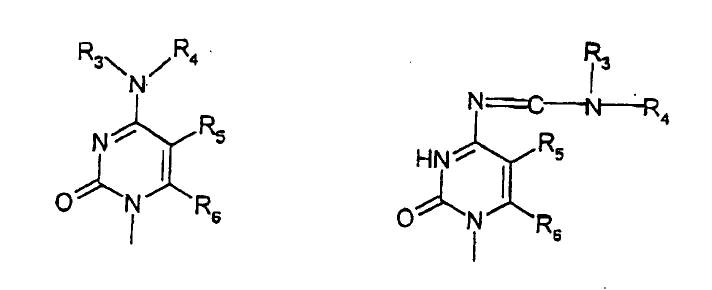 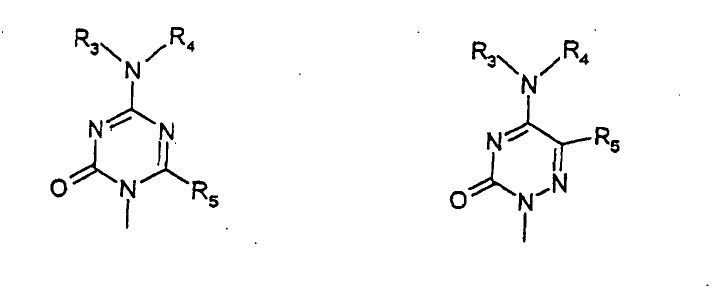 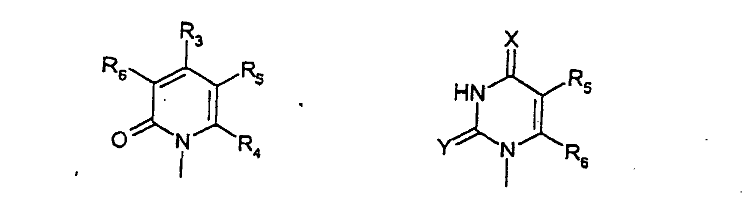 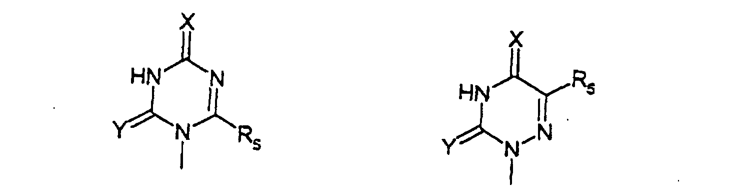 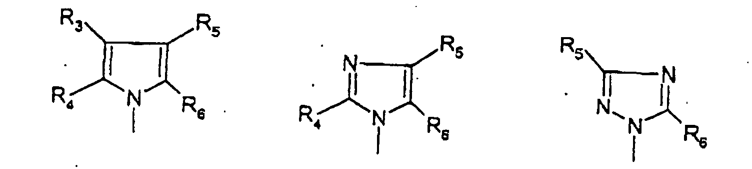 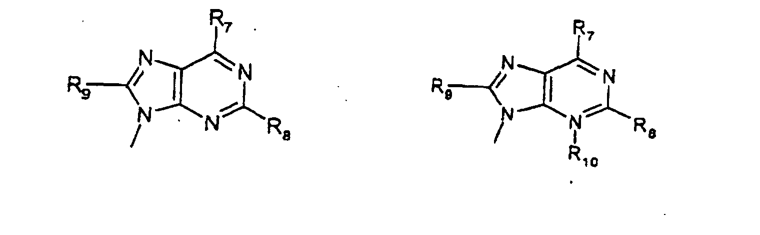 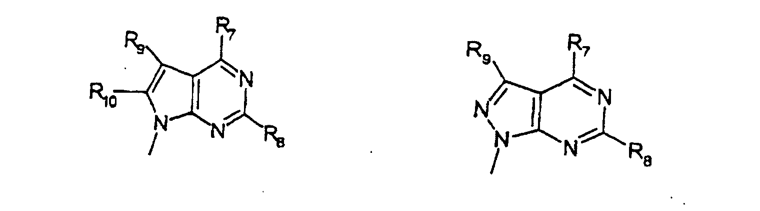 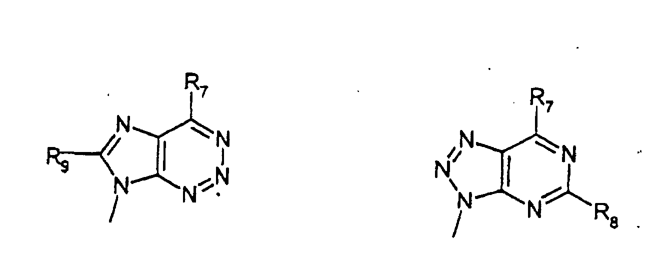 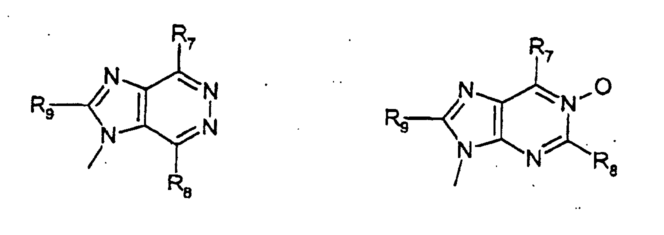 dans laquelle :
X est un atome d'oxygène ou de soufre ;
Y est un atome d'oxygène ou de soufre ;
R₃ et R₄ sont chacun indépendamment choisis dans le groupe constitué par un atome d'hydrogène, un groupe hydroxyle, amino, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyle ou aracyle en C₁-C₁₀ substitué ou non substitué ;
R₅ et R₆ sont chacun indépendamment choisis dans le groupe constitué par un atome d'hydrogène, un atome d'halogène, un groupe hydroxyle, amino, cyano, carboxy, carbamoyle, alcoxycarbonyle, hydroxyméthyle, trifluorométhyle, thioaryle, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué ;
R₇ et R₈ sont chacun indépendamment choisis dans le groupe constitué par un atome d'hydrogène, un groupe hydroxy, alcoxy, thiol, thioalkyle, amino, amino substitué, un atome d'halogène, un groupe cyano, carboxy, alcoxycarbonyle, carbamoyle, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué ;
R₉ et R₁₀ sont chacun indépendamment choisis dans le groupe constitué par un atome d'hydrogène, un groupe hydroxy, alcoxy, amino, amino substitué, un atome d'halogène, un groupe azido, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué.

4. Procédé selon la revendication 3, dans lequel la réaction avec le composé de formule (XIV) est réalisée en l'absence d'acide de Lewis.

5. Procédé selon la revendication 3, dans lequel la réaction avec le composé de formule (XIV) est réalisée en présence d'un acide de Lewis.

6. Procédé selon l'une quelconque des revendications 3 à 5, comprenant en outre l'oxydation du groupe Z du composé de formule (IX) pour donner un composé de formule (IX) dans laquelle le groupe Z est S=O ou SO₂.

7. Procédé selon l'une quelconque des revendications 3 à 6, comprenant en outre l'acylation du composé de formule (IX) avec de l'anhydride acétique pour donner un composé de formule (X) : 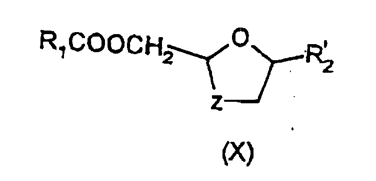 dans laquelle R₁ est un groupe protecteur d'hydroxyle et R'₂ est un groupe R₂ acylé.

8. Procédé selon la revendication 7, comprenant en outre la séparation du composé de formule (X) en ses isomères cis ou trans.

9. Procédé selon la revendication 7 ou la revendication 8, comprenant en outre l'hydrolyse des fonctions acyle R'₂ et R₁COOCH₂ du composé de formule (X), pour donner un composé de formule (I), un isomère géométrique de celui-ci ou un isomère optique de celui-ci :

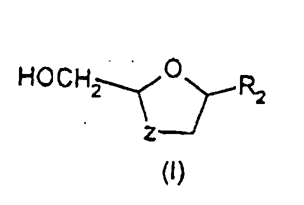

10. Procédé selon l'une quelconque des revendications 1 à 9, dans lequel R₁ est un groupe acyle comportant 1 à 16 atomes de carbone, non substitué ou substitué par un hétéroatome, ou est un groupe silyle.

11. Procédé selon la revendication 1, dans lequel le solvant inerte est choisi dans le groupe constitué par la pyridine, le toluène et le diméthylsulfoxyde.

12. Procédé de préparation d'un composé oxathiolane comprenant :
la réaction d'un mercaptoacétaldéhyde avec un composé de formule R_{y}OOCCHO, dans laquelle R_{y} est un groupe alkyle en C₁-C₁₂ substitué ou non substitué ou un groupe aryle en C₆-C₂₀ substitué ou non substitué, pour donner un composé de formule (XV)

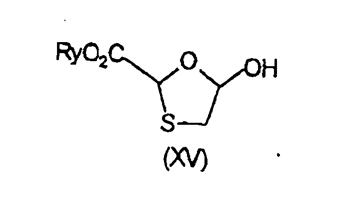

13. Procédé selon la revendication 12, comprenant en outre la transformation du groupe hydroxyle du composé de formule (XV) en une fonction partante L convenable pour donner un composé de formule (XVI) : 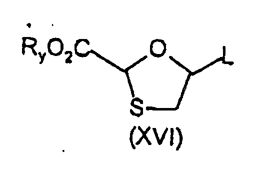 dans laquelle L est un groupe alcoxycarbonyle ; un atome d'halogène ; des thiolates substitués ou non substitués, saturés ou insaturés ; un groupe sélénino substitué ou non substitué, saturé ou insaturé ; des cétones aliphatiques ou aromatiques, substituées ou non substituées, saturées ou insaturées ; ou -OR ; et
R est un atome d'hydrogène, un groupe alkyle en C₁-C₆ substitué ou non substitué, saturé ou insaturé ; un groupe alcényle substitué ou non substitué, saturé ou insaturé, comportant jusqu'à 6 atomes de carbone ; un groupe acyle aliphatique ou aromatique, substitué ou non substitué ; un groupe alcoxycarbonyle substitué ou non substitué, saturé ou insaturé ; un groupe sulfonylimidazolide substitué ou non substitué ; un groupe carbonylimidazolide substitué ou non substitué ; un groupe aminocarbonyle aliphatique ou aromatique, substitué ou non substitué ; un groupe imidate d'alkyle substitué ou non substitué ; un groupe phosphinoyle substitué ou non substitué ; ou un groupe sulfonyle aliphatique ou aromatique, substitué ou non substitué.

14. Procédé selon la revendication 13, comprenant en outre la réaction du composé de formule (XVI) avec une base de type pyrimidine silylée, une base de type purine silylée, ou un analogue silylé d'une base de type pyrimidine ou purine, éventuellement en présence d'un acide de Lewis, pour produire un composé de formule (XVII) : 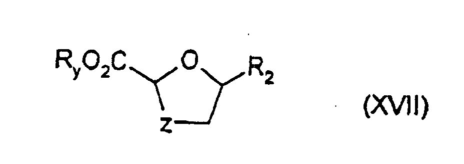 dans laquelle Z est S ; et
R₂ est choisi dans le groupe constitué par 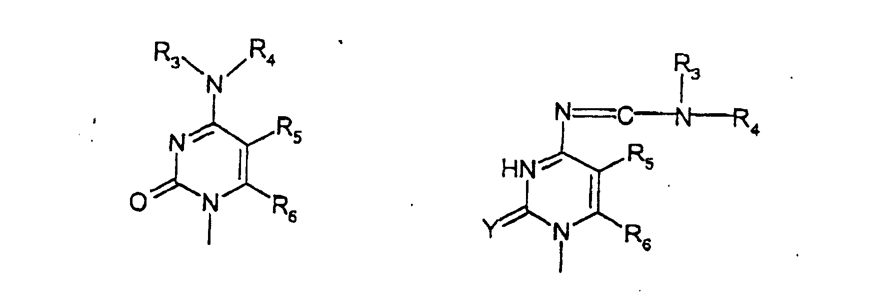 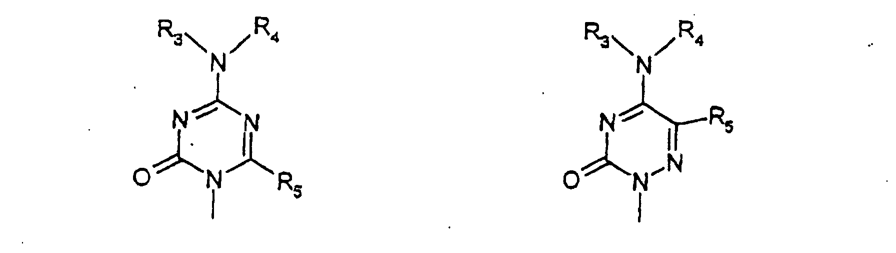 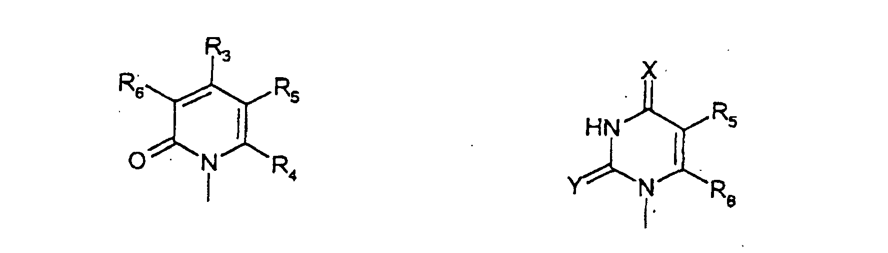 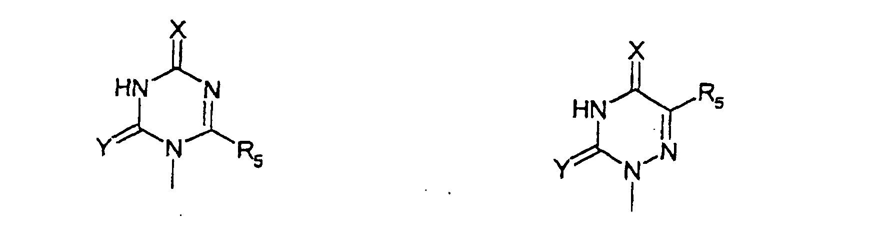 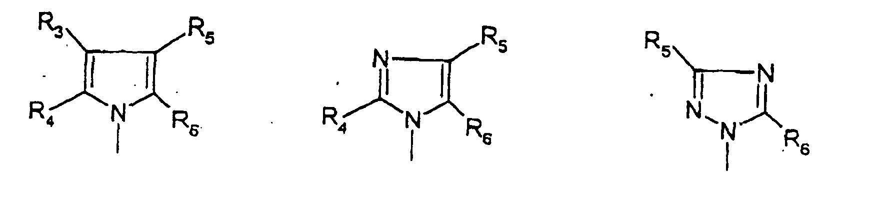 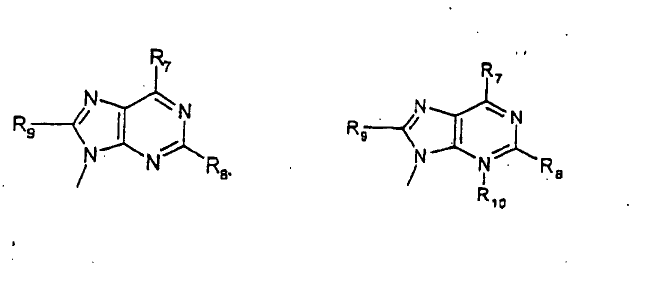 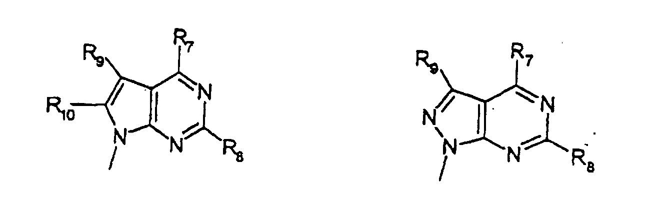 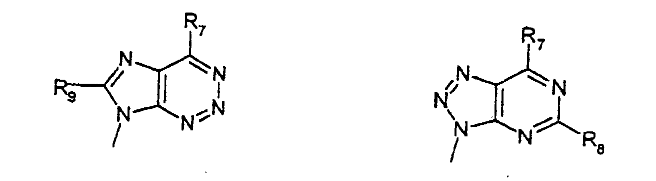 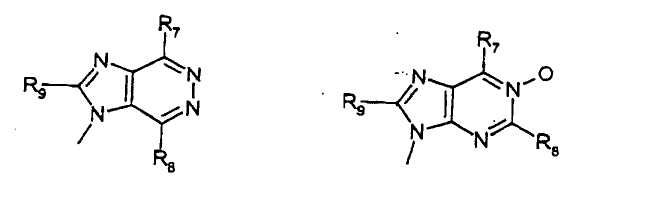 dans laquelle :
X est un atome d'oxygène ou de soufre ;
Y est un atome d'oxygène ou de soufre ;
R₃ et R₄ sont chacun indépendamment choisis dans le groupe constitué par un atome d'hydrogène, un groupe hydroxyle, amino, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyle ou aracyle en C₁-C₁₀ substitué ou non substitué ;
R₅ et R₆ sont chacun indépendamment choisis dans le groupe constitué par un atome d'hydrogène, un atome d'halogène, un groupe hydroxyle, amino, cyano, carboxy, carbamoyle, alcoxycarbonyle, hydroxyméthyle, trifluorométhyle, thioaryle, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué ;
R₇ et R₈ sont chacun indépendamment choisis dans le groupe constitué par un atome d'hydrogène, un groupe hydroxy, alcoxy, thiol, thioalkyle, amino, amino substitué, un atome d'halogène, un groupe cyano, carboxy, alcoxycarbonyle, carbamoyle, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué ;
R₉ et R₁₀ sont chacun indépendamment choisis dans le groupe constitué par un atome d'hydrogène, un groupe hydroxy, alcoxy, amino, amino substitué, un atome d'halogène, un groupe azido, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué.

15. Procédé selon la revendication 14, dans lequel ledit composé de formule (XVII) est principalement obtenu sous la forme de l'isomère cis.

16. Procédé selon la revendication 14 ou 15, comprenant en outre l'oxydation du groupe Z du composé de formule (XVII) pour donner un composé de formule (XVII) dans laquelle le groupe Z est S=O ou SO₂.

17. Procédé selon l'une quelconque des revendications 14 à 16, comprenant en outre la réduction du groupe R_{y}O₂C du composé de formule (XVII) avec un agent réducteur convenable pour donner un composé de formule (XVIII) :

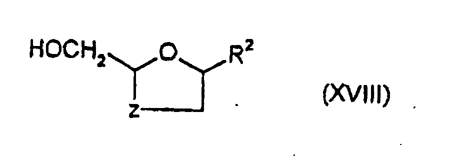

18. Procédé selon la revendication 17, comprenant en outre la séparation du composé en ses isomères cis ou trans.

19. Procédé selon la revendication 13, comprenant en outre la réaction du composé de formule (XVI) avec un acide de Lewis de type silyle contenant un atome d'halogène pour donner un composé de formule (XXVI) : 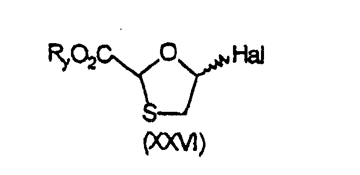 dans laquelle Hal est un atome d'halogène.

20. Procédé selon la revendication 19, comprenant en outre le couplage du composé de formule (XXVI) avec une base ou un analogue de celle-ci, R₂, dans des conditions basiques pour donner un composé de formule (XVII) : 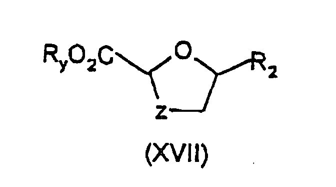 dans laquelle R₂ est choisi dans le groupe constitué par 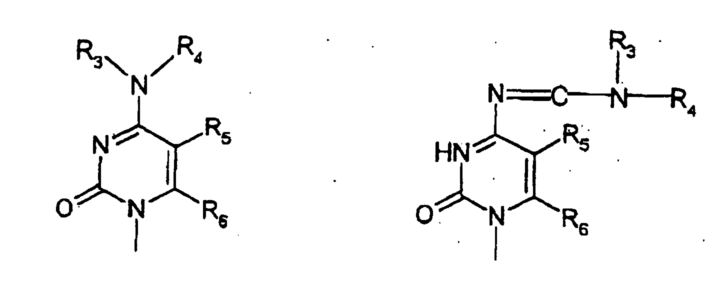 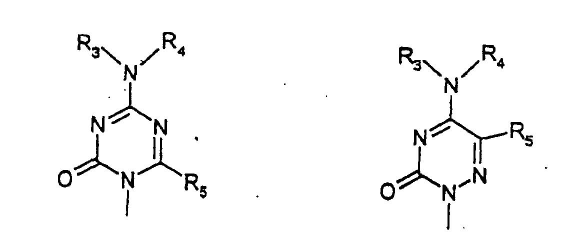 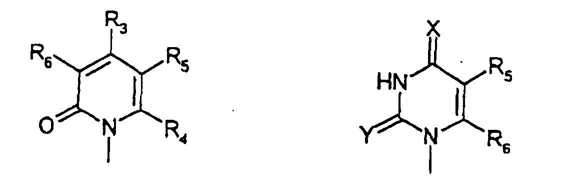 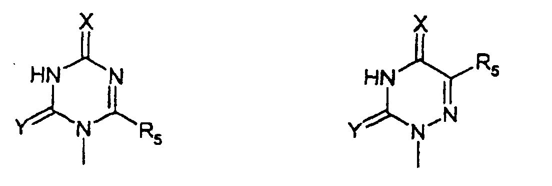 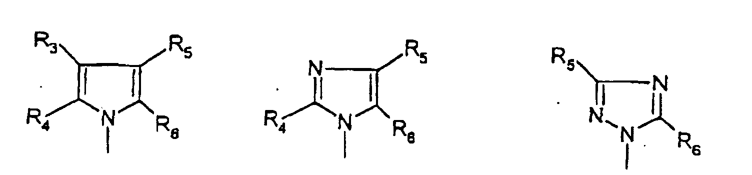 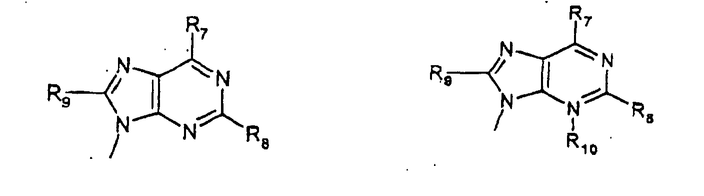 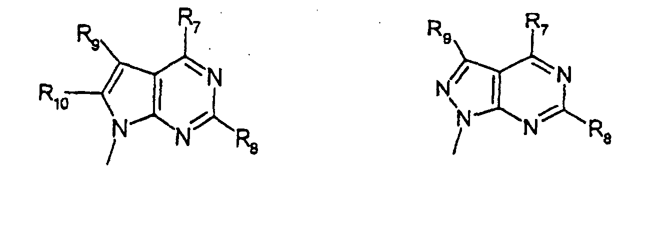 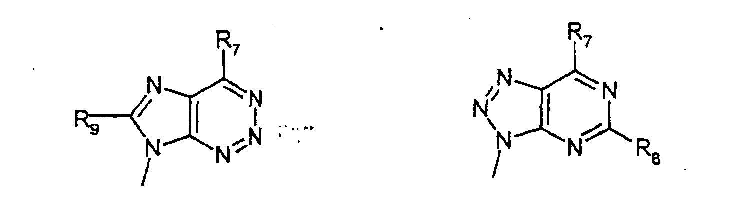 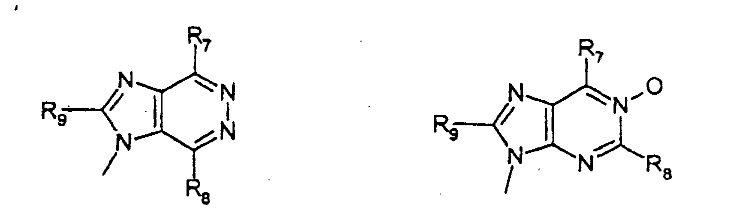 dans laquelle :
X est un atome d'oxygène ou de soufre ;
Y est un atome d'oxygène ou de soufre ;
R₃ et R₄ sont chacun indépendamment choisis dans le groupe constitué par un atome d'hydrogène, un groupe hydroxyle, amino, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyle ou aracyle en C₁-C₁₀ substitué ou non substitué ;
R₅ et R₆ sont chacun indépendamment choisis dans le groupe constitué par un atome d'hydrogène, un atome d'halogène, un groupe hydroxyle, amino, cyano, carboxy, carbamoyle, alcoxycarbonyle, hydroxyméthyle, trifluorométhyle, thioaryle, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué ;
R₇ et R₈ sont chacun indépendamment choisis dans le groupe constitué par un atome d'hydrogène, un groupe hydroxy, alcoxy, thiol, thioalkyle, amino, amino substitué, un atome d'halogène, un groupe cyano, carboxy, alcoxycarbonyle, carbamoyle, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué ;
R₉ et R₁₀ sont chacun indépendamment choisis dans le groupe constitué par un atome d'hydrogène, un groupe hydroxy, alcoxy, amino, amino substitué, un atome d'halogène, un groupe azido, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué.

21. Procédé selon la revendication 19, dans lequel ledit acide de Lewis est l'iodure de triméthylsilyle.

22. Procédé selon la revendication 19 ou la revendication 21, dans lequel Hal est l'atome d'iode.

23. Procédé selon l'une quelconque des revendications 20 à 22, dans lequel R₂ est une base de type purine.

24. Procédé selon la revendication 23, dans lequel la purine est la 6-chloropurine.

25. Procédé selon l'une quelconque des revendications 12 à 24, dans lequel le mercaptoacétaldéhyde est obtenu à partir d'un dimère de mercaptoacétaldéhyde dans un solvant inerte.

26. Procédé selon la revendication 25, dans lequel le solvant inerte est choisi dans le groupe constitué par la pyridine, le toluène et le diméthylsulfoxyde.

27. Procédé selon la revendication 13, comprenant en outre la réduction du groupe ester du composé de formule (XVI) et la protection du groupe hydroxyméthyle résultant avec une fonction protectrice d'hydroxy R₁ pour donner un composé de formule (XXII) : 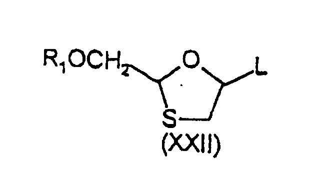 dans laquelle L est un groupe alcoxycarbonyle ; un atome d'halogène ; des thiolates substitués ou non substitués, saturés ou insaturés ; un groupe sélénino substitué ou non substitué, saturé ou insaturé ; des cétones aliphatiques ou aromatiques, substituées ou non substituées, saturées ou insaturées ; ou -OR ; et
R est un atome d'hydrogène, un groupe alkyle en C₁-C₆ substitué ou non substitué, saturé ou insaturé ; un groupe alcényle substitué ou non substitué, saturé ou insaturé, comportant jusqu'à 6 atomes de carbone ; un groupe acyle aliphatique ou aromatique, substitué ou non substitué ; un groupe alcoxycarbonyle substitué ou non substitué, saturé ou insaturé ; un groupe sulfonylimidazolide substitué ou non substitué ; un groupe carbonylimidazolide substitué ou non substitué ; un groupe aminocarbonyle aliphatique ou aromatique, substitué ou non substitué ; un groupe imidate d'alkyle substitué ou non substitué ; un groupe phosphinoyle substitué ou non substitué, saturé ou insaturé; ou un groupe sulfonyle aliphatique ou aromatique, substitué ou non substitué.

28. Procédé selon la revendication 27, dans lequel R₁ est un groupe acyle comportant 1 à 16 atomes de carbone, non substitué ou substitué par un hétéroatome, ou est un groupe silyle.

29. Procédé selon la revendication 28, dans lequel R₁ est un groupe t-butyldiméthylsilyle ou t-butyldiphénylsilyle.

30. Procédé selon l'une quelconque des revendications 27 à 29, comprenant en outre l'étape de réaction du composé de formule (XXII) avec une base de type pyrimidine silylée, une base de type purine silylée, ou un analogue silylé d'une base de type pyrimidine ou purine, éventuellement en présence d'un acide de Lewis, pour donner un composé de formule (XXIII) : 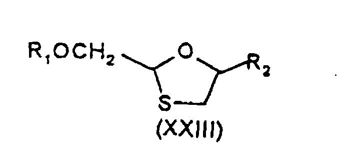 dans laquelle Z est S ; et
R₂ est choisi dans le groupe constitué par 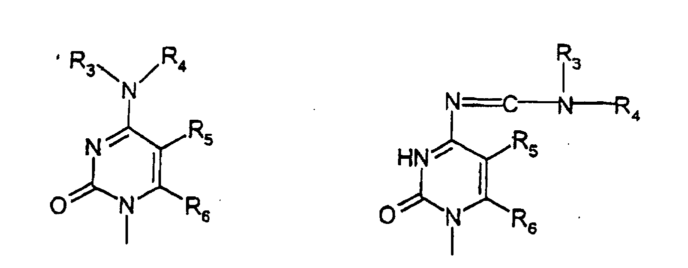 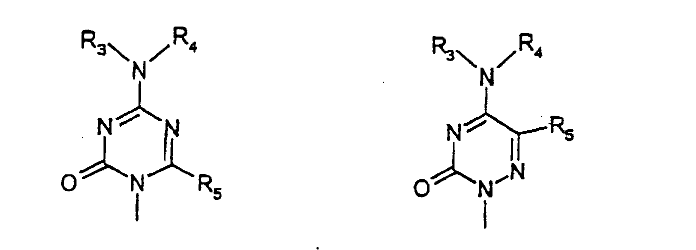 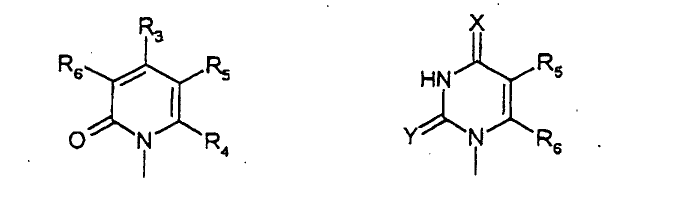 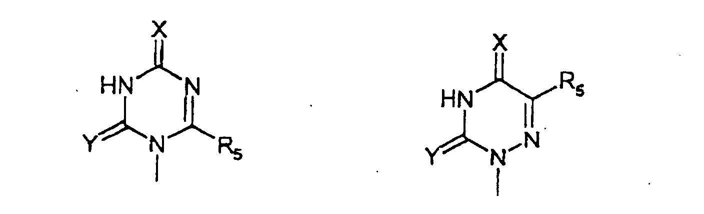 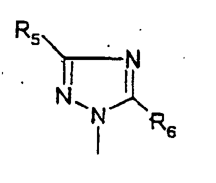 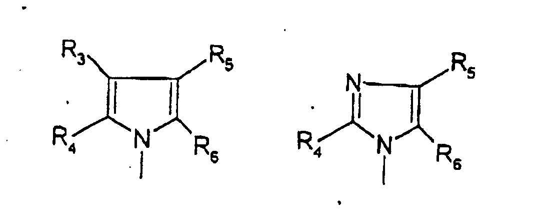 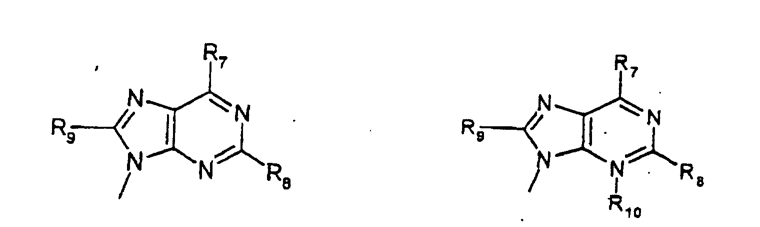 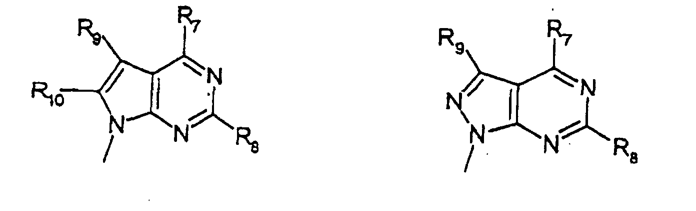 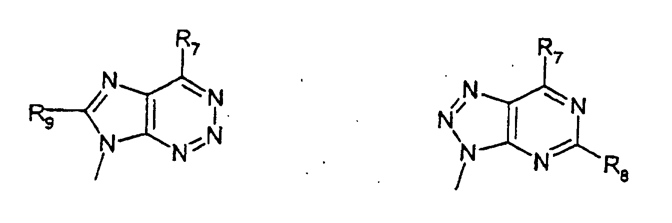 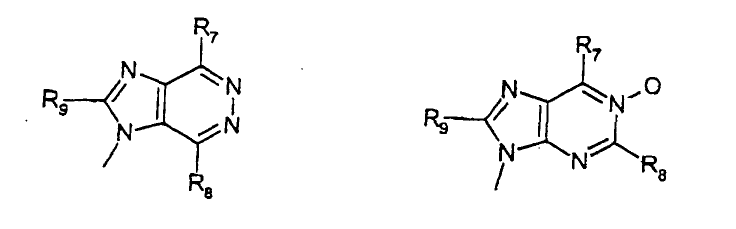 dans laquelle :
X est un atome d'oxygène ou de soufre ;
Y est un atome d'oxygène ou de soufre ;
R₃ et R₄ sont chacun indépendamment choisis dans le groupe constitué par un atome d'hydrogène, un groupe hydroxyle, amino, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyle ou aracyle en C₁-C₁₀ substitué ou non substitué ;
R₅ et R₆ sont chacun indépendamment choisis dans le groupe constitué par un atome d'hydrogène, un atome d'halogène, un groupe hydroxyle, amino, cyano, carboxy, carbamoyle, alcoxycarbonyle, hydroxyméthyle, trifluorométhyle, thioaryle, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué ;
R₇ et R₈ sont chacun indépendamment choisis dans le groupe constitué par un atome d'hydrogène, un groupe hydroxy, alcoxy, thiol, thioalkyle, amino, amino substitué, un atome d'halogène, un groupe cyano, carboxy, alcoxycarbonyle, carbamoyle, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué ;
R₉ et R₁₀ sont chacun indépendamment choisis dans le groupe constitué par un atome d'hydrogène, un groupe hydroxy, alcoxy, amino, amino substitué, un atome d'halogène, un groupe azido, alkyle en C₁-C₆ substitué ou non substitué, alcényle substitué ou non substitué, comportant jusqu'à 6 atomes de carbone, alcynyle substitué ou non substitué, comportant jusqu'à 6 atomes de carbone, et acyloxy en C₁-C₁₀ substitué ou non substitué.

31. Procédé selon la revendication 30, dans lequel le composé de formule (XXIII) est oxydé pour donner un composé de formule (XXIII) dans laquelle Z est S=O ou SO₂.

32. Procédé selon la revendication 31, comprenant l'acylation du groupe R₂ du composé de formule (XXIII) avec de l'anhydride acétique dans un solvant, pour donner un composé de formule (XXIV) : 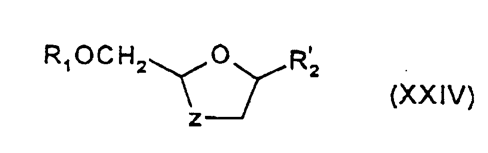 dans laquelle R'₂ est un groupe R₂ acylé.

33. Procédé selon la revendication 32, comprenant en outre la séparation du composé en ses isomères cis ou trans.

34. Procédé selon la revendication 32 ou la revendication 33, comprenant en outre l'hydrolyse du groupe R'₂ du composé de formule (XXIV) dans des conditions basiques pour donner un composé de formule (XXV).

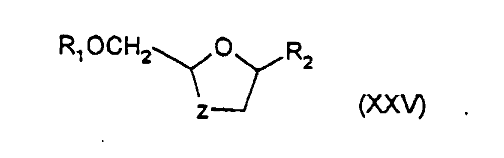

35. Procédé selon la revendication 34, comprenant en outre l'élimination de la fonction protectrice d'hydroxyle R₁ du composé (XXIII) pour donner un composé de formule (I) :

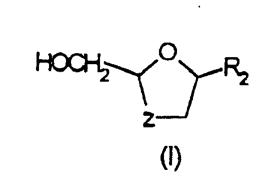

36. Procédé selon l'une quelconque des revendications 9, 17 et 35, comprenant en outre la transformation du composé de formule (I) en un sel pharmaceutiquement acceptable de celui-ci.

37. Procédé selon l'une quelconque des revendications 9, 17 et 35, comprenant en outre l'estérification du composé de formule (I) dans lequel le groupe OH est remplacé par un groupe R-CO-O- et R est H, un groupe alkyle à chaîne droite ou ramifiée, alcoxyalkyle, arylalkyle, aryloxyalkyle ou dihydropyridinyle substitué.

38. Procédé selon l'une quelconque des revendications 9, 17 et 35, comprenant en outre l'estérification du composé de formule (I) dans lequel le groupe OH est remplacé par un alkylsulfonylester, un aralkylsulfonylester, un ester sulfate, un ester d'acide aminé, un ester monophosphate, un ester diphosphate, un ester triphosphate ou un ester dérivé de HOOC(CH₂)_{q}COOH où q vaut de 0 à 10.

39. Procédé selon l'une quelconque des revendications 9, 17 et 35, comprenant en outre l'estérification du composé de formule (I) dans lequel le groupe OH est transformé en un ester et ledit ester est un ester alkylique en C₁-C₁₆, un benzylester non substitué, ou un benzylester substitué par au moins un atome d'halogène, un groupe alkyle en C₁-C₆, alcényle en C₂-C₆, alcoxy en C₁-C₆ saturé ou insaturé, nitro et trifluorométhyle.

40. Procédé selon l'une quelconque des revendications 27 à 39, dans lequel le mercaptoacétaldéhyde est obtenu à partir d'un dimère de mercaptoacétaldéhyde dissous dans un solvant inerte.

41. Procédé selon la revendication 40, dans lequel le solvant inerte est choisi dans le groupe constitué par la pyridine, le toluène et le diméthylsulfoxyde.

42. Intermédiaires utiles pour la production de composés oxathiolane, lesdits intermédiaires étant choisis dans le groupe constitué par :
(a) le trans-2-hydroxyméthyl-5-acétoxy-1,3-oxathiolane ;
(b) le cis-2-benzoyloxyméthyl-5-hydroxy-1,3-oxathiolane, le trans-2-benzoyloxyméthyl-5-hydroxy-1,3-oxathiolane ou un mélange de ceux-ci ;
(c) le cis-2-benzoyloxyméthyl-5-acétoxy-1,3-oxathiolane, le trans-2-benzoyloxyméthyl-5-acétoxy-1,3-oxathiolane ou un mélange de ceux-ci ;
(d) le cis-2-benzoyloxyméthyl-5-(4',5'-dichlorobenzoyloxy)-1,3-oxathiolane, le trans-2-benzoyloxyméthyl-5-(4',5'-dichlorobenzoyloxy)-1,3-oxathiolane ou un mélange de ceux-ci ;
(e) le cis-2-benzoyloxyméthyl-5-triméthylacétoxy-1,3-oxathiolane, le trans-2-benzoyloxyméthyl-5-triméthylacétoxy-1,3-oxathiolane ou un mélange de ceux-ci ;
(f) le cis-2-benzoyloxyméthyl-5-(2',2',2'-trichloro-éthoxycarbonyloxy)-1,3-oxathiolane, le trans-2-benzoyloxyméthyl-5-(2',2',2'-trichloroéthoxycarbonyloxy)-1,3-oxathiolane ou un mélange de ceux-ci ;
(g) le cis-2-benzoyloxyméthyl-5-(éthoxycarbonyloxy)-1,3-oxathiolane, le trans-2-benzoyloxyméthyl-5-(éthoxycarbonyloxy)-1,3-oxathiolane ou un mélange de ceux-ci ;
(h) le cis-2-benzoyloxyméthyl-5-(méthoxycarbonyloxy)-1,3-oxathiolane, le trans-2-benzoyloxyméthyl-5-(méthoxycarbonyloxy)-1,3-oxathiolane
ou un mélange de ceux-ci ;
(i) le cis-2-benzoyloxyméthyl-5-benzoyloxy-1,3-oxathiolane, le trans-2-benzoyloxyméthyl-5-benzoyloxy-1,3-oxathiolane ou un mélange de ceux-ci ;
(j) le cis-2-carboéthoxy-5-méthoxycarbonyloxy-1,3-oxathiolane, le trans-2-carboéthoxy-5-méthoxycarbonyloxy-1,3-oxathiolane et des mélanges de ceux-ci ;
(k) le cis-2-carboéthoxy-5-acétoxy-1,3-oxathiolane, le trans-2-carboéthoxy-5-acétoxy-1,3-oxathiolane et des mélanges de ceux-ci ;
(l) le cis-2-carboéthoxy-5-(N-4'-acétylcytosin-1'-yl)-1,3-oxathiolane ;
(m) le cis-2-carboéthoxy-5-(cytosin-1'-yl)-1,3-oxathiolane ;
(n) le cis-2-carboéthoxy-5-(uracil-1'-yl)-1,3-oxathiolane ;
(o) le cis-2-benzoyloxyméthyl-5-(cytosin-1'-yl)-1,3-oxathiolane ;
(p) le cis-2-éthyl-5-iodo-1,3-oxathiolan-2-carboxylate, le trans-2-éthyl-5-iodo-1,3-oxathiolan-2-carboxylate et des mélanges de ceux-ci ;
(q) le cis-2-éthyl-5-(6'-chloropurin-9'-yl)-1,3-oxathiolan-2-carboxylate, le trans-2-éthyl-5-(6'-chloropurin-9'-yl)-1,3-oxathiolan-2-carboxylate et des mélanges de ceux-ci ;
(r) le cis-2-éthyl-5-(6'-chloropurin-7'-yl)-1,3-oxathiolan-2-carboxylate, le trans-2-éthyl-5-(6'chloropurin-7'-yl)-1,3-oxathiolan-2-carboxylate et des mélanges de ceux-ci ;
(s) le cis-2-acétoxyméthyl-5-acétoxy-1,3-oxathiolane, le trans-2-acétoxyméthyl-5-acétoxy-1,3-oxathiolane et des mélanges de ceux-ci ;

43. Intermédiaire utile pour la production de composés oxathiolane répondant à la formule (XIII) : 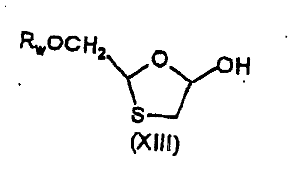 dans laquelle R_{w} est un atome d'hydrogène ou R₁, où R₁ est un groupe protecteur d'hydroxyle choisi parmi un groupe aralkyle (par exemple benzyle, diphénylméthyle ou triphénylméthyle), des groupes hétérocycliques tels que tétrahydropyranyle, acyle (par exemple acétyle ou benzoyle) et des groupes silyle tels que trialkylsilyle (par exemple t-butyldiméthylsilyle).
